# EUROPEAN PATENT SPECIFICATION

(11) **EP 3 065 808 B1**
(45) Date of publication and mention of the grant of the patent: **30.08.2023**
(21) Application number: 14859759.4
(22) Date of filing: 05.11.2014
(51) Int. Cl.: A61M 37/00, A61C 7/08, A61K 9/22, A61P 25/16

(54) **CONTINUOUS DRUG DELIVERY VIA THE MOUTH**
VORRICHTUNGEN UND VERFAHREN ZUR KONTINUIERLICHEN ORALEN ARZNEIMITTELVERABREICHUNG
DISPOSITIFS ET PROCÉDÉS POUR L'ADMINISTRATION DE MÉDICAMENT CONTINUE PAR LA BOUCHE

(30) Priority: 05.11.2013 US 201361899979 P; 10.01.2014 US 201461926022 P; 02.05.2014 US 201461987899 P; 27.08.2014 US 201462042553 P
(43) Date of publication of application: 14.09.2016
(73) Proprietor: SynAgile Corporation, Wilson, WY 83014 (US)
(72) Inventor: HELLER, Ephraim, Wilson, WY 83014 (US); HELLER, Adam, Austin, TX 78701 (US); REHLAENDER, Bruce, Lake Oswego, OR 97034 (US); SPIRIDIGLOZZI, John, Boston, MA 02127 (US)
(74) Representative: Witthoff Jaekel Steinecke Patentanwälte PartG mbB
(86) International application number: PCT/US2014/064137
(87) International publication number: WO 2015/069773

(56) References cited:
- WO-A1-2009/059242
- WO-A2-2004/069076
- WO-A2-2008/053297
- US-A1- 2004 158 194
- US-A1- 2007 015 763
- US-A1- 2009 163 859
- US-A1- 2011 208 163
- US-B2- 7 891 358

## Description

### FIELD OF THE INVENTION

The invention features a drug delivery device anchored in the mouth for continuously administering a drug in solid form or a fluid in which a drug is dissolved or suspended as characterized in the claims.

### BACKGROUND

This invention relates to devices and methods for continuous or semi-continuous drug administration via the oral route. It is an aim of this invention to solve several problems related to drugs with short physiological half-lives of drugs (e.g., shorter than 8 hours, 6 hours, 4 hours, 2 hours, 1 hour, 30 min, 20 min or 10 min) and/or narrow therapeutic windows of drugs that are currently dosed multiple times per day: it is inconvenient to take a drug that must be dosed multiple times per day or at night, the drug's pharmacokinetics and efficacy may be sub-optimal, and side effects may increase in frequency and/or severity. Continuous or semi-continuous administration is particularly beneficial for drugs with a short half-life and/or a narrow therapeutic window, such as levodopa (LD), anti-epileptics (e.g., oxcarbazepine, topiramate, lamotrigine, gabapentin, carbamazepine, valproic acid, levetiracetam, pregabalin), and sleep medications (e.g., zaleplon). Continuous or semi-continuous infusion in the mouth can provide for lesser fluctuation in the concentration of a drug in an organ or fluid, for example in the blood or plasma. Convenient, automatic administration of a drug can also increase patient compliance with their drug regimen, particularly for patients who must take medications at night and for patients with dementia.

Medical conditions managed by continuously orally administered drugs include Parkinson's disease, bacterial infections, cancer, pain, organ transplantation, disordered sleep, epilepsy and seizures, anxiety, mood disorders, post-traumatic stress disorder, cancer, arrhythmia, hypertension, heart failure, spasticity, dementia, and diabetic nephropathy. Some known drug delivering devices, parts thereof or compositions for treatment of Parkinson's disease are described in the following documents. WO 2004/069076 A2 describes devices that release drugs via passive diffusion or by means of an electromechanical pump. US 2007/015763A1 describes compositions of an atypical antipsychotic, for example ziprasidone, and a dopamine agonist for treatment of patients suffering from psychosis and movement disorders associated with Parkinson's disease and subcortical dementias. WO 2008/053297 A2 describes compositions comprising entacapone, levodopa and carbidopa, or pharmaceutically acceptable salts or hydrates thereof. WO 2009/059242 A1 describes composition comprises partial glycine agonist in a controlled release formulation for once a day administration to subjects with Parkinson's disease. US 2004/0158194 A1 describes drug dosage forms, which are housed in oral devices, and methods for controlled drug release. The controlled drug release may be passive, based on the dosage form, or electronically controlled. US 2011/0208163 A1 describes implantable medical devices for delivering a therapeutic substance via a catheter to a delivery site in a patient. US 7,891,358 B2 an inhaler not fastened to the mouth of a patient with a mechanism for maintaining constant aerosol flow. US 2009/0163859 A1 describes adapters for connecting an oral dose tip or a needle hub assembly to a syringe.

A challenge with most drug delivery devices in the prior art is that they are not designed for placement and operation in the mouth. Devices must be designed to be small, comfortable, and nonirritating, and to not interfere with speech, swallowing, drinking and/or eating. In the mouth saliva, food or drink may penetrate into the drug reservoir and/or the pump, thereby potentially unpredictably extracting and delivering the drug, or reacting with the drug, or clogging the delivery device. Pumps that have been suggested for operation in the mouth, such as osmotic tablets and mucoadhesive patches, often do not reliably provide constant rate drug delivery for extended periods of time under the conditions in the mouth. Drinking of hot or cold beverages may cause undesirable changes in drug delivery, e.g., delivery of a drug bolus. Likewise, sucking on the device may cause delivery of an unwanted bolus. Exposure to foods and liquids such as oils, alcohols, and acids may temporarily or permanently increase or decrease the drug delivery rate from the device. Intra-oral drug delivery devices must also administer the drug into a suitable location in the mouth, e.g., into a location where the drug does not accumulate in an unwanted manner or to a location where it is immediately swallowed. There is, therefore, a need for improved drug delivery devices that can operate comfortably, safely and reliably in the mouth over extended periods of time.

Intra-oral pumps have previously been proposed in inconvenient formats, e.g., wherein the device is located within a replacement tooth. There is a need for improved intra-oral drug delivery devices that can conveniently be inserted and removed by the patient, without requiring the insertion or removal of a replacement tooth, dental bridge, or denture. A problem with these and other pumps that reside in the mouth and that can continuously deliver drug in the mouth, such as controlled release osmotic tablets and muco-adhesive drug delivery patches, is that once drug delivery has begun it cannot be temporarily stopped. Temporarily stopping the drug delivery is desirable so that drug is not wasted and, more importantly, so that dispensed drug does not accumulate on the surface of the device while the device is removed from the mouth. Such an unquantified accumulation of drug on the surface of the device might lead to the undesired delivery of a bolus of an unknown quantity of drug to the patient when the device is placed back into the mouth. Maintenance of accurate rate of drug delivery when the ambient atmospheric pressure changes, e.g., during air-travel or at elevated locations, can also be challenging.

The pumps of the invention can provide constant rate, continuous administration of drugs in the mouth, and can be temporarily stopped when the devices are removed from the mouth.

Most drugs intended for oral administration are formulated as solids (e.g., pills, tablets), solutions or suspensions that are administered once or several times per day. Such drugs are not formulated to meet the requirements of continuous or semi-continuous, constant-rate, intra-oral administration. For example, many suspensions and solutions are formulated in relatively large daily volumes and/or in formulations that are physically or chemically unstable over the course of a day at body temperature; and pills and tablets are rarely formulated in units and dosage amounts appropriate for dosing frequently throughout the day.

Large quantities of drug must be administered to treat some diseases. For example, 1,000 mg of levodopa is a typical daily dose administered to patients with advanced Parkinson's disease. In order to continuously administer such large quantities of drug into the mouth in a fluid volume that will fit comfortably in the mouth (typically less than 5 mL) for many hours, it is sometimes necessary to employ concentrated, often viscous, fluid formulations of the drug. Use of viscous fluids can provide the small volumes, high concentrations, uniform drug dispersion, storage stability, and operational stability desired for the drugs and methods of the invention. Consequently, it is often necessary to employ miniaturized pumps tailored to provide the high pressures required to pump the viscous fluids. The drug devices and formulations of the invention address these unmet needs.

As a specific example, Parkinson's disease (PD) is characterized by the inability of the dopaminergic neurons in the substantia nigra to produce the neurotransmitter dopamine. PD impairs motor skills, cognitive processes, autonomic functions and sleep. Motor symptoms include tremor, rigidity, slow movement (bradykinesia), and loss of the ability to initiate movement (akinesia) (collectively, the "off" state). Non-motor symptoms of PD include dementia, dysphagia (difficulty swallowing), slurred speech, orthostatic hypotension, seborrheic dermatitis, urinary incontinence, constipation, mood alterations, sexual dysfunction, and sleep issues (e.g., daytime somnolence, insomnia).

After more than 40 years of clinical use levodopa therapy remains the most effective method for managing PD and provides the greatest improvement in motor function. Consequently, LD administration is the primary treatment for PD. LD is usually orally administered. The orally administered LD enters the blood and part of the LD in the blood crosses the blood brain barrier. It is metabolized, in part, in the brain to dopamine which temporarily diminishes the motor symptoms of PD. As the neurodegeneration underlying PD progresses, the patients require increasing doses of LD and the fluctuations of brain dopamine levels increase. When too much LD is transported to the brain, dyskinesia sets in (uncontrolled movements such as writhing, twitching and shaking); when too little is transported, the patient re-enters the off state. As PD progresses, the therapeutic window for oral formulations of LD narrows, and it becomes increasingly difficult to control PD motor symptoms without inducing motor complications. In addition, most PD patients develop response fluctuations to intermittent oral LD therapy, such as end of dose wearing off, sudden on/off's, delayed time to on, and response failures.

The devices, formulations and methods of the invention provide improved therapies for patients with PD.

### SUMMARY OF THE INVENTION

The present invention relates to the embodiments as characterized in the claims.

The invention features a drug delivery device, configured and arranged to be removably inserted in a patient's mouth by the patient as characterized in the claims.

In a first aspect, the invention features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) a mechanical pump as characterized in the claims; (iii) an oral liquid impermeable drug reservoir, the volume of the drug reservoir being from 0.1 mL to 5 mL (e.g., 0.1 mL to 1 mL, 1 mL to 2 mL, 2 mL to 3.5 mL, or 3.5 mL to 5 mL); and (iv) an automatic stop/start as characterized in the claims. The drug delivery device can be configured to be automatically stopped upon one or more of the following: (a) the drug delivery device, the pump, and/or the oral liquid impermeable reservoir are removed from the mouth; (b) the drug delivery device, the pump, and/or the oral liquid impermeable reservoir are disconnected from the fastener; or (c) the oral liquid impermeable reservoir is disconnected from the pump. The drug delivery device can be configured to be automatically started upon one or more of the following: (a) the drug delivery device, the pump, and/or the oral liquid impermeable reservoir are inserted into the mouth; (b) the drug delivery device, the pump, and/or the oral liquid impermeable reservoir are connected to the fastener; or (c) the oral liquid impermeable reservoir is connected to the pump. In particular embodiments, the automatic stop/start is selected from: a pressure sensitive switch, a clip, a fluidic channel that kinks, a clutch, a sensor, or a cap. The device further includes a suction-induced flow limiter or a temperature-induced flow limiter as characterized in the claims and optionally bite-resistant structural supports, or a pressure-invariant mechanical pump, or a combination thereof.

The invention further features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) a mechanical pump as characterized in the claims; (iii) an oral liquid impermeable drug reservoir, the volume of the drug reservoir being from 0.1 mLto 5 mL (e.g., 0.1 mL to 1 mL, 1 mL to 2 mL, 2 mL to 3.5 mL, or 3.5 mL to 5 mL); and (iv) a suction-induced flow limiter as characterized in the claims. In certain embodiments, the suction-induced flow limiter includes pressurized surfaces that are in fluidic (gas and/or liquid) contact with the ambient atmosphere via one or more ports or openings in the housing of the drug delivery device. In other embodiments, the suction-induced flow limiter is selected from a deformable channel, a deflectable diaphragm, a compliant accumulator, an inline vacuum-relief valve, and a float valve. The suction-induced flow limiter can be configured to prevent the delivery of a bolus greater than about 5%, 3%, or 1% of the contents of a fresh drug reservoir, when the ambient pressure drops by 41,369 kPa (6 psi), 27.579 kPa (4 psi), 13,790 kPa (2 psi), or 6,895 kPa (1 psi) for a period of 20 second, 40 seconds, one minute, or two minutes. The device optionally further includes an automatic stop/start, a temperature-induced flow limiter, bite-resistant structural supports, or a pressure-invariant mechanical pump.

The invention also features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) mechanical pump as characterized in the claims; (iii) an oral liquid impermeable drug reservoir, the volume of the drug reservoir being from 0.1 mL to 5 mL (e.g., 0.1 mL to 1 mL, 1 mL to 2 mL, 2 mL to 3.5 mL, or 3.5 mL to 5 mL); and (iv) a temperature-induced flow limiter as characterized in the claims. In certain embodiments, the temperature-induced flow limiter includes insulation with a material of low thermal conductivity proximate the drug reservoir and/or the pump. In certain embodiments, the pump is elastomeric and the temperature-induced flow limiter includes an elastomer selected from a natural rubber or a synthetic elastomer. The temperature-induced flow limiter can include an elastomer whose force in a fresh reservoir increases by less than 30%, 20%, or 10% when the oral temperature is raised from 37 to 55 °C for a period of one minute. In other embodiments, the pump includes a spring and the temperature-induced flow limiter includes a spring configured to produce a force in a fresh reservoir that increases by less than 30%, 20%, or 10% when the oral temperature is raised from 37 to 55 °C for a period of one minute. The temperature-induced flow limiter can include a spring including a 300 series stainless steel, titanium, Inconel (i.e., a family of austenitic nickel-chromium-based superalloys), and fully austenitic Nitinol. In still other embodiments, the pump is gas-driven and the temperature-induced flow limiter includes a gas having a volume of less than 40%, 30%, 20% or 10% of the volume of filled drug reservoir in a fresh reservoir at 37 °C and 89,632 kPa (13 psia). For example, the pump can be propellant-driven and the temperature-induced flow limiter includes a propellant having a pressure that increases by less than about 80%, 60%, or 40% when the oral temperature is raised from 37 to 55 °C for a period of one minute. The device optionally further includes a suction-induced flow limiter, an automatic stop/start, bite-resistant structural supports, or a pressure-invariant mechanical pump.

The invention further features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) a mechanical pump as characterized in the claims; (iii) an oral liquid impermeable drug reservoir, the volume of the drug reservoir being from 0.1 mLto 5 mL (e.g., 0.1 mL to 1 mL, 1 mL to 2 mL, 2 mL to 3.5 mL, or 3.5 mL to 5 mL); and (iv) bite-resistant structural supports. In certain embodiments, the bite-resistant structural supports are selected from: a housing that encapsulates the entire drug reservoir and pump components; posts; ribs; or a potting material. The device further includes a suction-induced flow limiter as characterized in the claims and optionally, an automatic stop/start, a temperature-induced flow limiter, or a pressure-invariant mechanical pump.

The invention also features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) a pressure-invariant mechanical pump; and (iii) an oral liquid impermeable drug reservoir, the volume of the drug reservoir being from 0.1 mL to 5 mL (e.g., 0.1 mL to 1 mL, 1 mL to 2 mL, 2 mL to 3.5 mL, or 3.5 mL to 5 mL) as characterized in the claims. The pressure-invariant mechanical pump is selected from a spring, an elastomer, compressed gas, and a propellant. In certain embodiments, the pressure-invariant mechanical pump includes pressurized surfaces that are in fluidic (gas and/or liquid) contact with the ambient atmosphere via one or more ports or openings in the housing of the drug delivery device. The pressure-invariant mechanical pump can be configured to maintain an internal pressure of greater than or equal to about 405,3 kPa (4 atm). In other embodiments, the pressure-invariant mechanical pump is configured such that the average rate of drug delivery increases or decreases by less than about 20%, 10%, or 5% at 101,353 kPa (14.7 psia) and at 77,911 kPa (11.3 psia), as compared to the average rate of delivery at 89,632 kPa (13.0 psia). The device further includes a suction-induced flow limiter as characterized in the claims and optionally an automatic stop/start, a temperature-induced flow limiter, or bite-resistant structural supports.

The invention further features a drug delivery device configured to be removably inserted in a patient's mouth and for continuous or semi-continuous intraoral administration of a pharmaceutical composition including a drug, the device including: (i) a fastener to removably secure the drug delivery device to a surface of the patient's mouth; (ii) a mechanical pump as characterized in the claims; and (iii) an oral liquid impermeable drug reservoir, the volume of the drug reservoir being from 0.1 mL to 5 mL (e.g., 0.1 mL to 1 mL, 1 mL to 2 mL, 2 mL to 3.5 mL, or 3.5 mL to 5 mL) as characterized in the claims. The mechanical pump is selected from: a spring, an elastomer, compressed gas, and a propellant. In still other embodiments, the oral liquid impermeable reservoir includes one or more of: metal reservoirs, plastic reservoirs, elastomeric reservoirs, metallic barrier layers, valves, squeegees, baffles, rotating augers, rotating drums, propellants, pneumatic pumps, diaphragm pumps, hydrophobic materials, and/or hydrophobic fluids. In some embodiments, the device is configured such that 4 hours after inserting a drug delivery device including a fresh reservoir in a patient's mouth and initiating the administration, less than 5%, 3%, or 1% by weight of the drug-including solid or drug-including fluid in the reservoir includes an oral liquid. In still other embodiments, the oral liquid impermeable drug reservoir includes a fluidic channel in a spiral configuration. The device further includes a suction-induced flow limiter as characterized in the claims and optionally an automatic stop/start, a temperature-induced flow limiter, a pressure-invariant mechanical pump, or bite-resistant structural supports.

In any of the above devices, the pump is a mechanical pump. The mechanical pump can be an elastomeric drug pump. The elastomeric drug pump can include an elastomeric balloon, an elastomeric band, or a compressed elastomer. The mechanical pump can be a spring-driven pump. The spring-driven pump can include a constant force spring. The spring-driven pump can include a spring that retracts upon relaxation. The mechanical pump can be a negative pressure pump (e.g., a pneumatic pump or a gas-driven pump). For example, a gas driven pump can include a gas in a first compartment and the drug in a second compartment, the gas providing a pressure exceeding 101,325 kPa (1 atm), 121.59 kPa (1.2 atm), or 151,988 kPa (1.5 atm). The gas-driven pump can include a compressed gas cartridge. In particular embodiments, the gas driven pump includes a gas, the volume of the gas being less than 35%, 25%, 15%, or 10% of the volume of the pharmaceutical composition. In other embodiments, the gas driven pump includes a gas generator. In some embodiments, the gas driven pump is a propellant-driven pump. The propellant-driven pump can include a fluid propellant, the fluid propellant having a boiling point of less than 37 °C at 101,325 kPa (1 atm). The fluid propellant can be a hydrocarbon, a halocarbon, a hydrofluoralkane, an ester, or an ether (e.g., 1-fluorobutane, 2-fluorobutane, 1,2-difluoroethane, methyl ethyl ether, 2-butene, butane, 1-fluoropropane, 1-butene, 2-fluoropropane, 1,1-difluoroethane, cyclopropene, propane, propene, diethyl ether, 1,1,1,2 tetrafluoroethane, 1,1,1,2,3,3,3 heptafluoropropane, 1,1,1,3,3,3 hexafluoropropane, octafluorocyclobutane or isopentane).

In an embodiment of any of the above devices, the drug delivery device can include two or more drug pumps and/or two or more drug reservoirs. In particular embodiments, the oral liquid impermeable reservoir is substantially impermeable to oxygen gas.

In another embodiment of any of the above devices, the drug reservoir includes a pharmaceutical composition and the pharmaceutical composition occupies greater than 33% (e.g., 33% to 70%, 50% to 80%, 66% to 90%) of the total volume of the drug reservoir and pump. The total volume of the one or more drug reservoirs and the one or more drug pumps can be less than 5 mL, 3 mL, or 2 mL.

In an embodiment of any of the above devices, the device is configured to be secured to the surface of one or more teeth of the patient. The fastener can include a band, a bracket, a clasp, a splint, or a retainer. In particular embodiments, the fastener includes a transparent retainer. For example, the fastener can include a partial retainer attached to fewer than 5 teeth, 4 teeth, or 3 teeth.

In another embodiment of any of the above devices, the drug delivery device includes one or more drug reservoirs and one or more pumps, and the drug reservoirs or the pumps are configured to be worn in the buccal vestibule.

In one embodiment of any of the above devices, the drug delivery device includes one or more drug reservoirs and one or more pumps, and the drug reservoirs or the pumps are configured to be worn on the lingual side of the teeth.

In still another embodiment of any of the above devices, the drug delivery device includes one or more drug reservoirs and one or more pumps, and the drug reservoirs or the pumps are configured to be worn simultaneously in the buccal vestibule and on the lingual side of the teeth.

In another embodiment of any of the above devices, the drug delivery device includes one or more drug reservoirs and one or more pumps, and the drug reservoirs or the pumps are configured bilaterally.

In still another embodiment of any of the above devices, the drug delivery device includes one or more drug reservoirs and one or more pumps, and the drug reservoirs or the pumps are configured to administer the pharmaceutical composition into the mouth of the patient on the lingual side of the teeth. For example, the drug delivery device can include a fluidic channel from the buccal side to the lingual side of the patient's teeth for dispensing the pharmaceutical composition.

In another embodiment of any of the above devices, the drug delivery device includes a fluidic channel in the fastener through which the pharmaceutical composition is administered into the mouth of the patient. For example, the drug delivery device can include a leak-free fluidic connector for direct or indirect fluidic connection of the fastener to the one or more drug reservoirs. The drug delivery device can include a flow restrictor in the fastener for controlling the flow of the pharmaceutical composition.

In another embodiment of any of the above devices, the drug delivery device includes a power source.

In an embodiment of any of the above devices, the drug reservoir is in fluid communication with a tube, channel, or orifice of less than 4 cm, 3 cm, 2 cm, 1 cm, 0.5 cm, or 0.2 cm length and the shear viscosity of the pharmaceutical composition is greater than about 0,05, 0,5, 5, 50 Pa·s (50, 500, 5,000, or 50,000 cP) (e.g., from 0,05 to 5 Pa·s (50 to 5000 cP) or from 5 to 50 Pa·s (5,000 to 50,000 cP), or from 50 to 200 Pa·s (50,000 to 200,000 cP)), and where the device is configured to administer the drug via the tube, channel, or orifice. In particular embodiments, the tube, channel, or orifice has a minimum internal diameter of greater than about 1 mm, 2 mm, 3 mm, 4mm, or 5mm (e.g., 1 to 3 mm, 2 to 4 mm, 3 to 6 mm, or 5 to 15 mm).

In any of the above devices, the drug delivery device includes a flow restrictor that sets the administration rate of the pharmaceutical composition. For example, the length of the flow restrictor can set the administration rate of the pharmaceutical composition. In particular embodiments, the flow restrictor is flared.

In an embodiment of any of the above devices, the drug delivery device is configured to deliver an average rate of volume of from about 0.015 mL/hour to about 1.25 mL/hour over a period of from about 4 hours to about 168 hours at 37 °C and a constant pressure of 89,632 kPa (13 psia), wherein the average rate varies by less than ± 20% or ± 10% per hour over a period of 4 or more hours. The drug delivery device can include oral fluid contacting surfaces that are compatible with the oral fluids, such that the average rate of delivery of the drug increases or decreases by less than ± 20% or ± 10% per hour after the device is immersed for five minutes in a stirred physiological saline solution at about 37 °C including any one of the following conditions, as compared to an identical drug delivery device immersed for five minutes in a physiological saline solution of pH 7 at 37 °C: (a) pH of about 2.5; (b) pH of about 9.0; (c) 5% by weight olive oil; and (d) 5% by weight ethanol.

In an embodiment of any of the above devices, the drug delivery device is configured to deliver an average rate of volume of from about 0.015 mL/hour to about 1.25 mL/hour over a period of from about 4 hours to about 168 hours at 37 °C and a constant pressure of 89,632 kPa (13 psia), wherein the volume is administered at the average rate in less than about 60, 30, or 10 minutes after the first insertion of the device into the patient's mouth.

In an embodiment of any of the above devices, the drug reservoir includes a suspension including at 37 °C solid particles of the drug, a concentration of the drug greater than about 2 M (e.g., 2 to 5 M), and a viscosity of the pharmaceutical composition in the drug reservoir of greater than about 1 Pa·s (1,000 cP) (e.g., 1 to 200 Pa·s (1,000 cP to 200,000 cP)). The drug delivery device can further include a suspension flow-enhancement element. The suspension flow enhancement element can be selected from: a drug with a multimodal particle size distribution wherein the ratio of the average particle diameters for the peaks is in the range of 3:1 to 7:1; a drug with a packing density in the range of 0.64 - 0.70; lubricants, glidants, anti-adhesives, or wetting agents; and modification of the surface properties of the fluidic channel to enhance the flow of particles. In particular embodiments, the suspension flow enhancement element includes a flared orifice, tube, or flow restrictor. In other embodiments, the suspension flow enhancement element includes an orifice, tube or flow restrictor minimum inner diameter at least 10 times greater than the maximum effective particle size. In certain embodiments, the suspension flow enhancement element includes pumping the suspension at a pressure of less than 1000 kPa (10 bars). The viscosity of the suspension can be greater than about 10 Pa·s (10,000 cP). In certain embodiments, the suspension includes a fluid carrier including an oil.

In any of the above devices, the drug reservoir includes a pharmaceutical composition and the pharmaceutical composition includes a drug. For example, the drug reservoir can include a pill, tablet, pellet, capsule, particle, microparticle, granule, or powder. In particular embodiments, the drug reservoir includes extruded and spheronized particles, or particles generated by spray drying, Wurster coating, or granulation and milling.
The solid optionally further includes a disintegrant. In particular embodiments, the pharmaceutical composition includes from 50% to 100% or from 75% to 100% (w/w) drug. In one embodiment, the drug reservoir does not include a fluid. In another embodiment, the drug reservoir includes a solid drug pharmaceutical composition and an aqueous or non-aqueous liquid (e.g., an edible non-aqueous liquid, such as a lubricant or oil). The non-aqeous, edible liquid can substantially reduce contact of the solid drug in the drug reservoir with saliva when the device resides in the mouth of a patient.

In another embodiment of any of the above devices, the drug reservoir includes a fluid including a drug (e.g., where the shear viscosity of the fluid is between about 0,01 Pa·s (10 cP) and about 50 Pa·s (50,000 cP) at 37 °C, for example between about 0,01 Pa s (10 cP) and about 1 Pa s (1,000 cP) at 37 °C, or between about 1-10 Pa·s (1,000-10,000 cP) at 37 °C, or between about 10 Pa·s (10,000 cP) and about 50 Pa·s (50,000 cP) at 37 °C. In certain embodiments, the drug reservoir can have a fluid wherein the volume fraction of the optionally solid drug or drugs is greater than 0.2, 0.4, 0.6, or 0.8. The fluid can include an aqueous solution. In another embodiment, the fluid includes a non-aqueous liquid. In particular embodiments, the fluid includes a supersaturated solution of the drug, an emulsion, a liposome including the drug, a suspension (e.g., an aqueous Newtonian suspension, an aqueous shear-thinning suspension, or an aqueous shear-thickening suspension). The fluid can be a drug suspension including a non-aqueous suspension in low molecular weight PEG, propylene glycol, glycerin, or non-digested oil. The fluid can be a drug suspension including a non-aqueous suspension in an edible oil. The fluid can be a drug suspension including a nanosuspension. The fluid can be a drug suspension including a temperature sensitive suspension (e.g., a suspension in cocoa butter, butter, in a low melting range edible oil, in a low melting range non-digested oil, or in a PEG blend). In particular embodiments, the fluid flows at 37 ± 2°C and solidifies below about 35 °C (e.g., at 33 °C). In some embodiments, the drug in the fluid includes levodopa or a levodopa prodrug.

In particular embodiments, the drug delivery device includes at least one drug chosen from the group of dopamine agonists, cyclosporine, tacrolimus, oxcarbazepine, capecitabine, 5-fluorouracil prodrugs, bupivacaine, fentanyl, quinidine, prazosin, zaleplon, baclofen, ACE inhibitors, ARB blockers, beta-lactams and cephalosporins, anti-epileptics, or any other drug or formulation described herein. For example, the drug delivery device can have a reservoir that includes a total of greater than 1 millimole of levodopa or a levodopa prodrug (when filled) (e.g. 1 to 10 millimoles, 5 to 20 millimoles, or 10 to 25 millimoles). Optionally, the reservoir further contains greater than 0.10 millimoles of carbidopa, a carbidopa prodrug, benserazide, or another DDC inhibitor (e.g. 0.1 to 1 millimoles, 0.5 to 2 millimoles, or 1 to 2.5 millimoles); and/or a COMT inhibitor (e.g., entacapone, tolcapone or opicapone); and/or a drug to treat gastroparesis (e.g., domperidone, nizatidine, monapride or cisapride); and/or an MAO-B inhibitor; and/or an adenosine A2 receptor antagonist.

In another embodiment of any of the above devices, the device includes a drug reservoir, the drug reservoir includes a suspension of drug particles; and the device also includes a fluidic channel or orifice for dispensing of the pharmaceutical composition, wherein the drug particle diameters at all maxima of the particle size distribution are smaller than 1/5^{th}, or smaller than 1/10^{th}, of the narrowest internal diameter segment of the fluidic channel or orifice.

In another embodiment of any of the above devices, the drug reservoir includes a suspension in oil of more than 500 mg levodopa per mL, or more than 500 mg of levodopa and carbidopa per mL e.g., 500 to 1,000 mg/mL); or including more than 600 mg levodopa per mL, or more than 600 mg of levodopa and carbidopa per mL; or including more than 700 mg levodopa per mL, or more than 700 mg of levodopa and carbidopa per mL; or including more than 800 mg levodopa per mL, or more than 800 mg of levodopa and carbidopa per mL. The suspension can maintain a substantially uniform solid drug concentration in the oil for at least 16 hours at 37°C, when flowing at an average rate of 0.02 - 0.25 mL per hour. In particular embodiments, the volume fraction of solids in the suspension of drug particles is greater than 0.65 or 0.75, e.g., 0.65 to 0.8. The suspension can include a non-aqueous carrier fluid.

In particular embodiments, the drug delivery device has a reservoir that includes a formulated drug product including a solid and not including a fluid. For example, the formulated drug product can include a solid selected from one or more pills, tablets, pellets, capsules, particles, microparticles, granules, or powders including the drug. Optionally, the formulated drug product is 50% to 100% (w/w) drug. For example, the formulated drug product can include extruded and spheronized particles, or particles generated by spray drying, Wurster coating, or granulation and milling.

In still other embodiments, the drug delivery device has a reservoir that includes a formulated drug product including a fluid. The fluid can have a shear viscosity at 37 °C that is 0,01-50 Pa·s (10-50,000 cP), that is 0,01-1 Pa·s (10-1,000 cP), that is 1-50 Pa·s (1,000-50,000 cP), or that is greater than 50 Pa·s (50,000 cP). In particular embodiments, the fluid includes an aqueous solution or suspension of the drug. In still other embodiments, the fluid includes a non-aqueous solution or suspension of the drug. In some embodiments, the fluid includes a suspension of the drug; a supersaturated solution of the drug; an emulsion including the drug; or a liposome including the drug. For example, when the formulated drug product includes a suspension, the suspension can include an aqueous Newtonian suspension; an aqueous shear-thinning suspension; an aqueous shear-thickening suspension; a non-aqueous suspension in low molecular weight PEG, propylene glycol, glycerin, edible oil, or medicinal paraffin oil; a nanosuspension; or a temperature sensitive suspension (e.g., a suspension in cocoa butter, butter, in a low melting range edible oil, in a low melting range non-digested oil, or in a PEG blend).

In one particular embodiment of the drug delivery devices of the invention, the device includes an indicator of: the quantity remaining of one or more drugs; the administration time remaining until empty; and/or that one or more of the drug reservoirs should be replaced.

In still other embodiments of the drug delivery devices of the invention, the drug exits the device through one or more orifices that are at least 0.25 cm from the nearest tooth; or the drug exits the device through one or more orifices that are at least 0.25 cm from the nearest gum surface or cheek surface.

The drug delivery device can be configured to deliver a bolus of less than 5% of the contents of a fresh drug reservoir, when immersed for five minutes or for one minute in a stirred physiological saline solution at about 55 °C, as compared to an identical drug delivery device immersed for the same period of time in a physiological saline solution of pH 7 at 37 °C.

In one particular embodiment of the drug delivery devices of the invention, the one or more drug reservoirs are able to withstand a bite from the patient with a force of at least 200 Newtons, without rupturing and without administering a bolus of greater than 5% of the drug contents, when the one or more reservoirs are newly inserted into the mouth.

In another particular embodiment of the drug delivery devices of the invention, the device is configured to deliver a bolus of less than about 5% of the contents of a fresh drug reservoir, when the device is sucked on by a patient for a period of about one minute, as compared to an identical drug delivery device at atmospheric pressure.

In general, for suspensions continuously delivered in the mouth, a high volume fraction of solids can be advantageous both because the volume is reduced and because settling, i.e., sedimentation, leading to an undesired solid drug concentration difference, is slowed. The inventors have discovered that orally deliverable oil-based suspensions, such as edible oil, e.g., vegetable oil based suspensions, can contain more than 600 mg LD per mL, such as more than 700 mg per mL, for example 800 mg LD per mL or more, yet the suspensions can be pumped. Their apparent viscosity can be lower, i.e., their apparent fluidity can be greater, than that of water-based suspensions with similarly high LD concentrations. For example a suspension of about 800 mg/mL levodopa in edible oil can be poured, and can be honey-like in its apparent viscosity at about 25°C. Because LD is more soluble in water than in oils, oil-based LD suspensions have the advantage of their solid or dissolved LD being less saliva-extracted than LD in suspensions made with water or aqueous solution. When an oil based suspension flows into the mouth the risk of leaching by saliva of yet undelivered LD is reduced. The oil-wetted drug is shielded against extraction by saliva, reducing the risk of excess dosing or accidental overdosing.

Optionally the suspensions can also comprise solid carbidopa. When containing solid carbidopa, the sum of the weights of levodopa and carbidopa per mL can be greater than 600 mg per mL, such as more than 650 mg per mL, for example more than 800 mg per mL. The weight fraction of the solid drug or drugs in the suspension can be greater than 0.6. When made with an edible oil, or paraffin oil, or a butter like cocoa butter that is solid at or above 25°C, for example at about 33°C, but is liquid at 37°C, concentrated solid drug suspensions, e.g., of LD, or of LD and carbidopa, can have low apparent viscosities. Because of the typically greater than 3 M suspended solid drug concentration, such as greater than 4 M suspended solid drug concentration, the volume of the drug suspension in the reservoir in the mouth can be small; for example, a daily dose of 1,000 mg of LD can be accommodated in a reservoir of less than 1.25 mL. Because oil can lubricate, i.e., reduce the friction, between flowing solid drug particles suspended in the oil, and also between the particles and the wall of a flow-channel, use of oil-based suspensions can reduce the pressure required for pumping at a particular flow rate. Typical flow rates for the edible oil, paraffin oil, or molten cocoa butter based suspensions can be between about 0.03 mL per hour and about 0.25 mL per hour.

Oil based suspensions can be also physically stable, i.e., sufficiently slowly sedimenting and maintaining the uniformity of their solid drug concentrations for at least 16 hours at 37°C, and can be fluid enough to allow their re-suspension for re-establishing a uniform solid drug concentration after 3 months, 6 months, or longer than 6 month storage at about 25°C. Particularly stable are dispersions of solid drug particles in lipids, including butters like cocoa butter, that are solid at their about 25°C storage temperature, while fluid when heated to within the melting range of their mixture of constuents after being placed in the mouth.

Adding of lubricants to suspensions, e.g., where the weight fraction of the solid drug is greater than about 0.6 can facilitate the movement of the suspension. The suspensions can be pumped, for example, by slippage or by a combination of flow and slippage. Slippage means that parts of the suspension, or even all of the suspension move, e.g., through a flow-controlling tube or orifice as a unit or as multiple units, each unit a plastically deformable block such as a cylindrical block. The movement, i.e., flow of the block or blocks can be retarded by friction between the moving block and the wall of the flow-controlling tube. The lubricant can reduce the friction and facilitate the flow. To facilitate the flow, a surface active food additive can be added. The surface active food additive can have a polar or a non-polar head and a long non-polar carbon chain, typically comprising between 8 and 22 carbon atoms. The surface active food additive can comprise, for example, a fatty acid monoester of glycerol, such as glyceryl monooleate or glyceryl stearate, or stearyl alcohol or cetyl alcohol.

In an embodiment of any of the above devices, the reservoirs are in fluid communication with a tube, channel, or orifice of less than 4 cm, 3 cm, 2 cm, 1 cm, 0.5 cm, or 0.2 cm length and the shear viscosity of the fluid including a drug is greater than about 0,05, 0,5, 5, 50 Pa·s (50, 500, 5,000, 50,000 cP), or that is greater than 50 Pa·s (50,000 cP), and where the device is configured to administer drug via the tube, channel, or orifice. In some embodiments, the tube, channel, or orifice has a minimum internal diameter of greater than about 1 mm, 2 mm, 3 mm, 4mm, or 5mm. In particular embodiments, the drug delivery device includes a first drug reservoir or drug pump on the left side of the mouth, and a second drug reservoir or drug pump on the right side of the mouth, wherein the first drug reservoir or drug pump and the second drug reservoir or drug pump are in fluidic contact through the device. The drug delivery device includes a flow restrictor that sets the infusion rate of the one or more drugs. For example, the length of the flow restrictor sets the infusion rate of the one or more drugs. The drug delivery device can include a fastener and one, two, or more fluidic channels in the fastener through which the fluid including a drug is delivered into the mouth of the patient. The drug delivery device can include a fastener and one, two, or more leak-free fluidic connectors for direct or indirect fluidic connection of the fastener to the one or more drug reservoirs. The drug delivery device can include a fastener and one, two, or more flow restrictors in the fastener for controlling the flow of the fluid. In particular embodiments, the drug delivery device includes a reservoir having a volume fraction of drug that is greater than 20 volume % of the fluid, greater than 40 volume % of the fluid, greater than 60 volume % of the fluid, or greater than 80 volume % of the fluid.

Described herein is also a method of manufacturing the drug delivery device of the invention, wherein the infusion rate of the drug is set by cutting the flow restrictor to a predetermined length.

In particular embodiments, the device of the invention includes one or more oral liquid impermeable drug reservoirs of 0.1-5 mL volume (e.g., 0.1-1 mL, 0.5-3 mL, or 3-5 mL), the reservoirs including one or more drugs, the device configured and arranged to continuously or semi-continuously administer the drugs into the patient's mouth at an average rate for a delivery period of not less than 4 hours and not more than 7 days (e.g., 8, 16, 24, 48, or 72 hours). The device may be configured and arranged to continuously or semi-continuously administer the drug into the patient's mouth at an average rate for a delivery period of not less than 4 hours and not more than 7 days at a rate in the range of 80% - 120% of the average rate in less than about 60, 30, or 10 minutes after the first insertion of the device into the patient's mouth. The one or more drug reservoirs can include an oral liquid impermeable reservoir or a reservoir that is permeable to oral liquids. The device includes one or more drug pumps. The device includes one or more drug reservoirs. The drug pump may contain the drug reservoir. The drug may be in a solid formulated drug product or a fluid formulated drug product. The administered volume or the administered weight of the drug may vary by less than ± 20% or ± 10% per hour over a period of 4 or more hours.

The reservoir can be substantially impermeable to gaseous or dissolved oxygen. In particular embodiments, the volume fraction of the fluid including the drug is greater than 1/3^{rd}, ½ , or 2/3^{rd} of the total volume of the drug delivery device. The total volume of the one or more drug reservoirs and the one or more drug pumps may be less than 7.5, 5, 3, or 2.5 mL.

The solid or fluid delivery device can be configured to deliver a bolus of less than 5% of the contents of a fresh drug reservoir, when immersed for five minutes or for one minute in a stirred physiological saline solution at about 55 °C, as compared to an identical drug delivery device immersed for the same period of time in a physiological saline solution of pH 7 at 37 °C. The drug delivery device can be configured to deliver a bolus of less than about 5, 3 or 1 % of the contents of a fresh drug reservoir, when the device is sucked on by a patient for a period of about one minute, as compared to an identical drug delivery device at atmospheric pressure. The drug delivery device includes one or more components that are configured and arranged to be removably secured to a surface of the patient's mouth. In particular embodiments, the drug delivery device is of a shape and size that cannot be accidentally swallowed by the patient. In some embodiments, the drug delivery device is configured for insertion proximal to a cheek or both cheeks.

In some embodiments, the drug delivery device includes two or more drug reservoirs, each of the drug reservoirs including a solid or fluid including a drug. Optionally, the two reservoirs are bridged. The bridge can optionally provide a fluidic connection between the reservoirs. The devices of the invention can be configured to provide a delivery period of 8 hours, 16 hours, 24 hours, or longer.

The drug delivery device can be a solid drug delivery device, configured and arranged to be removably inserted in a patient's mouth by the patient or caregiver, including an oral liquid impermeable reservoir of 0.1-5 mL volume (e.g., 0.1-1 mL, 0.5-3 mL, or 3-5 mL), the reservoir including a solid including a drug, wherein the drug remaining in the reservoir remains dry or substantially free of oral fluids after 4, 8, 16, 24, or 72 hours of continuous or semi-continuous drug administration into the mouth. In particular embodiments, the invention features one or more valves, squeegees, baffles, rotating augers, rotating drums, propellants, pneumatic pumps, diaphragm pumps, hydrophobic materials, and/or hydrophobic fluids that serve to keep liquids such as saliva, drinks (e.g., water, alcohol, etc.) or liquids in foods (e.g., vegetable oils, etc.) away from the dry drug. In other embodiments, the invention features multiple doses of solid drug within one or more impermeable reservoirs or compartments. In some embodiments, liquid from the mouth makes up less than 5%, 3%, or 1% of the weight of the drug-including solid in the reservoir after 4, 8, 16, 24, 48 or 72 hours of use in the mouth.

The drug delivery device can be a fluid drug delivering device, configured and arranged to be removably inserted in a patient's mouth by the patient or the caregiver, including an oral liquid impermeable reservoir of 0.1-5 mL volume (e.g., 0.1-1 mL, 0.5-3 mL, or 3-5 mL), the reservoir including a fluid including a drug, wherein the fluid drug remaining in the reservoir remains substantially free of oral fluids after 4, 8, 16, 24, or 72 hours of continuous or semi-continuous drug administration into the mouth. In particular embodiments, the invention features one or more valves, propellants, pneumatic pumps, diaphragm pumps, hydrophobic materials, and/or hydrophobic fluids that serve to keep liquids such as saliva, drinks (e.g., water, alcohol, etc.) or liquids in foods (e.g., vegetable oils, etc.) away from the fluid containing the drug. In other embodiments, the invention features multiple doses of fluid drug within the reservoir with each dose in one or more separate, impermeable reservoirs or compartments. In some embodiments, liquid from the mouth makes up less than 5%, 3%, or 1% of the weight of the drug-including fluid in the reservoir after 4, 8, 16, 24, 48 or 72 hours of use in the mouth.

In all embodiments the solid or fluid drug delivery device residing in the mouth includes a non-electric infusion pump (e.g., an elastomeric infusion pump, a spring-driven infusion pump, a negative pressure infusion pump, or a gas-driven infusion pump). For example, when the device is a gas-driven infusion pump, the pump can contain a volatile liquid residing in the drug compartment itself or in another compartment. Typically the volatile liquid is immiscible with the drug formulation, meaning that the solubility of the immiscible liquid in the aqueous or the non-aqueous drug formulation is less than about 20%, 10%, 3%, or 1% (w/w), and/or the solubilities of the major components of the drug formulation in the volatile liquid are less than about 20%, 10%, 3%, or 1% (w/w), the major components being the drug itself (like LD or LD prodrug) and the liquid in which the drug is dispersed or dissolved (such as water, or propylene glycol, or glycerol or alcohol). Typically, the volatile liquid boils at sea-level atmospheric pressure at a temperature below 37°C (i.e., the vapor pressure of the liquid is greater than 100 kPa (1 bar) at 37 °C). The volatile liquid can occupy less than 35% of the volume of the drug formulation in the reservoir (e.g., less than 25%, 15%, or 5% of the volume). Table 1 lists examples of propellant liquids and their approximate vapor pressures at 37 °C.

Because segregation or separation of the liquid propellant and the drug formulation could lead to oral delivery of propellant-enriched fluid and to lesser than intended dosing, the liquid propellant can be co-dispersed in the drug formulation. The propellant liquid can be present, for example, as an oil-in-water emulsion, formed optionally by adding an emulsifier, such as a lecithin, or by Pickering emulsification, where small solid drug or other particles stabilize the emulsion. In general, the emulsions are stable for at least 24 hours and can be re-formed by agitation, for example by shaking. The optionally oil-in-water emulsions can be foamable or non-foamable and can include an emulsifier such as lecithin, a protein, or a surfactant that can be non-ionic, including for example a Tween or polysorbate. Examples of emulsifiers in propellant including mixtures are listed for example in U.S. Patent No. 6,511,655 and in U.S. Patent Publication No. 2003/0049214. Alternatively the liquid propellant can be dissolved in the carrier liquid of a solid drug comprising formulation, e.g., when the carrier liquid is non-aqueous, for example when it is edible oil or medicinal paraffin oil. The propellant dissolving carrier liquid may optionally be a temperature sensitive liquid such as cocoa butter.

Optionally, the volatile liquid propellant pressurizing the gas-driven infusion pump is a hydrocarbon, a halocarbon, a hydrofluoroalkane, an ester or an ether, such as trans-dimethylcyclopropane; isopentane; perfluoropentane; 1-pentene; methyl formate; 1,1-dichloroethylene; isopentane; neopentane; cyclobutane; 1,1-dichloro-1-fluoroethane; cis-1-chloropropene; 1-fluorobutane; 2-methyl-1,3-butadiene; diethyl ether; ethylcyclopropane; n-pentane; trans-2-pentene; or 1,1,1,2-tetrafluoroethane in a first compartment of the reservoir and the drug in a second compartment of the reservoir, the volatile liquid providing a pressure exceeding 101,325 kPa (1 atm), the volatile liquid boiling at sea level atmospheric pressure and a temperature less than 37°C. In particular embodiments, the propellant is selected from 1-fluorobutane, 2-fluorobutane, 1,2-difluoroethane, methyl ethyl ether, 2-butene, butane, 1-fluoropropane, 1-butene, 2-fluoropropane, 1,1-difluoroethane, cyclopropene, propane, propene, diethyl ether, octafluorocyclobutane, isopentane, 1,1,1,3,3,3 hexafluoropropane, 1,1,1,2 tetrafluoroethane, and 1,1,1,2,3,3,3 heptafluoropropane.

Typically, the vapor pressure of the pressurizing liquid is about constant during the infusion of the drug, meaning that the pressure remains about unchanged at 37°C after part of the propellant evaporates.

In some embodiments of the invention the drug can be delivered in solid form. For example, the solid may be delivered by mechanical (e.g., spring-driven for dispensing pills, tablets or solid drug-coated ingestible non-toxic strips) pumps. The non-toxic strips including particles of the solid drug can be, for example, cellulosic (i.e., including cellulose or a derivative of cellulose) or they can include polylactic acid (also known as polylactate) degraded to lactic acid.

The solid drug may be dispensed into the mouth, where it is swallowed. The solid drug may disintegrate or disperse in the mouth, and may include disintegration aids or dispersion aids.

The solid or fluid drug delivery device of the invention can be configured and arranged such that drug administration from the device can be temporarily stopped by the patient for a period of time (i.e., stopped by the patient deploying a switch). For example, the drug delivery device can be configured and arranged such that drug delivery is temporarily stopped while one or more components of the device are removed from the mouth of the patient. In certain embodiments, the drug delivery device is configured and arranged such that drug delivery is temporarily stopped while the drug reservoir and/or the pump are not secured to a surface within the mouth of the patient. In other embodiments, the drug delivery device is configured and arranged such that drug delivery is temporarily stopped while two or more components of the device are disconnected. In another embodiment, the drug delivery is temporarily stopped by the installation of a cap.

The invention features compositions of carbidopa that minimize the amount of toxic hydrazine. The invention includes an oral liquid impermeable reservoir containing a suspension of CD in a fluid volume of 0.20 - 5.0 mL, wherein the concentration of CD suspended in the fluid is 50 - 500 mg/mL. The invention features a CD suspension including less than about 4, 1, or 0.25 mg of hydrazine per 500 mg of CD after the suspension has been stored at 5 °C for 1 year, or at 25 °C for 3 months, 6 months, 12 months, or 24 months. The invention features a CD suspension including less than about 1 ppm of hydrazine when the drug reservoir has been stored at 5 °C for 1 year, or at 25 °C for 3 months, 6 months, 12 months, or 24 months.

In an embodiment of any of the above devices, the volume of the device is less than 7.5 mL, less than 5 mL, or less than 3 mL.

In any of the above solid or fluid drug delivery devices, the device can include an indicator of: the quantity remaining of one or more drugs; the infusion time remaining until empty; and/or that one or more of the drug reservoirs should be replaced.

In any of the above solid or fluid drug delivery devices, the drug can exit the device through one or more orifices that are at least 0.25 cm from the nearest tooth, or from the nearest gum surface or cheek surface.

In any of the above fluid delivery devices, the drug-including fluid can exit the device into the mouth through a tube, channel, or orifice of less than 4 cm, 3 cm, 2 cm, 1 cm, 0.5 cm, or 0.2 cm length, wherein the shear viscosity of the fluid including a drug is greater than about 0,05, 0,5, 5, 50, 500 Pa·s (50 cP, 500 cP, 5,000 cP, 50,000 cP, or 500,000 cP).

In any of the above fluid delivery devices, the drug-including fluid can exit the device into the mouth through a tube, channel, or orifice having an internal diameter greater than about 1 mm, 2 mm, 3 mm, 4mm or 5mm.

The orifice can be optionally of a flared shape. The flare can have an angle of less than 90 degrees versus the axis of the orifice.

In any of the above fluid delivery devices, the device can be configured and arranged to infuse the fluid including one or more drugs from the one or more drug reservoirs into the mouth at an hourly rate in the range of 0.015 - 0.25 mL/hour, in the range 0.05 - 0.25 mL/hour, in the range of 0.25- 0.5 mL/hour, in the range of 0.5 - 0.75 mL/hour, in the range of 0.75 - 1.0 mL/hour, or in the range of 1.0 - 1.25 mL/hour.

In any of the above solid or fluid drug delivery devices, the device can be configured and arranged to administer the solid or fluid active pharmaceutical ingredient at an average rate of 0.01 - 1 mg per hour, 1 - 10 mg per hour, 10 - 100 mg per hour, or greater than 100 mg per hour. In other embodiments, the solid or fluid drug product (i.e., the active pharmaceutical ingredient plus excipients) is delivered at an average rate of 0.01 - 1 mg per hour, 1 - 10 mg per hour, 10 - 100 mg per hour, or greater than 100 mg per hour. In any of the above solid or fluid drug delivery devices, the device can be configured and arranged to administer the drug at least once every 120 minutes, at least once every 90 minutes, at least once every 60 minutes, at least once every 30 minutes, at least once every 15 minutes, at least once every 10 minutes, at least once every 5 minutes or continuously. For delivery at night while the patient is asleep, the device can be configured and arranged to administer the drug at least once every 4 hours, at least once every 2 hours, at least once every hour, more frequently than once per hour, or continuously.

In any of the above solid or fluid drug delivery devices, the one or more drug reservoirs can be configured to withstand a bite from the patient with a force of at least 200 Newtons, without rupturing and without infusing a bolus of greater than 5% of the drug contents, when the one or more reservoirs are newly inserted into the mouth.

In any of the above fluid-delivery devices, the device includes a flow restrictor that sets the infusion rate of the drug (e.g., wherein the length of the flow restrictor sets the infusion rate of the drug, or wherein the infusion rate of the drug is set by cutting the flow restrictor to a predetermined length).

In any of the above solid or fluid drug delivery devices, the reservoir can include two or more drugs.

In any of the above fluid delivery devices, the drug (e.g., LD) or prodrug (e.g., LD prodrug) containing fluid can be aqueous, or non-aqueous, or mixed aqueous-non aqueous. It can contain, for example a non-toxic alcohol, such as propylene glycol, glycerol, sorbitol, or ethanol, an edible oil, an edible lipid melting at or below 37°C, e.g., a butter softening or melting at or below 37°C, or it can be an aqueous solution of a sugar, the sugar concentration exceeding 20 weight percent, for example a solution including greater than 50 weight % sucrose.

In any of the above fluid delivery devices, the fluid can include an aqueous, non-aqueous, or mixed aqueous - non-aqueous suspension of the drug where part or most or essentially all of the drug is solid, i.e., it is not dissolved; or it can be an aqueous solution of the drug, a non-aqueous solution of the drug, a suspension of the drug, a supersaturated solution of the drug, an emulsion including the drug, a liposome including the drug, a fatty acid salt of a LD prodrug, a liquid salt of a LD prodrug, or at least one drug chosen from the group of dopamine agonists, cyclosporine, tacrolimus, oxcarbazepine, capecitabine, 5-fluorouracil prodrugs, bupivacaine, fentanyl, quinidine, prazosin, zaleplon, baclofen, ACE inhibitors, ARB blockers, beta-lactams and cephalosporins. When basic, e.g., because they include heterocyclic nitrogen atoms, the drugs can be optionally administered as their more soluble salts, e.g., hydrochloride salts or carboxylic acid salts.

In any of the above fluid delivery devices, the drug (e.g., LD, CD, or one of their prodrugs) may be formulated using a variety of approaches, including aqueous suspensions (e.g., aqueous Newtonian suspensions, aqueous shear-thinning (pseudoplastic) suspensions, or aqueous shear-thickening (dilatant) suspensions); non-aqueous suspensions (e.g., suspensions in low molecular weight PEG, suspensions in propylene glycol, suspensions in glycerin, suspensions in edible oil, or suspensions in medicinal paraffin oil); nanosuspensions or colloids (e.g., aqueous nanosuspensions or non-aqueous nanosuspensions); temperature sensitive suspensions (e.g., suspensions in cocoa butter, suspensions in low melting range edible oils, suspensions in low softening or melting range non-digested oils, or suspensions in PEG or PEG blends); and microparticulate formulations (e.g., extruded and spheronized particles, particles generated by spray drying, particules generated by Wurster coating, or particles generated by granulation and milling), optionally with the microparticles in a lubricating suspension.

In particular embodiments of any of the above solid or fluid drug delivery devices, the drug in the reservoir residing in the mouth includes a total of greater than 1 millimole of LD. The drug in the reservoir can further include greater than 0.10 millimoles of carbidopa, of a carbidopa prodrug, or of benserazide. The drug in the reservoir can further include a COMT inhibitor (e.g., entacapone, tolcapone or opicapone), a drug to treat gastroparesis (e.g., domperidone, Nizatidine, Relamorelin, Monapride or Cisapride), a MAO-B inhibitor, or an adenosine A2 receptor antagonist. In particular embodiments, the volume fraction of the LD or LD prodrug is greater than 20 volume % of the solid or fluid, greater than 40 volume % of the solid or fluid, greater than 60 volume % of the solid or fluid, or greater than 80 volume % of the solid or fluid. In another embodiment, the reservoir contains a fluid which includes a total of greater than 1 millimole of a LD prodrug (e.g., LDEE, LDME, or a salt thereof) of 0.25M or greater concentration (e.g., greater than or equal to 0.5, 1.0, 1.5, 2.0, 2.5, 3.0, or 3.5 M). In still other embodiments, the pH of the orally infused fluid is about 8 or less, about 7 or less, about 6 or less, about 5 or less, about 4 or less or is 3 or less, or 2 or less.

In some embodiments, the solid or fluid in the intra-oral reservoir further includes a reducing agent in an amount greater than or equal to 0.07 millimoles, greater than or equal to 0.30 millimoles, or present in a sufficient quantity to prevent the discoloration of the mouth by oxidation products of the infused LD, LD prodrugs or DDC inhibitors. The agent can be, for example, ascorbic acid or an ascorbate salt. The added ascorbate or ascorbic acid can be stable in a fluid when a non-toxic diol or a polyol such as propylene glycol, glycerol or sorbitol is added, such that more than half of the ascorbic acid or ascorbate is retained after storage at about 25 °C for at least a week, for example for a month, two months or three months. Typically, the weight ratio of the added diol or polyol to water can be greater than 1:10, for example 1:5, 1:3, 1:2, or 1:1, or from 1:10 to 1:1. In particular embodiments, the formulation includes a diol or polyol, but no water. In some embodiments optionally solid ascorbic acid, sodium ascorbate or another ascorbate salt is co-suspended in a butter like cocoa butter that is solid at or above 25°C, for example at about 33°C, but is liquid at 37°C. Because of the slow diffusion of O₂ in solids and because of the slight solubility of ascorbic acid and ascorbate salts in thermally sensitive edible oil-rich solid compositions that are liquid at body temperature, i.e., near 37°, the expected 25°C shelf lives of the ascorbate or ascorbic acid comprising compositions are expected to be greater than 6 months, e.g., greater than 1 year, e.g., greater than 2 years.

In any of the above fluid delivery devices, the viscosity of the fluid delivered into the mouth at 37 °C is 0,0012-50 Pa·s (1.2-50,000 cP), is 0,002-1 Pa·s (2-1,000 cP), is 1-50 Pa·s (1,000-50,000 cP), or is greater than 50 Pa·s (50,000).

In any of the above devices, the drug delivery device can further include excipients, e.g., taste-modifying excipients to improve the taste of the solid or fluid.

The solid or fluid drug delivery device of the invention may optionally include a fastener for securing the drug delivery device to the teeth of the patient. The fastener, the one or more pumps, and the one or more drug reservoirs may include a single unit, or they may be removably coupled to each other. In certain embodiments, the fastener includes one, two or more drug reservoirs removably secured to the fastener. In particular embodiments, the fastener includes one, two or more pumps of the invention removably secured to the fastener. In some embodiments, the fastener is a retainer including a housing for holding one, two or more drug reservoirs or pumps of the invention. The one, two or more drug reservoirs or the one, two or more pumps can be removably secured in the buccal vesible, on the lingual side of the teeth, in both the buccal vestibule and on the lingual side of the teeth, or removably secured bilaterally. In particular embodiments, the drug delivery device is configured to administer the solid or fluid including a drug into the mouth of the patient on the lingual side of the teeth, optionally through the fastener. The drug delivery device can include one, two, or more fluidic channels through which the fluid including a drug is delivered into the mouth of the patient, optionally through the fastener. The drug delivery device can include one, two, or more leak-free fluidic connectors for direct or indirect connection to one, two, or more drug reservoirs, optionally through the fastener. In particular embodiments, the drug delivery device includes one, two, or more flow restrictors for controlling the flow of the fluid including a drug from the drug reservoirs, optionally in the fastener. The solid or fluid drug delivery device includes a pump mechanism, optionally in the fastener. The pump can be removable and disposable or alternatively it can be integrated into the fastener such that some, or all, of the fastener is re-usable for some period of time (e.g., one week, one month, six months, or one year).

In a related aspect, the invention features a pharmaceutical composition including a suspension containing a drug suitable for continuous or frequent intermittent intra-oral delivery, the suspension including at 37 °C solid particles of the drug, a concentration of the drug greater than 2 M (e.g., 2 to 5 M), and a viscosity of greater than 10 Pa·s (100 Poise), wherein the suspension remains free of sedimented solid drug for 6 months or more, wherein said drug is levodopa, a levodopa prodrug, baclofen, or a pharmaceutically acceptable salt thereof, and wherein said suspension comprises a non-aqueous carrier fluid.. In particular embodiments, the weight fraction of solid drug particles having maximal diameters that are smaller than 5 micrometers and that are larger than 0.5 micrometers is greater than 50% (e.g., 50% to 70%, 60% to 80%, or 70% to 90%). In certain embodiments, the solid drug particle maximal diameters are bimodally or multimodally distributed. The pharmaceutical composition includes a non-aqueous carrier. In particular embodiments, the density of the aqueous carrier is greater than 1.2 g cm⁻³ (e.g., 1.2 to 2.2 g cm⁻³). The particles include baclofen, levodopa or a pharmaceutically acceptable salt thereof or a levodopa prodrug.

In particular embodiments, the fluid includes low molecular weight PEG, propylene glycol, glycerin, or non-digested oil. In other embodiments, the fluid includes an edible oil.

The invention features the pharmaceutical composition for continuous or semi-continuous intraoral administration including a suspension in oil of more than 500 mg levodopa per mL, or more than 500 mg of levodopa and carbidopa per mL (e.g., 500 to 1,000 mg/mL); or including more than 600 mg levodopa per mL, or more than 600 mg of levodopa and carbidopa per mL; or including more than 700 mg levodopa per mL, or more than 700 mg of levodopa and carbidopa per mL; or including more than 800 mg levodopa per mL, or more than 800 mg of levodopa and carbidopa per mL. In particular embodiments, the suspension maintains a substantially uniform solid drug concentration in the oil for at least 16 hours at 37°C, when flowing at an average rate of 0.02 - 0.25 mL per hour.

The invention features the pharmaceutical composition for continuous or semi-continuous intraoral administration including a suspension of drug particles wherein the volume fraction of solids is greater than 0.65 (e.g., greater than0.75, or is between 0.65 to 0.8). The suspension includes a non-aqueous carrier fluid (e.g., an oil). In particular embodiments, the suspension includes bimodally or multimodally distributed drug particle sizes. In one particular embodiment, (a) the weight based amount of the larger drug particles equals or exceeds that of the smaller drug particles when the particle size distribution is bimodal, and (b) the weight based amount of the largest drug particles equals or exceeds that of the smallest drug particles, when the particle size distribution is multimodal. In certain embodiments, the large drug particle:small drug particle weight ratio is between 1.2 and 1.8. The pharmaceutical composition can further include a lubricant and/or a temperature sensitive suspension. In certain embodiments, the pharmaceutical composition has substantially no taste when continuously infused into the mouth at a rate of 0.125 mL per hour. In other embodiments, the suspension maintains a substantially uniform solid drug concentration in the suspending fluid when stored for at least 6 months at about 25°C. In still other embodiments, the pharmaceutical composition has a shear viscosity of 10 Pa s-50 Pa·s (500 Poise). In some embodiements, the suspension: (i) maintains a non-uniform solid drug concentration in the suspending fluid when stored for at least 6 months at about 25°C, and subsequently (ii) a substantially uniform solid drug concentration is achieved when the pharmaceutical composition is shaken by hand for a period of about 60 seconds.

The invention features the pharmaceutical composition for continuous or semi-continuous intraoral administration including a suspension in an oil carrier wherein the sum of the concentrations of the solid drug particles is greater than 3 M, and wherein the uniformity of its drug concentration is maintained within about +/- 10 %, when flowing for 8 hours or more at a flow rate between 0.02 mL/hour and 0.25 mL/hour.

The invention features the pharmaceutical composition comprising a temperature-sensitive suspension of levodopa or a levodopa prodrug. Tthe concentration of the levodopa or levodopa prodrug can be 500 mg/mL or greater. In particular embodiments, the pharmaceutical composition includes cocoa butter. The pharmaceutical composition can be a solid or semi-solid at 5 °C, 25 °C, or 33 °C.

In another aspect, the invention features the device of the invention including the pharmaceutical composition of the invention.

In a related aspect, the invention features a pharmaceutical composition for use in a method of administering said pharmaceutical composition to a patient, the method including removably attaching the device of the invention to an intraoral surface of the patient. The method can further include detaching the device from the intraoral surface. In one particular embodiment, the method includes administering the drug to the patient for a delivery period of not less than about 4 hours, 6 hours, 8 hours, or 12 hours and/or not more than about 4 days, 5 days, or 7 days. In particular embodiments, the device includes a drug reservoir containing a volume of drug and the method includes oral administration at a rate in the range of from 15 microliters per hour to about 1.25 mL per hour during the delivery period. In some embodiments, the fluctuation index of the drug is less than or equal to 2.0, 1.5, 1.0, 0.75, 0.50, 0.25, or 0.15 during the delivery period. The method can include oral administration at a rate in the range of from about 0.015 mL/hour to about 0.25 mL/hour; from about 0.25 mL/hour to about 0.5 mL/hour; from about 0.5 mL/hour to about 0.75 mL/hour; from about 0.75 mL/hour to about 1.0 mL/hour; or from about 1.0 mL/hour to about 1.25 mL/hour. In particular embodiments, the device includes a drug reservoir containing a pharmaceutical composition including a drug and the drug is administered to the patient at an average rate of not less than 0.01 mg per hour and not more than 125 mg per hour (e.g., 0.01 - 1 mg per hour, 10 - 100 mg per hour, or greater than 100 mg per hour). In one embodiment of the delivery method, the pharmaceutical composition is administered to the patient at least once every 60 minutes, at least once every 30 minutes, at least once every 15 minutes, or is administered to the patient continuously. In particular embodiments, the delivery period is at least 4 8, 16, 24, or two days. In one particular embodiments, the device includes a drug reservoir containing a fluid pharmaceutical composition including a drug, wherein the fluid pharmaceutical composition flows at 37 ± 2°C and and is solid or semi-solid at 5 °C, 25 °C, or 33 °C, the method further including stopping the flow of the fluid pharmaceutical composition by lowering the temperature the drug reservoir. Lowering the temperature can include lowering the temperature the drug reservoir to ambient temperature, such as by immersing the drug reservoir in water. In any of the above delivery methods, the method can further include treating a disease in the patient, wherein the disease is selected from: anesthesia, bacterial infections, cancer, pain, organ transplantation, disordered sleep, epilepsy and seizures, anxiety, mood disorders, post-traumatic stress disorder, cancer, arrhythmia, hypertension, heart failure, spasticity, and diabetic nephropathy. For example, the method can further include treating Parkinson's disease, wherein the drug is levodopa or a levodopa prodrug.

Described herein is also a method for treating a disease in a patient, the method including: (a) inserting into the mouth of the patient the drug delivery device of the invention; (b) administering the drug from the reservoir into the mouth of the patient for a period of at least 4 hours; and (c) removing the drug delivery device from the mouth.

Described herein is also a method for treating a disease in a patient, the method including: (a) inserting a drug delivery device into the patient's mouth; (b) starting a drug administration from the device; (c) administering into the patient's mouth one or more drugs, using continuous administration or semi-continuous administration, for a period of 4 hours to 7 days at an hourly rate in the range of 0.015 - 1.25 mL/hour or 0.01 - 100 mg/hour; and (d) removing the drug delivery device from the mouth, wherein the drug delivery device includes a oral liquid impermeable reservoir of 0.1-5 mL volume (e.g., 0.1-1 mL, 0.5-3 mL, or 3-5 mL), and the reservoir including a solid or fluid including a drug.

Described herein is also a method for treating a disease in a patient, the method including: (a) inserting a drug delivery device into the patient's mouth; (b) starting a drug administration from the device; (c) administering into the patient's mouth one or more drugs, using continuous administration or semi-continuous administration, at an hourly rate in the range of 0.015 - 1.25 mL/hour or 0.01 - 125 mg/hour; (d) removing the drug delivery device from the mouth; and (e) stopping the drug delivery from the device; wherein (i) the drug delivery device includes a reservoir of 0.1-5 mL volume (e.g., 0.1-1 mL, 0.5-3 mL, or 3-5 mL), and the reservoir including a solid or fluid including a drug; and (ii) steps a, b, c, d and e are performed at least twice over a period of 4 hours to - 7 days.

In any of the above methods, the drug can include dopamine agonists, cyclosporine, tacrolimus, oxcarbazepine, capecitabine, 5-fluorouracil prodrugs, bupivacaine, fentanyl, quinidine, prazosin, zaleplon, baclofen, ACE inhibitors, ARB blockers, beta-lactams or cephalosporins.

In any of the above methods, the disease to be treated can be selected from one or more of anesthesia, bacterial infections, cancer, pain, organ transplantation, disordered sleep, epilepsy and seizures, anxiety, mood disorders, post-traumatic stress disorder, cancer, arrhythmia, hypertension, heart failure, spasticity, dementia, and diabetic nephropathy.

In any of the above methods, the drug can include LD, a LD prodrug, or a salt thereof.

In a particular, the disease to be treated is motor or non-motor complications associated with Parkinson's disease. The motor or non-motor complication can include tremor, akinesia, bradykinesia, dyskinesia, dystonia, cognitive impairment, gastric emptying, retarded gastrointestinal transit, and/or disordered sleep.

The invention further features a drug delivery device for treating Parkinson's disease in a patient as characterized in the claims Said device being designed for: (a) removably inserting into the patient's mouth, the drug delivery device including a oral liquid impermeable reservoir of 0.1-5 mL volume (e.g., 0.1-1 mL, 0.5-3 mL, or 3-5 mL), and the reservoir including a solid or fluid including a total of greater than 1 millimole of LD or a LD prodrug; (b) administering into the patient's mouth the solid or fluid for a period of at least 8 hours at an hourly rate in the range of 0.03 - 1.25 mL/hour or 20 - 125 mg/hour of LD, such that a circulating plasma LD concentration greater than 400 ng/mL and less than 7,500 ng/mL is continuously maintained for a period of at least 8 hours during the infusion; and (c) removing the drug delivery device from the mouth.

The invention further features a drug delivery device for treating Parkinson's disease in a patient as characterized in the claims, the device being designed for: (a) inserting the drug delivery device of the invention into the patient's mouth, the device having a drug reservoir including levodopa or a levodopa prodrug; (b) administering into the patient's mouth the levodopa or a levodopa prodrug for a period of at least 8 hours at an hourly rate in the range of 10 - 125 mg/hour, such that a circulating plasma LD concentration greater than 1,200 ng/mL and less than 2,500 ng/mL is continuously maintained for a period of at least 8 hours during the administration; and (c) removing the drug delivery device from the mouth.

In particular embodiments of the drug delivery device for treating Parkinson's disease, the fluctuation index of LD is less than or equal to 2.0, 1.5, 1.0, 0.75, 0.50, 0.25, or 0.15 for a period of at least 8 hours during the administration. In particular embodiments, during the administration the circulating LD plasma concentration varies by less than +/- 20% or +/- 10% from its mean for a period of at least 1 hour.

In another aspect, the invention features a drug delivery device for treating Parkinson's disease in a patient, the method including continuous or semi-continuous administration of the pharmaceutical composition of the invention into the patient at a rate of 10 - 125 mg/hour for a period of from about 4 hours to about 168 hours.

In particular embodiments of the drug delivery device for treating Parkinson's disease, the disease is a motor or non-motor complication of Parkinson's disease (e.g., tremor, akinesia, bradykinesia, dyskinesia, dystonia, cognitive impairment, or disordered sleep).

In particular embodiments, a circulating plasma LD concentration greater than 400 ng/mL is achieved within 60 minutes of the initiation of the administration; a circulating plasma LD concentration greater than 800 ng/mL is continuously maintained for a period of at least 8 hours during the administration; a circulating plasma LD concentration greater than 1,200 ng/mL is continuously maintained for a period of at least 8 hours during the administration; a circulating plasma LD concentration greater than 1,600 ng/mL is continuously maintained for a period of at least 8 hours during the administration; a circulating plasma LD concentration greater than 2,000 ng/mL is continuously maintained for a period of at least 8 hours during the administration; a circulating plasma LD concentration greater than 800 ng/mL is achieved within 60 minutes of the initiation of the administration; a circulating plasma LD concentration greater than 1,200 ng/mL is achieved within 60 minutes of the initiation of the administration; a circulating plasma LD concentration greater than 1,600 ng/mL is achieved within 60 minutes of the initiation of the administration; a circulating plasma LD concentration greater than 2,000 ng/mL is achieved within 60 minutes of the initiation of the administration; a circulating plasma LD concentration less than 5,000 ng/mL is continuously maintained for a period of at least 8 hours during the administration; a circulating plasma LD concentration less than 3,500 ng/mL is continuously maintained for a period of at least 8 hours during the administration; or a circulating plasma LD concentration less than 2,500 ng/mL is continuously maintained for a period of at least 8 hours during the administration. In particular embodiments, a circulating plasma LD prodrug concentration less than 100 ng/mL is continuously maintained for a period of at least 8 hours during the administration; a circulating plasma LD prodrug concentration less than 50 ng/mL is continuously maintained for a period of at least 8 hours during the administration; a circulating plasma LD prodrug concentration less than 25 ng/mL is continuously maintained for a period of at least 8 hours during the administration. In particular embodiments, during the administration the circulating LD plasma concentration varies by less than +/-20%, or by less than +/- 10%, from its mean for a period of at least 1 hour.

In a related aspect, the invention features a stable, infusible pharmaceutical composition including a suspension of carbidopa in a fluid at a concentration of 50 mg/mL to 500 mg/mL, wherein the amount of hydrazine is less than 1 mg of hydrazine per 500 mg of CD after storage at 25°C for a period of 3 months and wherein said fluid comprises a non-aqueous carrier fluid, an oil, or an edible oil.

### ABBREVIATIONS AND DEFINITIONS

The term "about," as used herein, refers to a number that is ±10% of a value that this term precedes.

The term "administration" or "administering" refers to a method of giving a dosage of a therapeutic drug, such as LD or a LD prodrug to a patient. The drug may be formulated as a solid or a fluid. Fluids may be infused. The dosage form of the invention is preferably administered into the mouth or nasal cavity, optionally using a drug delivery device such as a solid drug dispenser, an infusion pump or an osmotic device, and the drug can be absorbed anywhere within the mouth, nasal cavity, or alimentary canal, e.g., buccally, sublingually, or via the stomach, small intestine, or large intestine. Typical durations of administration from a single device or drug reservoir are greater than 4, 8, 12, or 16 hours per day, up to and including 24 hours per day. Administration can also take place over multiple days from a single device or drug reservoir, e.g., administration of a drug for 2 or more days, 4 or more days, or 7 or more days.

As used herein, "aqueous" refers to formulations of the invention including greater than 10% or 20 % (w/w) water and, optionally, a cosolvent (e.g., propylene glycol, glycerol or ethanol) or solute (e.g., a sugar).

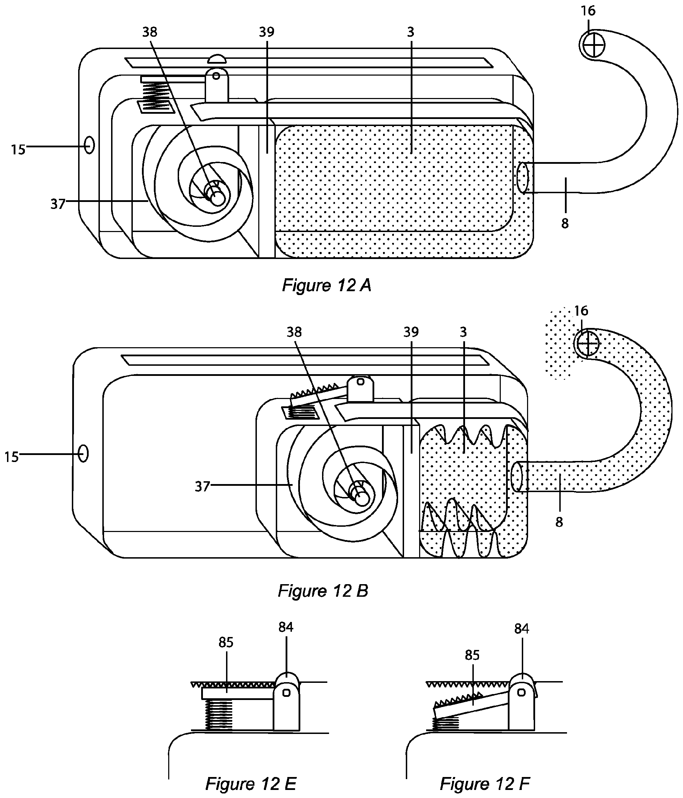

The term "automatic stop/start," as used herein, refers to an element switching automatically between drug administering mode and non-administering mode upon actuation by an external stimulus (e.g., detachment of the device of the invention from an intraoral surface). Automatic stop/start encompasses automatically stopping delivery, automatically starting delivery, or both. For example, the automatic stop/start can be a pressure sensitive switch, a clip, a fluidic channel that kinks, a clutch (see Figures 12E and 12F).

The term "bite-resistant structural supports," as used herein, refers to structural elements in the drug delivery device that enable them to withstand a patient's bite with a force of at least 200 Newtons, without rupturing and without infusing a bolus of greater than 5% of the drug contents, when a fresh reservoir is newly inserted into the mouth.

The term "carbidopa prodrug" refers to carbidopa esters, carbidopa amides, and salts thereof, such as the hydrochloride salt of carbidopa ethyl ester, carbidopa methyl ester, or carbidopa amide.

The term "CD" refers to Carbidopa.

As used herein, "co-administered" or "co-infused" refers to two or more pharmaceutically active agents, formulated together or separately, and administered or infused into the mouth simultaneously or within less than 60, 30, 15, or 5 minutes of each other.

The term "COMT" refers to catechol-O-methyl transferase.

As used herein "continuous administration" or "continuous infusion" refers to uninterrupted administration or infusion of a drug in solid or fluid form.

The term "DDC" refers to DOPA decarboxylase.

As used herein the term "drug" refers also to its pharmaceutically acceptable salts when the active ingredient is an acid or a base. It can be, for example, a hydrochloride or a maleate of a base or a sodium salt of an acid.

As used herein, the terms "effective particle size" and "particle size" are used interchangeably and refer to a mixture of particles having a distribution in which 50% of the particles are below and 50% of the particles are above a defined measurement. The "effective particle size" refers to the volume-weighted median diameter as measured by a laser/light scattering method or equivalent, wherein 50% of the particles have a smaller diameter, while 50% have a larger diameter. The effective particle size can be measured by conventional particle size measuring techniques well known to those skilled in the art. Such techniques include, for example, optical microscopy, electron microscopy, sedimentation, field flow fractionation, photon correlation spectroscopy, light scattering (e.g., with a Microtrac UPA 150), laser diffraction, and centrifugation.

As used herein the term "emulsion" refers to a fluid comprising at about 37°C an aqueous and oil or organic phase, such as milk comprising butterfat and water. An emulsion may remain homogeneous, i.e., it may not substantially phase separate in 2 days at 25°C or in 1 day at 37°C. The term encompasses oil in water emulsions and water in oil emulsions.

As used herein, the term "fastener" refers to an element for attaching the device of the invention, or its components, to a surface of the mouth (e.g., to the teeth). Exemplary methods of attachment are fasteners banded, adhered, cemented or glued to one, two or more teeth; dental appliances; splints; transparent retainers; metal wire Hawley retainers; partial retainers on one side of the mouth (e.g., attached to 3, 4, or 5 teeth); thermo or vacuum-formed Essix retainers typically including a polypropylene or polyvinylchloride material, typically .020" or .030" thick; thermo-formed Zendura retainers including polyurethane; bonded (fixed) retainers including a passive wire bonded to the tongue-side of lower or upper teeth; muco-adhesives that adhere to the oral mucosal tissue and slowly erode; and fasteners that conform or are molded to fit a patient's teeth or soft tissue, similar to dental splints used to treat bruxism and sleep apnea. Similarly, the drug delivery devices, drug pumps, drug reservoirs and other devices of the invention may be directly or indirectly attached to a removable denture, a prosthetic tooth crown, a dental bridge, a moral band, a bracket, a mouth guard, or a dental implant.

As used herein the term "fluctuation index" refers to the magnitude of the rise and fall of drug level in plasma relative to the average plasma concentration, and is defined as [Cₘₐₓ₋Cₘᵢₙ]/ C_{avg}. The fluctuation index is measured over a specified period of time. The time period can begin, for example, after the drug's plasma concentration: has reached the steady-state concentration; has reached 90% of the steady-state concentration; or 30, 60, or 120 minutes after any of the drug delivery devices of the invention has been inserted into the mouth and begun to deliver drug. The time period can end, for example: at the end of the use period specified in the instructions for use of the drug delivery device; when the drug reservoir is 90% depleted or substantially depeleted; or about 4, 8, 16, 24, 72, or 168 hours after the start of the time period.

As used herein, the term "fluid" encompasses any drug-including liquid or gel that can be pumped. The fluid can be a Newtonian or a non-Newtonian fluid. It can be, for example, a viscous Newtonian or non-Newtonian suspension. The term encompasses, for example, true solutions, colloidal solutions, emulsions, pastes, suspensions, and dense semi-solid toothpaste-like suspensions deforming under pressure sufficiently to be extruded into the mouth. The fluid infused can be aqueous, non-aqueous, single phase, two-phase, three- phase or multiphase. The emulsions can be, for example, oil-in-water or water-in-oil, and can include micelles and/or liposomes.

As used herein, "formulated drug product" refers to the drug together with its excipients and fluid carrier, if any.

As used herein, "infused" or "infusion" includes infusion into any part of the body, preferably infusion into the mouth or nasal cavity.

The term "LD" refers to levodopa, also known as L-DOPA, or a salt thereof.

The term "LDEE" refers to levodopa ethyl ester, or a salt thereof.

The term "LDME" refers to levodopa methyl ester, or a salt thereof.

The term "LD prodrug" refers to a pharmaceutical composition suitable for infusion, preferably for infusion into the mouth, forming LD upon its hydrolysis. Examples include levodopa amides, levodopa esters, levodopa carboxamides, levodopa sulfonamide, levodopa ethyl ester, and levodopa methyl ester, and their salts. The salts are usually formed, in the cases of levodopa esters and levodopa amides, by neutralizing their basic amines with an acid; and, in the cases of levodopa carboxamides and levodopa sulfonamide, by neutralizing their carboxylic acids or sulfonic acids with a base. Examples of infusible LD prodrugs are provided in patent applications WO 2012/079072 and WO 2013/184646.

The term "MAO-B" refers to monoamine oxidase-B.

As used herein, "mouth" includes the areas of the oral cavity, including those areas of the oral cavity adjacent the lips, cheeks, gums, teeth, tongue, roof of the mouth, hard palate, soft palate, tonsils, uvula, and glands.

The term "non-aqueous" refers to the liquid carrier in formulations. The non-aqueous liquid carrier typically melts or softens below 37°C and contains less than 20 % (w/w) water (e.g., less than 10%, 5%, 3%, 2%, 1.5%, 1%, 0.5%, or less than 0.1% (w/w). Exemplary liquid carriers include alcohols, lipids, edible oils, butters, and paraffin oils melting or softening below 37°C.

As used herein, the term "operational life" means the time period during which the solid or the infusion solution containing the drug (e.g., LD or LD prodrug) is suitable for delivery into a patient, under actual delivery conditions. The operational life of the drugs (e.g., LD or LD prodrugs) delivered by the devices of the invention can be greater than 12 hours, 24 hours, 48 hours, 72 hours, 96 hours (4 days), or 7 days. It typically requires that the product is not frozen or refrigerated. The product is typically infused at or near body temperature (about 37°C).

As used herein, a "oral liquid impermeable reservoir" means a reservoir including one or more drugs to be administered into the patient's mouth, wherein 1, 4, 8, 16, 24, 48 or 72 hours after placing a drug delivery device including a fresh reservoir in a patient's mouth and initiating the administration, less than 5%, 3%, or 1% by weight of the drug-including solid or drug-including fluid in the reservoir includes an oral liquid. The one or more drugs may be in solid form or in fluid form. Oral liquids include saliva, water, water-diluted alcohol and other fluids commonly found in the mouth or that are drunk by the patient. Exemplary oral liquid impermeable reservoirs can be made of a metal, or a plastic that can be elastomeric. Metallic reservoirs can include, for example aluminum, magnesium, titanium or iron alloys of these. When made of a plastic it can have a metallic barrier layer; or include plastics or elastomers that do not substantially swell in water, used for example for packaging of food, or for drink-containing bottles, or in a fabric of washable clothing (e.g., polyamides like Nylon or polyesters like Dacron), or in stoppers or seals of drink containing bottles, or in septums of vials containing solutions of drugs. Examples include polyolephins like polyethylene and polypropylene; other vinylic polymers like polystyrene and polyvinylchloride; polyvinylidene chloride, polyacrylates and polymethacrylates, e.g., polymethyl methacrylate and polymethyl acrylate; and polycarbonates; and polysilicones or their copolymers. Ingress of oral liquids into openings in the reservoir can be prevented or minimized by the use of one or more valves, squeegees, baffles, rotating augers, rotating drums, propellants, pneumatic pumps, diaphragm pumps, hydrophobic materials, and/or hydrophobic fluids. In some embodiments, the invention features multiple doses of solid drug within multiple, impermeable reservoirs or compartments.

The abbreviation "M" means moles per liter. Usage of the term does not imply, as it often does in chemistry, that the drug is dissolved. As used herein 1 M means that a 1 liter volume contains 1 mole of the combination of the undissolved (often solid) and/or the dissolved drug. For example, 1 M LD means that there is 197 mg of solid (undissolved) and dissolved LD in one mL.

The term "PD" refers to Parkinson's disease.

The term "PEG" refers to polyethylene glycol.

As used herein, the term "pH" refers to the pH measured using a pH meter having a glass electrode connected to an electronic meter.

The term "pressure-invariant pump," as used herein, refers to a pump whose average rate of drug delivery increases or decreases by less than about 20%, 10%, or 5% at 101,353 kPa (14.7 psia) and at 77,911 kPa (11.3 psia), as compared to its average rate of delivery at 89,632 kPa (13.0 psia).

As used herein, "pump" refers to any mechanism capable of administering a solid or fluid formulated drug product over a period of 4 or more hours. Examples of pumps according to the present invention include mechanical devices with moving parts that are not battery-powered (e.g., gas-driven pumps, spring-driven pumps, shape memory alloy driven pumps, and elastomeric pumps),.

The terms "semi-continuous administration" and "frequent administration," as used interchangeably herein, refer to an administration (e.g., infusion) of a drug in solid or fluid form at a frequency of at least once every 120 minutes, and preferably at least every 90, 60, 30, 15, or 5 minutes.

As used herein, the term "shelf life" means the shelf life of the drug delivered by the inventive device (e.g., LD or LD prodrug), in its form as a product sold for use by consumers, during which period the product is suitable for use by a patient. The shelf life of the drugs (e.g., LD or LD prodrugs) administered by the devices of the invention can be greater than 3, 6, 12, 18, or preferably 24 months. The shelf life may be achieved when the product is stored frozen (e.g., at about -18 °C), stored refrigerated (at about 5 ± 3 °C, for example at about 4 ± 2 °C), or stored at room temperature (e.g., at about 25°C). The drug (e.g., LD or LD prodrug) product sold to consumers may be the drug-containing solid or fluid, e.g., suspension or solution ready for infusion, or it may be its components. For example, the LD prodrug product for use by consumers may be the dry solid LD prodrug and, optionally, the solution used for its reconstitution; or the LD prodrug stored in an acidic solution; etc.

As used herein, a "solid drug" refers to one or more drugs formulated in solid dose forms, as opposed to a solution or suspension. The solid may be in the form of pills, tablets, pellets, spheres, capsules, particles, microparticles (e.g., made by extrusion/spheronization), granules, powders, coatings of plastic (e.g., cellulosic or polylactic acid) strips, or other similar solid dosage forms known in the art. The solid drug formulation may include additional excipients, such as binders, disintegrants, glidants, lubricants, taste modifiers, etc. The solid drug may be dry or may be surrounded by a fluid, such as an aqueous or non-aqueous lubricant. The solid drug formulation may include a single solid, multiple disceet solids, or a large number of discreet solids (e.g., a powder). For example, to dose LD/CD every 15 minutes over a period of 16 hours, the solid may include 64 individual solid pills, spheres, tablets or capsules, with one solid administered at each dosing. The solids of the invention may include 1 - 1,000 discreet solids, e.g., 1, 2, 3, 4, 2-10, 11-50, 51-100, 101-500, 501-1,000, or 4-1,000 discreet solids. In the case of a powder, the solids of the invention may include greater than 1,000 discreet solids. To minimize the volume of the delivered solids, in preferred formulations the one or more drugs (e.g., LD/CD) includes greater than 50%, 60%, 70%, 80%, 90%, or 95% by weight of the solid, with excipients making up the balance. The drug-including solid may be carried on, incorporated in, or integrated with an edible or non-toxic plastic, such as polylactic acid, alginic acid or any alginate, e.g., calcium alginate, chitin, chitosan, gelatin, starch or its amylose and amylopectin, or cellulose and its derivatives.

As used herein, "stable" refers to stable formulations of any of the drugs administered by the devices of the invention. Stable formulations exhibit a reduced susceptibility to chemical transformation (e.g., oxidation and/or hydrolysis) prior to administration into a patient. Stable drug formulations have a shelf life of equal to or greater than 3, 6, 12, 18, or 24 months, and an operational life of greater than or equal to 8 hours, 12 hours, 16 hours, 24 hours, 48 hours, 72 hours, 96 hours, or 7 days. In the context of LD and/or CD containing formulations, "stable" refers to formulations which are oxidatively stable, and optionally, physically stable. Oxidatively stable formulations are those having a shelf life during which less than 20%, 10%, 5%, 4%, 3%, 2% or less than 1% of the LD and/or CD is oxidized when stored for a period of 3, 6, 12, 18, or 24 months. Optionally, for LD and/or CD-containing formulations such as viscous suspensions, the term "stable" may also refer to formulations that substantially retain their homogeneity when stored without agitation, such as shaking, for at least 2 hours, for example for at least 4 hours, 8 hours, 24 hours, or 3 days. For example, stable suspensions may not separate to form two or more fluids differing in their LD or CD concentrations by more than 20%, 10% or 5 %. In the context of LD prodrugs, "stable" refers to formulations that are "oxidatively stable" and "hydrolytically stable." Stable solid or liquid formulations of LD prodrugs are those having a shelf life during which less than 10%, 5%, 4%, 3%, 2% or less than 1% of the LD prodrug is oxidized or hydrolyzed when stored for a period of 3, 6, 12, 18, or 24 months. In general, solutions of the stable LD prodrug formulations remain clear, meaning that they have no substantial visible precipitate, after their storage. Stable solid or liquid formulations of LD prodrugs have an operational life during which less than 10%, 5%, 4%, 3%, 2% or less than 1% of the LD prodrug is oxidized or hydrolyzed over a period of 8 hours, 12 hours, 16 hours, 24 hours, 48 hours, 72 hours, 96 hours, or 7 days. An "oxidatively stable" LD prodrug formulation exhibits a reduced susceptibility to oxidation during its shelf life and/or its operational life, during which less than 10%, 5%, 4%, 3%, or less than 2% of the LD prodrug is oxidized. A "hydrolytically stable" LD prodrug formulation exhibits a reduced susceptibility to hydrolysis during its shelf life and/or operational life in which less than 20%, 10%, 5%, 4%, 3%, 2% or less than 1% of the LD prodrug is hydrolyzed.

As used herein, "substantially free of LD precipitate" refers to solutions of LD prodrugs that are clear and without a visible precipitate of LD.

As used herein, "substantially free of oxygen" refers to compositions packaged in a container for storage or for use wherein the packaged compositions are largely free of oxygen gas (e.g., less than 10%, or less than 5%, of the gas that is in contact with the composition is oxygen gas) or wherein the partial pressure of the oxygen is less than 1999836 kPa (15 torr), 1333224 kPa (10 torr), or 0,667 kPa (5 torr). This can be accomplished by, for example, replacing a part or all of the ambient air in the container with an inert atmosphere, such as nitrogen, carbon dioxide, argon, or neon, or by packaging the composition in a container under a vacuum.

As used herein, "substantially free of water" refers to compositions packaged in a container (e.g., a cartridge) for storage or for use wherein the packaged compositions are largely free of water (e.g., less than 2%, 1%, 0.5%, 0.1%, 0.05%, or less than 0.01 % (w/w) of the composition is water). This can be accomplished by, for example, drying the constituents of the formulation prior to sealing the container.

The term "suction-induced flow limiter," as used herein, refers to one or more elements preventing the delivery of a bolus greater than about 5, 3, or 1% of the contents of a fresh drug reservoir, when the ambient pressure drops by 13,790 kPa (2 psi) for a period of one minute. The suction-induced flow limiter can include pressurized surfaces that are in fluidic (gas and/or liquid) contact with the ambient atmosphere via one or more ports or openings in the housing of the drug delivery device. Alternatively, the suction-induced flow limiter can be selected from a deformable channel, a deflectable diaphragm, a compliant accumulator, an inline vacuum-relief valve, and a float valve.

As used herein the term "suspension" refers to a mixture comprising at least one liquid carrier and particles of at least one solid. The liquid carrier can be aqueous or non-aqueous, e.g., edible oil based. A suspension may remain homogeneous, i.e., its solid particles may not substantially sediment or float in 2 days at 25°C or in 1 day at 37°C. Alternatively, its solid particles may sediment or float after standing for 2 days at 25°C or for 1 day at 37°C, but the solid particles may be re-suspended by agitation, e.g., by shaking. Suspensions may be, for example, free flowing suspensions or plug flow pastes with slip between tube wall and plug.

The term "suspension flow-enhancement element," as used herein, refers to one or more elements that substantially prevent pressure-induced separation of pumped, viscous suspensions, e.g., formulations with particular multimodal particle size distributions, packing densities, and flow-enhancing excipients; flaring of the orifice, tube, or flow restrictor; orifice, tube or flow restrictor inner diameters substantially larger than the maximum effective particle size; and selection of specific combinations of viscosity, orifice/tube inner diameter, particle size, and pressure.

The term "temperature-induced flow limiter," as used herein, refers to one or more elements preventing the delivery of a bolus greater than about 5% of the contents of a fresh drug reservoir, when immersed for five minutes or for one minute in a stirred physiological saline solution at about 55 °C, as compared to an identical drug delivery device immersed for the same duration in a physiological saline solution of pH 7 at 37 °C. The temperature-induced flow limiter can include insulation with a material of low thermal conductivity proximate the drug reservoir and/or the pump. Optionally, the temperature-induced flow limiter includes an elastomer, a spring, or a gas.

As used herein, the term "treating" refers to administering a pharmaceutical composition for prophylactic and/or therapeutic purposes. To "prevent disease" refers to prophylactic treatment of a patient who is not yet ill, but who is susceptible to, or otherwise at risk of, a particular disease. To "treat disease" or use for "therapeutic treatment" refers to administering treatment to a patient already suffering from a disease to ameliorate the disease and improve the patient's condition. The term "treating" also includes treating a patient to delay progression of a disease or its symptoms. Thus, in the claims and embodiments, treating is the administration to a patient either for therapeutic or prophylactic purposes.

As used herein "viscosity" means dynamic viscosity also known as shear viscosity.

Other features and advantages of the invention will be apparent from the following Detailed Description, the drawings, and the claims.

### BRIEF DESCRIPTION OF THE DRAWINGS

Figure 1A depicts a drug delivery device that is removably attached to a tooth using a fastener 1. The pump **2** and drug reservoir **3** are contained within a housing 4 and are disposable. Figure 1B depicts an embodiment in which a portion **5** of the drug delivery device is reusable, and a removable pump **2** and drug reservoir **3** can be disposable. Figure 1C depicts an embodiment in which a pump **2** and a drug reservoir **3** comprise a single component.

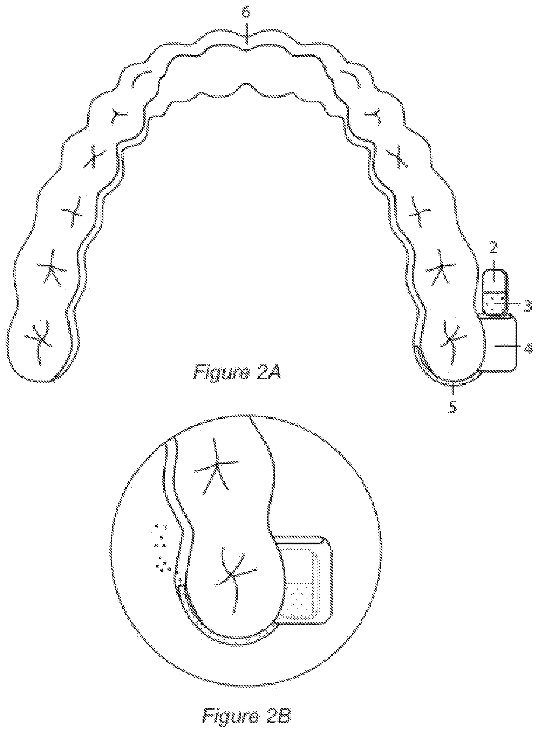

Figure 2A depicts an embodiment of the drug delivery device in which the pump **2** and/or drug reservoir **3** is fastened to either the upper or lower teeth using a transparent retainer **6.** One, two or more pumps and/or one or more drug reservoirs are secured on the buccal side of the transparent retainer **6.** One, two, or more drug pumps and/or drug reservoirs may be secured unilaterally, on either the right or left sides, positioned in the buccal vestibule or, alternatively, on the lingual side of the teeth. Figure 2B is a close up showing the drug pump and reservoir attached to the transparent retainer **6** and dispensing drug to the lingual side of the mouth through a tube **5.**
Figure 3 depicts a drug delivery device in which the pump **2** and drug reservoir **3** are configured to be positioned both on the lingual side of the teeth and in the bucal vestibule. The drug reservoir is fastened on the lingual side of the teeth, while a drug pump and an optional gas pump **11** are positioned on the buccal side of the teeth.
Figure 4A depicts a fastener in the form of a transparent retainer **6,** including two bilateral housings **4** (shown empty) on the buccal side of the teeth into which drug pumps and/or drug reservoirs may be inserted. Figure 4B depicts a fastener in the form of an invisible retainer **6,** including two bilateral housings **4** (shown filled) on the lingual side of the teeth into which drug pumps and/or drug reservoirs **3** have been inserted.
Figures 5A and 5B illustrate a drug delivery device including a pressurized, drug-filled elastomer. The elastomer provides pressure that delivers the drug at a constant rate through a narrow internal diameter tubing, with the rate determined by the properties of the elastomer and the inner diameter of the narrow bore tubing. Figure 5A is a representation of an empty elastomeric drug delivery device, while figure 5B represents a fresh, pressurized, drug-filled elastomeric drug delivery device.
Figures 5C and 5D illustrate an elastomeric band-driven pump employing a rubber band **10** to pull a piston **13** to apply pressure to the drug reservoir **3.**
Figure 6 illustrates a conveyor-belt driven by a spring motor **17** that delivers discreet solid doses **18** (e.g., pills, granules, pellets, particles, etc.) carried on a backing material **19,** through a duckbill valve **22,** into the mouth.
Figure 7 illustrates the use of a spring-driven motor **17** to advance a string **23** to which solid drug **24** is attached (e.g., in the form of multiple discreet solid pills), transporting the solid drug out of the dry oral liquid impermeable drug reservoir **3,** through a duckbill valve **22,** to a position in which it is exposed to saliva in the mouth, where the solid drug can dissolve or disperse.
Figure 8 illustrates a conveyor-belt driven by a spring motor **17** that delivers discreet solid doses (e.g., pills, granules, pellets, particles, etc.) including a series of squeegees **25** that propel the drug from the dry oral liquid impermeable drug reservoir **3,** through a duckbill valve **22,** into the mouth.
Figure 9 illustrates the use of an auger 26 to capture and deliver drug **27** from a drug reservoir **3** and into the mouth.
Figure 10 illustrates the use of a motor to rotate two columnar or conical shaped drums **29** that are attached to the oral liquid impermeable drug reservoir **3.**
Figure 11 illustrates a spring-driven pump employing a spring **33** to push a series of solid drugs doses in the shape of disks **34,** each dose separated by a thin layer of a material **35** that slowly dissolves or disperses in saliva.
Figures 12A, 12B, 12C and 12D illustrate spring-driven pumps in which a constant force spring is used to compress the drug reservoir **3.**
Figures 12E and 12F illustrate a spring loaded clutch mechanism **85** useful in the devices of the invention. The clutch mechanism engages the piston **39** to inhibit the force transmission to the drug reservoir **3** prior to use. When the device is removed from the mouth, the protrusion **84** is disengaged, which stopping the release of drug from the drug reservoir **3.**
Figures 13, 14,15A, and 15B illustrate battery powered drug delivery devices for the delivery of solid dose forms which are not according to the invention and are present for illustration purposes only.
Figure 16 illustrates a disk **54** which contains compartments filled with drug particles **55** that are injected by an air pressure bolus at a pre-determined rate through an orifice **56** that is fixed in place with respect to the rotating disk. The rotation of the disk via a spring mechanism, exposes a single compartment and the bolus of air delivers the drug from that compartment to the mouth.

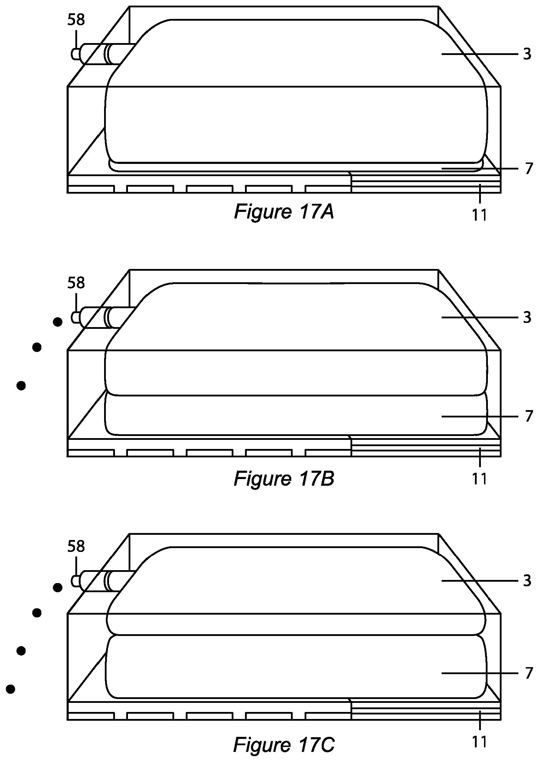

Figures 17A, 17B and 17C illustrate a drug delivery device wherein a first elastomeric drug reservoir **3** is compressed by a second elastomeric reservoir or balloon **7** containing gas or propellant. In Figure 17A, the drug delivery device includes a housing containing a first, full elastomeric drug reservoir **3**; a second empty elastomeric reservoir **7**; and an gas pump **11** and electronics. In one embodiment air is pumped by the electronic (e.g., piezoelectric) pump **11** into the second elastomeric reservoir **7.** The pressure from the second elastomeric reservoir **7** compresses the first elastomeric drug reservoir **3** containing the drug, forcing the drug out of the reservoir through a flow restrictor **58** at a constant rate. Figure 17B illustrates the system with a first, half-full drug reservoir **3** and a second, elastomeric reservoir **7** half-filled with pressurized air. Figure 17C illustrates the system when the drug reservoir **3** is close to empty. In another embodiment, saliva can be pumped by the electronic pump **11** into the second elastomeric reservoir **7.** The devices with electronic pumps are not according to the invention and are shown for illustration purposes only.
Figure 18 shows a schematic of a typical two stage gas pressure regulator.

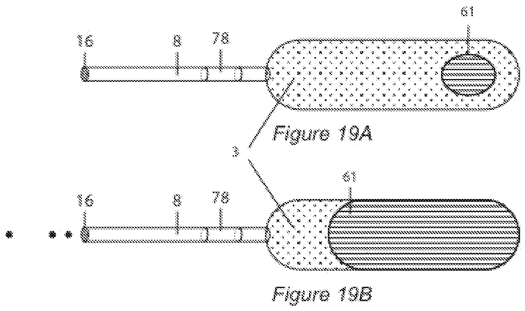

Figures 19A and B illustrate a drug delivery device including an elastomeric compartment **61** containing propellant within a rigid drug reservoir **3.** The propellant within the elastomeric compartment has a vapor pressure that pressurizes the drug compartment at a specific pressure when exposed to body temperature, and pushes the drug through a narrow bore tubing. Figure 19A shows the compressed elastomeric compartment **61** containing propellant within the full drug reservoir **3.** Figure 19B shows the nearly empty drug reservoir **3** and the expanded elastomeric compartment **61** containing propellant.
Figures 20A and 20B illustrate illustrate a propellant-driven drug delivery device for the delivery of solid dose forms.
Figures 20C, 20D, 20E, and 20F illustrate illustrate a propellant-driven drug delivery device for the delivery of suspensions.
Figure 21A depicts a SCT osmotic tablet substantially full of drug. Figure 21B depicts the same SCT osmotic tablet substantially depleted of drug. Figures 21 A and 21 B are not according to the invention and are present for illustration purposes only.
Figure 22A depicts an AMT osmotic tablet substantially full of drug. Figure 22B depicts the same AMT osmotic tablet substantially depleted of drug. Figures 22 A and 22 B are not according to the invention and are present for illustration purposes only.
Figures 23A, 23B, 24A, 24B, 24C, 24D, 25A, 25B, and 25C illustrate mechanisms which make the drug delivery rate of drug delivery devices insensitive to ambient pressure changes in the mouth.
Figures 26A and B are graphs of the temperature in two locations in the mouth after ingestion of a hot beverage.
Figures 27A and B are graphs of the temperature in two locations in the mouth after ingestion of a cold beverage.
Figure 28 illustrates an embodiment of efficient drug packing using drug particles with a tri-modal size distribution.
Figures 29A and B are micrographs depicting L-DOPA particles formed by jet milling to reduce the average size of the particles from 52 µm to 3.4 µm (excluding fines) (see Example 22).
Figure 30 illustrates a method of gravure printing a solid drug composition on a plastic ribbon or sheet.

### DETAILED DESCRIPTION OF THE INVENTION

The present invention relates to the embodiments as characterized in the claims.

The devices, compositions, and methods of the invention are useful for continuous or semi-continuous oral delivery of medicaments.

While syringes, drug reservoirs and pumps outside the mouth can be large because space is typically available, space in the mouth for a drug delivery device is limited and is particularly limited when a drug delivery device is so small that it does not interfere with speaking, swallowing, drinking, or eating. Consequently, the delivered drug, its reservoir and its delivery device must occupy a small volume. In the exemplary management of Parkinson's disease the concentration of the orally infused LD prodrug and/or LD including fluid of the invention can be typically greater than 1 M, such as greater than 1.5 M, 2 M, 2.5 M, 3 M, 3.5 M, 4 M or 4.5 M. These are substantially higher concentrations than the 0.1 M LD concentration of the Duodopa gels that are commercially available for jejunal, gastric or nasogastric infusions. The concentrated drug solution or suspension can be viscous, for example its viscosity at 37°C can be greater than 0,05 Pa·s (50 cP), such as greater than 0,1 Pa·s (100 cP), 0,2 Pa·s (200 cP), 0,5 Pa·s (500 cP), 1 Pa·s (1000 cP), 10 Pa·s (10,000 cP), or 100 Pa·s (100,000 cP) or 1000 Pa·s (1,000,000 cP). It can be a viscous suspension, e.g., toothpaste-like in its viscosity, the viscosity being greater than about 20 Pa·s (20,000 cP), for example greater than 50 Pa·s (50,000 cP). The earlier practice of infusion of viscous fluids through long tubings, typically longer than 50 cm, such as those used for nasogastric, gastric or jejunal infusions, required that their internal diameter be large and/or that the pumping pressure be high. Furthermore, when the earlier suspensions were infused through the longer tubings, the likelihood of blockage of the flow because of clustering of the suspended LD particles increased and translucent, very fine particle colloids were used to reduce blockage. In contrast, the here disclosed orally infused, more much concentrated suspensions of the invention are typically opaque because they can contain large solid particles scattering visible-wavength light. The much more concentrated and much more viscous orally infused suspensions can be rich in particles with dimensions greater than 500 nm, 1 µm or even 5 µm. The suspensions can be orally infused, for example, using orifices in reservoirs that are shorter than 2 mm or 1 mm, and/or through tubings that are typically shorter than 5 cm, e.g., shorter than 4 cm, 3 cm, 2 cm or 1 cm.

Alternatively, the drug can also be administered as a solid. Delivery of solid drug can be done using a mechanical apparatus that is timed to inject one or more discreet solids of a particular weight to provide the appropriate dosage to the patient. For example, for a total dosage of LD of 1500 mg/day, the solid mass could be 20.8 mg, delivered every 15 mins for 18 hours. In this example, the 20.8 mg could include a single solid or multiple solids. To minimize the volume of the delivered solids, in preferred formulations the one or more drugs (e.g., LD/CD) includes greater than 50%, 60%, 70%, 80%, 90%, or 95% by weight of the solid, with other excipients making up the balance.

### MOUTH

The drugs may be administered intraorally (i.e., onto or near any intraoral surface, e.g., the lips, cheeks, gums, teeth, tongue, roof of the mouth, hard palate, soft palate, tonsils, uvula, and glands). The drugs administered intraorally are typically swallowed by the patient, together with the patient's saliva, and can be absorbed in the patient's gastrointestinal tract, e.g., in the small intestines or large intestines. In some cases, absorption of drugs delivered by the drug delivery devices of the invention may take place partially or even primarily through the mucous membranes in the mouth, e.g., buccal or sublingual absorption.

Drugs or formulations which may be irritating to the cheeks or gums are preferably administered a distance away from these surfaces, in order to reduce the concentration of the drug at these surfaces through dilution by saliva and diffusion. Examples of such drugs or formulations can include drugs or formulations that are, for example, acidic, basic or oxidizing. For example, the drug may exit the one or more orifices of the drug delivery device 0.25, 0.5, or 0.75 cm from the nearest gum surface or the nearest cheek surface. In one embodiment, the drug delivery device is secured to the teeth and positioned immediately adjacent to the roof of the mouth; the administered drug exits the delivery device from one or more orifices on the bottom side of the delivery device, proximate the tongue and away from the cheeks, gums, and roof of the mouth. In another embodiment at least part of the drug delivery device, including at least one reservoir, is positioned near a cheek.

Drugs that may cause discoloration or erosion of the teeth are preferably administered a distance away from the teeth in order to reduce their concentration at tooth surfaces through dilution by saliva and diffusion. Examples of such drugs or formulations include drugs or formulations that are protein-reactive, acidic or oxidizing. For example, the drug may exit the drug delivery device via one or more orifices located 0.25, 0.5, or 0.75 cm from the nearest tooth. In one embodiment, the drug delivery device is secured to the teeth and positioned immediately adjacent to the roof of the mouth; the administered drug exits the delivery device via one or more orifices on the bottom side of the delivery device, proximate the tongue and away from the teeth and roof of the mouth.

In an alternative embodiment, the infused drugs may be infused into the nasal cavity from a drug delivery device held in the mouth, via a flexible tube or cannula. When drug is infused into the nasal cavity by the drug delivery devices of the invention, drug absorption is typically primarily through the membranes in the nasal cavity.

### MEDICATIONS AND DISEASES

The devices and methods of the invention are suitable for the administration of a variety of drugs that have a short half-life and/or a narrow therapeutic range. Complementary drugs may be co-administered or co-infused with these drugs. Such complementary drugs may improve the pharmacokinetics, improve the efficacy, and/or reduce the side effects of the primary drugs.

Exemplary diseases/medical conditions that may be treated with the devices and methods of the invention, and corresponding drugs and exemplary ranges of daily doses and of average administration rates, are listed below:
- Parkinson's disease: levodopa, levodopa prodrugs, and dopamine agonists (such as Pramipexole (0.1 - 10 mg per day, 0.004 - 0.42 mg/hr), Bromocriptine, Ropinirole (0.25 - 10 mg per day, 0.01 - 0.42 mg/hr), Lisuride, Rotigotine). Examples of complementary drugs for Parkinson's disease, which may optionally be co-infused, are DDC inhibitors (such as carbidopa, carbidopa prodrugs, and benserazide (50 - 600 mg per day, 2.1 - 25 mg/hr)), COMT inhibitors (such as entacapone, tolcapone and opicapone), MAO-B inhibitors (such as Rasagiline and Selegiline), adenosine A2 receptor antagonists (such as Istradefylline), and gastroparesis drugs (such as Domperidone, Nizatidine, Relamorelin, Monapride and Cisapride).
- Anesthesia: bupivacaine, lidocaine.
- Anxiety: oxcarbazepine (300 - 3,000 mg per day, 12.5 - 125 mg/hr), prazosin (0.2 - 5 mg per day, 0.01 - 0.21 mg/hr).
- Arrhythmia: quinidine (300 - 2,000 mg per day, 12.5 - 83 mg/hr)
- Bacterial infections: beta-lactam antibiotics (e.g., cephalosporins).
- Cancer: capecitabine (1,000 - 10,000 mg per day, 42 - 417 mg/hr) and other 5-fluorouracil prodrugs.
- Dementia: Rivastigmine.
- Diabetes: oral insulins
- Diabetic nephropathy: angiotensin receptor blockers.
- Disordered sleep: Zaleplon (3 - 20 mg per day, 0.38 - 0.83 mg/hr for 8 hours at night), gamma hydroxy butyrate (10 - 200 mg per day, 1.3 - 25 mg/hr for 8 hours at night), Zolpidem (3 - 20 mg per day 0.38 - 0.83 mg/hr for 8 hours at night), triazolam.
- Epilepsy and seizures: Oxcarbazepine (300 - 3,000 mg per day, 12.5 - 125 mg/hr), topiramate (200-500 mg per day, 8.3-20.8 mg/hr), lamotrigine (100-700 mg per day, 4.2-29.2 mg/hr), gabapentin (600-3,600 mg per day, 25-150 mg/hr), carbamazepine (400 - 1,600 mg per day, 16.7 - 66.7 mg/hr), valproic acid (500 - 5,000 mg per day, 20.1 - 208 mg/hr), levetiracetam (1,000 - 3,000 mg per day, 41.7 - 125 mg/hr), pregabalin (150 - 600 mg per day, 6.25 - 25 mg/hr).
- Heart failure: ACE inhibitors, angiotensin receptor blockers.
- Hypertension: Prazosin (0.2 - 5 mg per day, 0.01 - 0.21 mg/hr), ACE inhibitors, angiotensin receptor blockers.
- Mood disorders: Oxcarbazepine (300 - 3,000 mg per day, 12.5 - 125 mg/hr), lithium.
- Organ transplantation: Cyclosporine (150 - 1,500 mg per day, 6.3 - 62.5 mg/hr), Tacrolimus (3 - 25 mg per day, 0.13 - 1.04 mg/hr).
- Pain: Fentanyl (0.05 - 2.0 mg per day, 0.002 - 0.083 mg/hr), Dilaudid (2 - 50 mg per day, 0.83 - 2.1 mg/hr).
- Post-traumatic stress disorder: Prazosin (0.25 - 5 mg per day, 0.01 - 0.21 mg/hr).
- Spasticity: Baclofen.

The drugs and methods of the invention may be used for treating dental and maxillofacial conditions, such as xerostomia, dental caries, local infections (e.g., fluconazole, diflucan, nystatin, or clotrimazole for thrush) in the mouth or throat, and local pain in the mouth or throat (e.g., lidocaine).

Dry mouth (xerostomia) and hyposalivation are more prevalent in older patients and are a common side effect of medications, including medications for the treatment of PD. Patients with PD also commonly experience difficulty swallowing (dysphagia), which often results in drooling (sialorrhea). Drugs for the treatment of xerostomia, hyposalivation, dysphagia and/or sialorrhea may be delivered using the devices and methods of the invention. Examples of drugs for xerostomia and hyposalivation are saliva stimulants such as organic acids (e.g., citric acid, ascorbic acid, malic acid) or their acidic salts and parasympathomimetic drugs (e.g., choline esters such as pilocarpine hydrochloride, and cholinesterase inhibitors). Examples of drugs for dysphagia are Scopolamine, Tropicamide, Glyccopyrolate, and Botulinum Toxin. Examples of drugs for excess salivation are anticholinergics such as glycopyrrolate. In a preferred embodiment, drugs for the treatment of xerostomia, hyposalivation, and/or dysphagia are co-administered with the LD or LD prodrugs, using the drug delivery devices and methods of the invention. In another preferred embodiment, intra-oral administration of an anti-Parkinson's medication itself stimulates increased salivation and/or more frequent or improved swallowing. For example, intra-oral administration of LDEE stimulates salivation and results in more frequent swallowing, as described in the Examples.

Gastroparesis, or delayed gastric emptying, is common in people with PD. Drugs for the treatment of gastroparesis may be delivered using the devices and methods of the invention. In one embodiment, drugs for the treatment of gastroparesis are co-administered with the LD or LD prodrugs, using the drug delivery devices and methods of the invention. In another embodiment, drugs for the treatment of gastroparesis are administered using other methods of drug delivery known in the art (i.e., they are not administered via continuous or frequent intra-oral delivery) while LD or LD prodrugs are infused intra-orally. Examples of drugs for the treatment of gastroparesis are Metoclopramide, Cisapride, Erythromycin, Domperidone, Sildenafil Citrate, Mirtazapine, Nizatidine, Acotiamide, Ghrelin, Levosulpiride, Tegaserod, Buspirone, Clonidine, Relamorelin, Serotonin 5-HT4 agonists and dopamine D2 antagonists.

Methylation of LD, whereby 3-methoxy-L-DOPA (3-OMD) is produced, is one of the major metabolic paths of LD. It increases the amount of LD required by Parkinson's disease patients and because the conversion shortens the half-life of plasma LD, it also increases the frequency at which LD or LD/CD or CD need to be administered in order to manage the symptoms of Parkinson's disease. The conversion of LD to 3-OMD is catalyzed by the enzyme catechol-O-methyl transferase, COMT. Administration of a COMT inhibitor can reduce the required dosage of LD or LD/CD, or in earlier stages of PD, even provide for managing the disease without LD or LD/CD. The two most frequently used COMT inhibitors, entacapone and tolcapone are, however short-lived.

Entacapone does not cross the blood-brain barrier and is less toxic than Tolcapone, which crosses the barrier. The plasma half-life of Entacapone is, however, merely 0.4-0.7 hours, making it difficult to maintain a sufficient plasma level of the drug without administering large and frequent doses of the drug. In clinical practice, one 200 mg tablet is often administered with each LD/CD or LD/Benserazide dose. The maximum recommended dose is 200 mg ten times daily, i.e., 2,000 mg. Continuous oral administration of Entacapone could reduce the dosage and/or frequency of administration of the drug and its side effects. The reduced dosage could alleviate side effects such as dyskinesia and/or gastrointestinal problems, nausea, abdominal pain or diarrhea.

Entacapone could be continuously orally administered in a daily dose of less than 1000 mg per 16 hours while the patient is awake (such as less than 500 mg per 16 awake hours), for example as an aqueous suspension comprising small particles, e.g., less than 100 µm average diameter, such as less than 30 µm, 10 µm, 3 µm or 1 µm particles of Entacapone. Alternatively, it could be administered as a suspension in a non-aqueous solution, such an edible oil, cocoa-butter, propylene glycol, or glycerol.

Tolcapone is a reversible COMT inhibitor of 2-3 hour half-life. It exerts its COMT inhibitory effects in the central nervous system as well as in the periphery. Its use is limited by its hepatotoxicity. The typical dose of Tolcapone in PD management is 100 - 200 mg three times daily. Tolcapone may also be effective in the treatment of Hallucinogen Persisting Perception Disorder, decreasing visual symptoms. Continuous oral administration of Tolcapone could reduce its dosage and/or frequency of administration and its hepatotoxicity. The reduced dosage could alleviate its hepatotoxicity. Its daily dose could be less than 500 mg per 16 awake hours, such as less than 300 mg per 16 awake hours. It could be continuously orally administered, for example, as an aqueous suspension comprising small particles, e.g., less than 100 µm average diameter, such as less than 30 µm, 10 µm, 3 µm or 1 µm particles of the drug. Alternatively, it could be administered as a suspension in a non-aqueous solution, such an edible oil, cocoa-butter, propylene glycol, or glycerol.

Because administration according to this invention is typically into the mouth, it is preferred that the drugs selected for administration are those whose taste is neutral or pleasant, as perceived by a majority of patients. Taste masking or modifying excipients may be added to the formulations of drugs whose taste is unpleasant, as perceived by a majority of patients.

Drugs delivered as solids may be formulated with excipients to increase disintegration or dispersion.

Many types of drugs may be delivered in accordance with the invention. Drugs which may in principle be used for treatment according to the invention are any known drugs, wherein the drugs may be present in the form according to the invention as such, or in the form of the active ingredient, optionally in the form of a pharmaceutically acceptable salt of the active ingredient. Drugs which may be delivered in accordance with the invention include, without limitation, analgesics and antiinflammatory agents (e.g., aloxiprin, auranofin, azapropazone, benorylate, diflunisal, etodolac, fenbufen, fenoprofen calcim, flurbiprofen, ibuprofen, indomethacin, ketoprofen, meclofenamic acid, mefenamic acid, nabumetone, naproxen, oxyphenbutazone, phenylbutazone, piroxicam, sulindac), antihelmintics (e.g., albendazole, bephenium hydroxynaphthoate, cambendazole, dichlorophen, ivermectin, mebendazole, oxamniquine, oxfendazole, oxantel embonate, praziquantel, pyrantel embonate, thiabendazole), anti-arrhythmic agents (e.g., amiodarone HCl, disopyramide, flecainide acetate, quinidine sulphate, anti-bacterial agents (e.g., benethamine penicillin, cinoxacin, ciprofloxacin HCl, clarithromycin, clofazimine, cloxacillin, demeclocycline, doxycycline, erythromycin, ethionamide, imipenem, nalidixic acid, nitrofurantoin, rifampicin, spiramycin, sulphabenzamide, sulphadoxine, sulphamerazine, sulphacetamide, sulphadiazine, sulphafurazole, sulphamethoxazole, sulphapyridine, tetracycline, trimethoprim), anti-coagulants (e.g., dicoumarol, dipyridamole, nicoumalone, phenindione), antidepressants (e.g., amoxapine, maprotiline HCl, mianserin HCl, nortriptyline HCl, trazodone HCl, trimipramine maleate), antidiabetics (e.g., acetohexamide, chlorpropamide, glibenclamide, gliclazide, glipizide, tolazamide, tolbutamide), anti-epileptics (e.g., beclamide, carbamazepine, clonazepam, ethotoin, methoin, methsuximide, methylphenobarbitone, oxcarbazepine, paramethadione, phenacemide, phenobarbitone, phenytoin, phensuximide, primidone, sulthiame, valproic acid, topirimate, lamotrigine, gabapentin, levetiracetam, pregabalin), antifungal agents (e.g., amphotericin, butoconazole nitrate, clotrimazole, econazole nitrate, fluconazole, flucytosine, griseofulvin, itraconazole, ketoconazole, miconazole, natamycin, nystatin, sulconazole nitrate, terbinafine HCl, terconazole, tioconazole, undecenoic acid), antigout agents (e.g., allopurinol, probenecid, sulphin-pyrazone), antihypertensive agents (e.g., amlodipine, benidipine, darodipine, dilitazem HCl, diazoxide, felodipine, guanabenz acetate, isradipine, minoxidil, nicardipine HCl, nifedipine, nimodipine, phenoxybenzamine HCl, prazosin HCl, reserpine, terazosin HCl), antimalarials (e.g., amodiaquine, chloroquine, chlorproguanil HCl, halofantrine HCl, mefloquine HCl, proguanil HCl, pyrimethamine, quinine sulphate), anti-migraine agents (e.g., dihydroergotamine mesylate, ergotamine tartrate, methysergide maleate, pizotifen maleate, sumatriptan succinate), anti-muscarinic agents (e.g., atropine, benzhexol HCl, biperiden, ethopropazine HCl, hyoscyamine, mepenzolate bromide, oxyphencylcimine HCl, tropicamide), anti-neoplastic agents and immunosuppressants (e.g., aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, procarbazine HCl, tamoxifen citrate, testolactone), anti-protazoal agents (e.g., benznidazole, clioquinol, decoquinate, diiodohydroxyquinoline, diloxanide furoate, dinitolmide, furzolidone, metronidazole, nimorazole, nitrofurazone, ornidazole, tinidazole), anti-thyroid agents (e.g., carbimazole, propylthiouracil), anxiolytic, sedatives, hypnotics and neuroleptics (e.g., alprazolam, amylobarbitone, barbitone, bentazepam, bromazepam, bromperidol, brotizolam, butobarbitone, carbromal, chlordiazepoxide, chlormethiazole, chlorpromazine, clobazam, clotiazepam, clozapine, diazepam, droperidol, ethinamate, flunanisone, flunitrazepam, fluopromazine, flupenthixol decanoate, fluphenazine decanoate, flurazepam, haloperidol, lorazepam, lormetazepam, medazepam, meprobamate, methaqualone, midazolam, nitrazepam, oxazepam, pentobarbitone, perphenazine pimozide, prochlorperazine, sulpiride, temazepam, thioridazine, triazolam, zopiclone), β-Blockers (e.g., acebutolol, alprenolol, atenolol, labetalol, metoprolol, nadolol, oxprenolol, pindolol, propranolol), cardiac inotropic agents (e.g., amrinone, digitoxin, digoxin, enoximone, lanatoside C, medigoxin), corticosteroids (e.g., beclomethasone, betamethasone, budesonide, cortisone acetate, desoxymethasone, dexamethasone, fludrocortisone acetate, flunisolide, flucortolone, fluticasone propionate, hydrocortisone, methylprednisolone, prednisolone, prednisone, triamcinolone), diuretics: acetazolamide, amiloride, bendrofluazide, bumetanide, chlorothiazide, chlorthalidone, ethacrynic acid, frusemide, metolazone, spironolactone, triamterene), anti-parkinsonian agents (e.g., bromocriptine mesylate, lysuride maleate), gastrointestinal agents (e.g., bisacodyl, cimetidine, cisapride, diphenoxylate HCl, domperidone, famotidine, loperamide, mesalazine, nizatidine, omeprazole, ondansetron HCl, ranitidine HCl, sulphasalazine), histamine H,-receptor antagonists (e.g., acrivastine, astemizole, cinnarizine, cyclizine, cyproheptadine HCl, dimenhydrinate, flunarizine HCl, loratadine, meclozine HCl, oxatomide, terfenadine), lipid regulating agents (e.g., bezafibrate, clofibrate, fenofibrate, gemfibrozil, probucol), nitrates and other anti-anginal agents (e.g., amyl nitrate, glyceryl trinitrate, isosorbide dinitrate, isosorbide mononitrate, pentaerythritol tetranitrate), opioid analgesics (e.g., codeine, dextropropyoxyphene, diamorphine, dihydrocodeine, meptazinol, methadone, morphine, nalbuphine, pentazocine), sex hormones (e.g., clomiphene citrate, danazol, ethinyl estradiol, medroxyprogesterone acetate, mestranol, methyltestosterone, norethisterone, norgestrel, estradiol, conjugated oestrogens, progesterone, stanozolol, stibestrol, testosterone, tibolone), and stimulants (e.g., amphetamine, dexamphetamine, dexfenfluramine, fenfluramine, mazindol).

The above-stated compounds are predominantly stated by their international nonproprietary name (INN) and are known to the person skilled in the art. Further details may be found, for example, by referring to International Nonproprietary Names (INN) for Pharmaceutical Substances, World Health Organization (WHO).

### DRUG DELIVERY DEVICES

The drug delivery devices of the present invention are designed to address the requirements for a device that is inserted into the mouth by the patient or caregiver, that resides in the mouth while it is administering drug, and that can be removed from the mouth by the patient or caregiver. Preferred drug delivery devices include oral liquid impermeable reservoirs.

The drug delivery devices typically have a total volume of less than about 10 mL, and preferably less than 7.5, 5.0, or 3.0 mL. Preferred volumes for the drug delivery devices are 0.5-3.0 mL, to minimize interference with the patient's mastication, swallowing and speech.

The drug delivery devices of the invention preferably contain bite-resistant structural supports that enable them to withstand a patient's bite with a force of at least 200 Newtons, without rupturing and without infusing a bolus of greater than 5% of the drug contents, when unused reservoirs are newly inserted into the mouth. Bite-resistant structural supports, for example, can include a structural housing that encapsulates the entire drug reservoir, propellant reservoir and pump components, either protecting individual components, the entire device, or both. Structural housings can be constructed of any tough, impact-resistant, material that is compatible with the oral anatomy. Metals such as stainless steel or titanium, polymers such as poly (methyl methacrylate) and composites such as Kevlar, are examples of tough materials that are compatible with the oral anatomy. Other structural elements can include posts or ribs in the housings that are placed in locations such that compression is not possible due to the stiffness of the housing components being increased. In another example, structural elements, such as ribs and posts, allow some flexure of the housing, but do not allow sufficient flexure to deform the components of the pump. In another example, the pump housing can be made of a material that allows some flexure and there is sufficient volume within the housing such that the drug reservoir and or propellant reservoir, can deform or become displaced when pressure is applied but maintain their structural integrity. In another embodiment, some of the previously described elements can be incorporated into a design, and the entire internal volume of the device is potted with a tough biocompatible material such as an epoxy or a thermoplastic.

To prevent their being accidentally swallowed or aspirated into the trachea, the drug delivery devices of the invention are secured in the mouth. They may be secured to any interior surface of the mouth, such as one or more teeth, the roof of the mouth, the gums, the lips or the cheek within the mouth of the patient. In order to obtain a secure and comfortable fit, the devices may be molded to fit on or attach to a surface within the mouth of a patient, such as the teeth or the roof of the mouth, or they may conform to at least one cheek. In some embodiments, the drug delivery devices are secured such that they are positioned on the teeth, on a cheek, between the gums and the cheek, between the gums and the lips, or at the roof of the mouth. Examples are a curved, elongated shape of greater than 4 cm length in its curved form (e.g., greater than 5, 6 or 7 cm) that can be placed between the gums and the cheek and lips; or drug delivery devices positioned adjacent to both cheeks and connected with a bridge, optionally forming fluidic contacts with both the left and the right parts.

The drug delivery device may include a rigid plastic, a deformable plastic or a plastic that deforms so easily that it can conform to contours of the mouth of the patient, for example, to contours of the cheek, or of the roof mouth, or the floor of the mouth, or the front gum near a lip, or the teeth. The easily deformed plastic may include, for example, elastomeric butyl rubber, elastomeric silicone or polyurethane. It can be a less deformable, for example substantially oxygen or water impermeable, plastic, such as poly(vinylidene chloride), poly(vinyl chloride), poly(triflurorochloro)ethylene, polyethylene terephthalate)), polyether polycarbonate, or high density, high crystallinity polyethylene. Alternatively, for example, when the administered drug formulation is not an aqueous acid corroding the metal, the drug delivery device may include a metal, such as stainless steel or alloyed titanium, aluminum or magnesium. In an alternative embodiment, the drug delivery device includes multiple segments connected by flexible connectors, so that the drug delivery device is able to conform to the shape of the surface on which it is mounted.

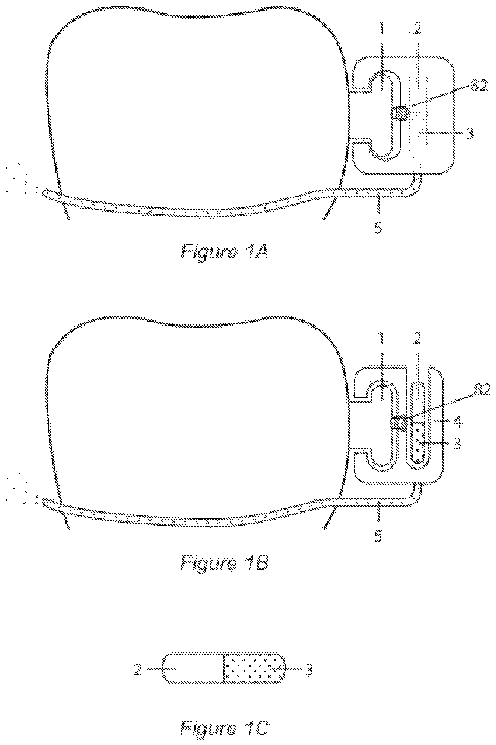

The drug delivery devices of the invention may be attached to the teeth or other interior surfaces of the mouth by a fastener, as shown in Figures 1A and 1B. The fastener **1**, the one or more pumps **2**, and the one or more drug reservoirs **3** may include a single unit or they may include separate components, with the fastener remaining in the mouth when the one or more pumps or one or more reservoirs are removed. Figure 1A shows an embodiment where a pump **2**, and a drug reservoir **3** include a single removable component that can be attached to the fastener 1. Drug is delivered into the mouth via a tube **5** which may optionaly include a flow restrictor. Figure 1B shows an embodiment including a reusable housing **4**, and a disposable pump **2** and drug reservoir **3.** The fastener **1**, one or more drug pumps and one or more drug reservoirs may be removably attached to each other using magnets, clips, clasps, clamps, flanges, latches, retaining rings, snap fasteners, screw mounts, or other attachment mechanisms known in the art. In preferred embodiments, the fastener comprises a transparent retainer or a partial retainer on one side of the mouth (e.g., attached to 3, 4, or 5 teeth).

A preferred embodiment of the device is shown in Figure 2, where the pump and/or oral liquid impermeable reservoir is secured to either the upper or lower teeth using a transparent retainer 6. One, two or more pumps and/or one or more drug reservoirs are secured on the buccal side of the transparent retainer. One, two, or more drug pumps **2** and/or drug reservoirs **3** may be secured unilaterally, on either the right or left sides, positioned in the buccal vestibule or, alternatively, on the lingual side of the teeth. The drug pump and reservoir are attached to the transparent retainer via a housing **4.** Drug is delivered into the mouth via a tube **5** which may optionaly include a flow restrictor. The tube **5** serves to carry the drug from the buccal to the lingual side of the teeth, where the drug may be more readily swallowed. The tube may be molded into the retainer.

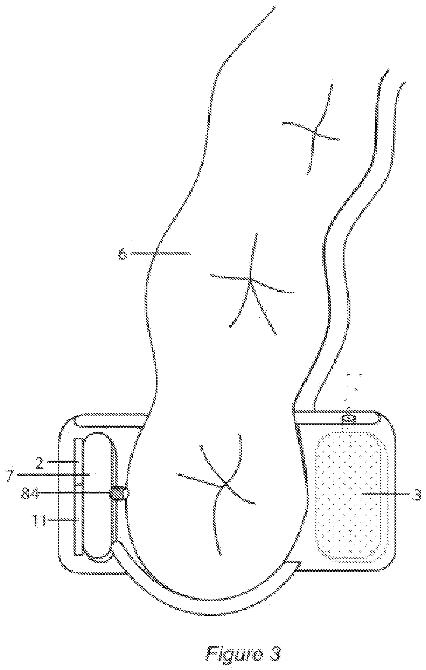

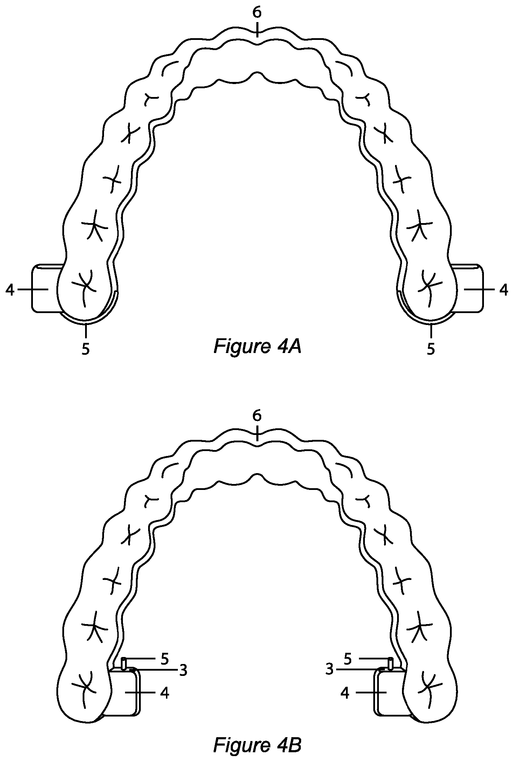

In a related embodiment, illustrated in Figure 3, the pumps **2** and reservoirs **3** can be configured to be positioned both on the lingual side of the teeth and in the buccal vestibule. In this embodiment, the pump **2** is used to fill an elastomeric compartment **7,** described in greater detail in Figures 17 A, 17B, and 17C, which drives the drug from the drug reservoir **3.** In another related embodiment, illustrated in Figure 4, one, two or more pumps and/or oral liquid impermeable reservoirs may be secured bilaterally, on both the right and left sides, positioned in the buccal vestibule or on the lingual side of the teeth, or both buccally and lingually. Figure 4A depicts a fastener in the form of an invisible retainer **6**, including two bilateral housings **4** (shown empty) on the buccal side of the teeth into which drug pumps and/or drug reservoirs may be inserted. Figure 4B depicts a fastener in the form of an invisible retainer **6**, including two bilateral housings **4** (shown filled) on the lingual side of the teeth into which drug pumps and/or drug reservoirs have been inserted.

Optionally, two or more oral liquid impermeable drug reservoirs may be in fluidic contact with each other. Optionally, the transparent retainer **6** may include 2, 3, 4 or more layers of different hardness, to ease insertion and removal of the transparent retainer from the teeth. For example, the transparent retainer **6** may include a dual laminate with a softer, inner, tooth-contacting layer, and a harder, outer layer contacting the cheeks and tongue.

The one or more pumps and/or oral liquid impermeable reservoirs may be removably attached to the transparent retainer using magnets, clips, clasps, clamps, flanges, latches, retaining rings, snap fasteners, screw mounts, or other attachment mechanisms known in the art. In one embodiment, the transparent retainer includes one, two, or more housings into which one, two, or more pumps and/or the oral liquid impermeable reservoir are inserted. The one, two or more housings may be molded or formed to the shape of the one, two or more pumps and/or oral liquid impermeable reservoirs.

For delivery of some drugs, such as LD or LD prodrugs, it is desirable to administer the drug-including solid or fluid on the lingual side of the teeth, rather than on the buccal side of the teeth, in order to minimize the residence time of the drug in the mouth, thereby avoiding potential accumulation of the drug in the buccal vestibule and minimizing potentially irritating exposure of the buccal tissue to the drug. In a preferred embodiment, the fastener (e.g., a transparent retainer or a partial retainer) includes one, two, or more fluidic channels to transport the drug-including fluid into the mouth from the one, two or more pumps and/or oral liquid impermeable reservoirs. The fluidic channels can transport the drug-including fluid from one, two, or more oral liquid impermeable reservoirs located on the buccal side of the teeth to the lingual side of the teeth. For example, the fluidic channels can include one, two or more tubes that are molded into the fastener. The fluidic channels can, for example, pass behind the rear molars, above the mandibular arch, so that they do not cross the biting surface of the teeth. The fluidic channels may include a diameter of less than 0.25 mm, 0.25-1 mm, 1-2 mm, 2-3 mm, or greater than 3 mm. The fluidic channels may include a fluidic path length in the fastener of less than 1 mm, 1-3 mm, 3-5 mm, 5-10 mm, or greater than 10 mm.

The one, two or more pumps and/or one, two, or more oral liquid impermeable drug reservoirs can be in fluid communication with the one, two, or more fluidic channels in the fastener (e.g., a transparent retainer or a partial retainer) via any type of leak-free fluidic connector known in the art, such as leak-free snap fastner or screw-mount. The leak-free fluidic connector preferably includes metal, to improve durability. Optionally, the one, two or more pumps and/or the one, two, or more oral liquid impermeable reservoirs do not deliver drug when they are not mounted on the fastener, while mounting these on the fastener initiates delivery of the drug. Similarly, drug delivery is temporarily halted when the pumps and/or oral liquid impermeable reservoirs are dismounted from the fastener.

In one embodiment, the one, two or more fluidic channels may include one, two or more flow restrictors. The one, two or more flow restrictors may include metal tubes that are molded into the fastener (e.g., a transparent retainer or a partial retainer). By incorporating flow restrictors into a reusable fastener, the disposable drug delivery device and/or oral liquid impermeable reservoir need not include a flow restrictor that accurately controls the rate of infusion.

In another embodiment, a reusable fastener (e.g., a transparent retainer or a partial retainer) may include a pump and/or power source. With a reusable pump and/or power source incorporated into the fastener, the disposable portion of the drug delivery device and/or the oral liquid impermeable reservoir need not include the pump and/or power source, thereby reducing overall cost.

The fastener or its components, such as the housings, may be manufactured using methods known in the art, such as thermoforming, injection molding, pressure molding, and laminating.

The drug delivery device may be a single unit, or it may have two, three, four, five or more components. The drug delivery device may have one, two, three, four, five or more oral liquid impermeable reservoirs in which the solid or fluid drug formulation is contained. These one or more reservoirs may form a single component, or they may form multiple components.

The drug delivery devices may be reusable, disposable, or they may have one or more reusable components and one or more disposable components. In a preferred embodiment, the fastener is reusable, and may be reused for a period of equal to or greater than 7, 30, 60 or 180 days, or one year or two years. In another preferred embodiment, the one or more oral liquid impermeable reservoirs are single use, disposable components. The pump, may be either reusable or disposable. A flow restrictor, may be a single use disposable or may be reused.

The oral liquid impermeable reservoir may be refillable with a solid or fluid drug formulation. In a preferred embodiment, the oral liquid impermeable reservoir is a single use disposable. The oral liquid impermeable reservoir may be filled by the user. In preferred embodiments, the oral liquid impermeable reservoir is prefilled.

The drug delivery device further includes one, two, three, four or more orifices for releasing the drug from the device into the mouth.

Durations of administration from a single drug delivery device or oral liquid impermeable reservoir typically exceed 4, 8, 12, or 16 hours per day, up to and including 24 hours per day. Administration can also take place over multiple days from a single device or oral liquid impermeable reservoir, e.g., administration of a drug for 2 or more days, 4 or more days, or 7 or more days. The devices can be designed such that they can be worn when the patient is awake or asleep.

It is desirable that the patient be able to temporarily remove the drug delivery device from the mouth, for example, to eat meals, brush teeth, or at times when the patient does not want or need the medication (e.g., at night). Consequently, the drug delivery devices and/or some of its components (such as the pump and/or the oral liquid impermeable reservoirs) can be temporarily removable. It is, however, acceptable for some components, such as the fastener, to remain in the mouth if these do not interfere with the patient's activities. For example, a band, a fastener cemented or glued to one or more teeth, a retainer, or a muco-adhesive patch adhered to the oral mucosa, and which holds the pump and/or oral liquid impermeable reservoir in place, may remain in the mouth when the pump and/or the oral liquid impermeable reservoir are removed.

It is desirable that the drug delivery device include an indicator of: the quantity remaining of one or more drugs; the infusion time remaining until empty; and/or that one or more of the oral liquid impermeable reservoirs is empty and should be replaced.

The drug delivery devices of the current invention are configured and arranged to administer one or more solid or fluid drug formulations from one or more oral liquid impermeable reservoirs including a total volume of 0.1 - 10 mL of drugs, e.g., 0.1-1.0, 1.0-2.0, 2.0-3.0, 3.0-4.0, 4.0-5.0, 5.0-6.0, 6.0-7.0, 7.0-8.0, 8.0-9.0, or 9.0-10 mL. They are configured and arranged to administer the one or more solid or fluid drug formulations at a rate in the range of 0.03 - 1.25 mL/hour, e.g., 0.03 - 0.10, 0.10-0.20, 0.20-0.30, 0.30-0.40, 0.40-0.50, 0.50-0.60, 0.60-0.70, 0.70-0.80, 0.80-0.90, 0.90-1.0, 1.0-1.1, or 1.1-1.25 mL/hour. In some embodiments, they are configured and arranged to administer the drug, (i.e., the active pharmaceutical ingredient) at an average rate of 0.01 - 1 mg per hour, 1 - 10 mg per hour, 10 - 100 mg per hour, or greater than 100 mg per hour. In other embodiments, the drug product (i.e., the active pharmaceutical ingredient plus excipients) is delivered at an average rate of 0.01 - 1 mg per hour, 1-10 mg per hour, 10 - 100 mg per hour or greater than 100 mg per hour.

The one or more drugs may be administered at a constant rate or at a non-constant rate that varies over the course of the administration period. For example, the drug delivery device may be programmed to administer drugs according to a drug delivery profile over the course of the administration period. The drug delivery device may also have an on-demand bolus capability, whereby the patient or caregiver may initiate the delivery of a bolus of drug.

In preferred embodiments, the drug delivery device administers one or more solid or fluid drug formulations via continuous and/or frequent administration, e.g., infusion. In a preferred embodiment, the solid or fluid drug administration rate is held constant or near constant for a period of 4, 8, 12, 16 or 24 hours during the day. For example, the administered volume may vary by less than ±10% or ± 20% per hour, or by ±10% or ± 20% per 15 minute period, over a period of 4, 8, 12, 16 or 24 hours. In another embodiment, the solid or fluid drug administration rate is held about constant during the awake hours of the day. In another embodiment, the solid or fluid drug formulation administration rate is held about constant during the asleep hours. In another embodiment, the solid or fluid drug formulation administration rate is held about constant during the awake hours of the day, except for the delivery of a bolus at about the time of waking. In one embodiment, the administration rate can be set prior to insertion in the mouth by the patient or by the caregiver. In another embodiment, the administration is semi-continuous and the period between the infusions is less than the biological half-life of the drug t_{1/2}; for example it can be less than one half of t_{1/2}, less than 1/3rd of t_{1/2}, or less than V4 of t_{1/2}, or less than 1/10th of t_{1/2}.

For fluid drug formulations, it is desirable to deliver the solutions or suspensions of the invention using drug delivery devices that are small, efficient, inexpensive, and reliable. This can be particularly challenging when these fluids are viscous. It is also desirable to minimize the pressure required to pump the fluid. In preferred drug delivery devices for fluids of greater than 0,05 Pa·s (50 cP), for example, 0,05-0,1 Pa·s (50-100 cP), 0,1-1 Pa·s (100-1,000 cP), 1-10 Pa·s (1,000-10,000 cP), 10-50 Pa·s (10,000-50,000 cP), 50-250 Pa·s (50,000-250,000 cP), or greater than 250 Pa·s (250,000 cP), the drug can exit the device through a tube, channel, or orifice of less than 4 cm, 3 cm, 2 cm, 1 cm, 0.5 or 0.2 cm length. For example, the fluid may be delivered through an optionally flexible cannula, or it may be delivered through an orifice without utilizing any type of tubing or cannula. To further minimize the pressure required to pump the fluid, the tube, channel or orifice through which the drug exits the device may have an internal diameter of greater than 1, 2, 3, 4, or 5 mm, for example, 1 mm - 5 mm, 1 mm - 3 mm, 2 mm - 4 mm, or 3 mm - 5 mm.

### Pumps

The pumps for the drug delivery devices must be suitable for miniature devices carried safely and comfortably in the mouth. Any suitable mechanical pump, as characterized in the claims, may be used. The pump and the oral liquid impermeable reservoir may be distinct. In preferred embodiments, the pump also serves as the oral liquid impermeable reservoir.

Miniature pumps are advantageous for placement in the mouth. For example, the solid or fluid including the drug may include greater than 33%, 50%, 66%, or 75% of the total volume of the drug delivery device.

*Non-electric pumps.* Pumps that do not require a battery can be smaller and have fewer moving parts than battery-requiring electrical pumps. One group of nonelectric disposable pumps of the invention is based on the physical principle that mechanical restriction within the flow path can determine the flow rate of a pressurized fluid. The pressure on the fluid may be generated by a variety of mechanisms using nonelectric power, including a stretched elastomer, a compressed elastomer, a compressed spring, a propellant, and a cartridge of pressurized gas. The restriction of flow may be provided by an orifice (e.g., in the drug reservoir) or by narrow-bore tubing (such as a metal, glass or plastic pipe) or by a capillary.

Because different patients may require different doses of drug, it is desirable for the drug delivery devices of the invention to be available as a product line of multiple products, each product having a different drug administration rate. The desired flow rate may be obtained by selecting a flow restrictor of the appropriate inner diameter and length. Exemplary flow restrictor materials are glasses, ceramics, metals, strong difficult to deform polymers (e.g., polyvinyl chloride) and their composites. In one embodiment, the plastic flow restricting tubing may be cut to the length providing the desired flow rate. Use of a narrow-bore tubing as a flow restrictor simplifies the manufacturing process for such a product line. During the manufacturing process a narrow-bore tubing with constant inner diameter may be cut into multiple segments of fixed length A, to provide reproducible flow restrictors for products with one flow rate. A different portion of the narrow-bore tubing with constant inner diameter may be cut into multiple segments of fixed length B, to provide reproducible flow restrictors for products with a second flow rate.

In another embodiment, when the reservoir is metallic one or more pinholes in the reservoir wall can include the flow restrictor, i.e., a desired flow rate can be obtained by the number of pinholes and the diameter of the one or more pinholes.

Flow rate is affected by the pressure gradient across the flow restrictor and by fluid viscosity. A significant source of inaccuracy in existing pump products is that viscosity is strongly affected by temperature. An important benefit of carrying within the mouth the drug delivery devices of the invention is that the temperature is held nearly constant at about 37 °C, thereby minimizing variations in the infusion rate.

The formulations of the invention are often viscous fluids. Use of viscous fluids is often desired to achieve the small volumes, high concentrations, uniform drug dispersion, storage stability, and operational stability desired for the drugs and methods of the invention. Consequently, it is often desired to employ pump mechanisms that can provide the pressures required to pump the viscous fluids.

The pressure generated by elastomeric, spring-driven and gas-driven pumps on fluid is typically in the range of 33,331 to 159,987 kPa (250 to 1200 mm Hg), depending on flow rate and cannula size, but can be higher. For example, the pressure may be 33,331-66,661, 66,661-99,992 kPa, 99,992 kPa-133,322, 133,322-166,653 or above 133,623 kPa (250-500, 500-750, 750-1,000, 1,000-1250, or above 1,250 mm Hg). The pressurizing gas can be chemically generated, for example electrolytically generated, e.g., by electrolyzing water. Higher pressures may be achieved with osmotic pumps, such as controlled release osmotic tablets, which may generate pressures equal to or greater than 333,306, 666,612 or 133,224 kPa (2,500, 5,000, or 10,000 mm Hg).

The drug delivery device may be kept in the mouth while the patient is eating and drinking, or may be removed for eating or drinking. Preferably, the introduction into the mouth of food or liquid, including food or liquids that are hot, cold, acidic, basic, oily, or alcoholic, does not have a clinically significant effect on the drug delivery. For example, such conditions may affect the solubility of the drug; the volume of the drug-including fluid in the reservoir; the viscosity of the drug-including fluid in the oral liquid impermeable reservoir; the volume of the gas in the reservoir (if present); the diffusivity of mass-transport limiting membranes (if present); and/or the force exerted by elastomers or springs (if present). Some drug delivery technologies, such as controlled release muco-adhesive drug delivery patches, can deliver large drug boluses when in contact with a hot, cold, acidic, basic, oily, or alcoholic liquid in the mouth. Such boluses may result in undesirable clinical effects, and should be minimized. In one embodiment, the solid or fluid drug delivery devices of the invention deliver a bolus of less than 5%, 4%, 3% or 2% of the contents of a fresh oral liquid impermeable reservoir, when immersed for 5 minutes or for 1 minute in a beaker containing a stirred physiological saline solution that is hot (at about 55 °C), cold (at about 1 °C), acidic (at about pH 2.5), basic (at about pH 9), oily (an physiological saline solution containing 5% by weight of olive oil), or alcoholic (a physiological saline solution containing 5% by weight ethanol), as compared to an identical drug delivery device immersed for the same duration in a physiological saline solution of pH 7 at 37 °C. For example, a LD or LD prodrug delivery device may deliver a bolus of less than 0.5, 0.25, 0.12, or 0.06 millimoles of LD or LD prodrug under these conditions.

*Battery powered pumps* - *are not according to the invention and are present for illustration purposes only.* Other than than powering the pump, the battery can power optional electronic controls and communication capabilities (e.g., radio frequency receivers) for programmed drug delivery and remote control of the drug delivery by a transmitting device. A miniature battery may be used to drive the pump or dispensing mechanism for the delivery of the solid or fluid drug. Any low power pump drive mechanism known in the art may be used, such as syringe, hydraulic, gear, rotary vane, screw, bent axis, axial piston, radial piston, peristaltic, magnetic, piezoelectric, diaphragm and memory alloy, such as nitinol, based.

An advantage of battery powered pumps for use in the mouth is that it is possible to temporarily stop the drug delivery from the device if the patient wishes to temporarily remove the drug delivery device from the mouth. This can be accomplished, for example, by turning off the electric power to the pump.

One example of a battery powered pump is a miniature diaphragm pump that uses the motion of a piezoelectric crystal to fill a chamber with drug from a reservoir in one motion and to expel the drug from the chamber in the opposite motion. Typically, the frequency of oscillation of the piezoelectric crystal is less than about 20,000 Hz, 5,000 Hz, or 1,000 Hz, so as to avoid the higher frequencies where biological membranes are ultrasonically disrupted or where free radicals are more likely to be generated through a sonochemical process. A significant advantage to the diaphragm pump is that it can be used to very accurately deliver materials of both high and low viscosity, as well as solids such as granules or powders.

Another examples of a battery powered pump is a miniature electroosmotic pump as disclosed, for example, in U.S. Patent Publication Nos. 2013/0041353, 2013/0156615, 2013/0153797 and 2013/0153425, in PCT Publication No. WO2011/112723, and in Korean Patent Publication No. KR101305149. Typically the volume of the miniature electroosmotic pump, including its battery or batteries, is smaller than the volume of the fluid in the unused oral liquid impermeable reservoir. For example, the volume of the pump can be less than 1/2, less than 1/3rd, less than 1/4 or less than 1/5th of the volume of the unused oral liquid impermeable reservoir. When an electroosmotic pump is used with a refillable reservoir, the battery powering the pump can be replaced upon refilling. To provide different patients with different dose rates, oral liquid impermeable reservoirs may be filled with the drug at different concentrations. Alternatively, the flow rate of the electroosmotic pump can be adjusted by controlling the applied voltage or the applied current, or by varying the cross sectional fluid contacting area of the membrane sandwiched between the electrodes. Optionally, the applied voltage or current can be remotely adjusted by incorporating a short range RF receiver in the insert.

Another category of battery powered pumps is positive displacement pumps. Two examples of battery powered positive displacement pumps that can be used to deliver the drug are gear pumps and peristaltic pumps. One of the main advantages of the use of a positive displacement pump is that the flow rate is not affected by changes in ambient pressure. The gear pump, in one embodiment, uses two rotors that are eccentrically mounted and intermeshed with their cycloid gearing. As a result a system of several sealed chambers exists at all times and are moved toward the outlet of the pump, one at a time. An example of a gear pump is the Micro annular gear pump mzr-2521 from HNP Mikrosysteme GmbH. A second type of battery powered positive displacement pump is the peristaltic pump. Peristaltic pumps use a series of rollers to pinch a tube creating a vacuum to draw the material from a reservoir, thereby creating and moving a volume of drug within subsequent roller volumes to deliver the drug toward the outlet of the pump. An example of a battery powered peristaltic pump is the RP-TX series micro peristaltic pump from Takasago Electric, Inc.

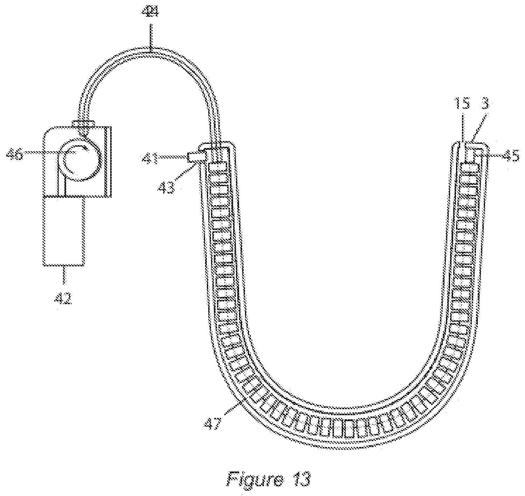

Further examples of battery-powered pumps are provided below, in the descriptions of Figures 13, 14 and 15, however, as indicated above, such pumps are not according to the invention and are presented for illustration purposes only.

*Elastomeric infusion pumps.* In elastomeric infusion pumps, the pressure on the fluid is generated by the force of a stretched or compressed elastomer. An example of an elastomeric, partially disposable, constant-rate medication infusion pump with flow restrictor is the CeQur PaQ insulin patch pump, described in U.S.S.N. 12/374,054 and U.S. Patent No. 8,547,239.

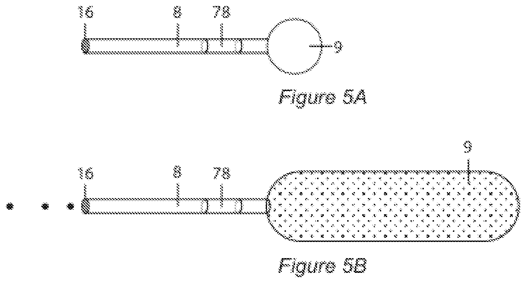

Figures 5A and 5B show an embodiment of an elastomeric drug reservoir that can be filled with a drug to pressurize the drug and to pump the fluid at a controlled rate through the use of a narrow-bore tubing **8** that serves as a flow restrictor. Figure 5A shows the elastomeric reservoir **9** when empty of drug and Figure 5B shows the elastomeric balloon **9** when pressurized due to expansion of the elastomer by filling with the drug.

Preferably, the elastomeric membrane is protected by an outer protective shell. The outer protective shell can either be a conformable elastomer or a more rigid plastic, which may be molded to a surface of the mouth. The membranes of elastomeric pumps may include both natural and synthetic (e.g., thermoplastic) elastomers (e.g., isoprene rubber, latex, silicon, and polyurethanes), and can be made of a single or multiple layers. The type of elastomer and the geometry of the elastomeric balloon 9 determine the pressure generated on the fluid when the balloon is stretched. Multiple-layer elastomeric membranes can generate higher pressures than the single-layer membranes. Higher driving pressures are of benefit for achieving faster flow rates and for pumping viscous fluids.

To minimize the change in flow rate as the fluid is delivered, it is preferred to utilize sufficiently high tension in the elastomeric membrane such that the difference between the starting and ending pressure on the fluid is less than 30%, 20%, or 10% of the starting pressure.

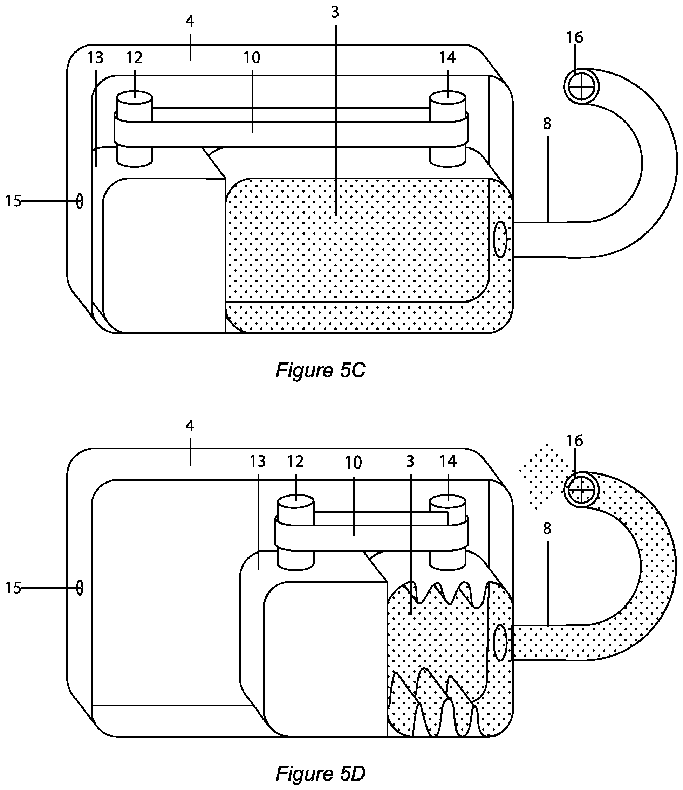

Another embodiment of an elastomer-driven pump is the use of an elastomeric band **10** (e.g., a rubber band, see Figures 5C and 5D) to apply a constant force to a drug reservoir **3** driving the drug through a narrow bore tubing **8** with a check valve **16** (or one-way valve) at the downstream end. Elastomers are known to have material properties where large strain values can be imparted on them with relatively small changes in stress and, in some regions of the stress-strain curve, no change in stress at all. In one embodiment of an elastomeric band pump, a stretched polyisoprene band is used. Polyisoprene has desirable material properties in that, within a specific region of the stress-strain curve, significant changes in strain result in virtually no change in stress. In this embodiment, the elastomeric rubber band **10** is used within the range in the stress-strain curve where the stress remains within the elastic region from the beginning to the end of the stroke of the motion of the piston. In this embodiment, one end of the elastomeric band **10** is placed onto the post **12** attached to the piston **13** while the other end is placed onto the stationary post **14.** The tension on the elastomeric band **10** applies a force to the drug reservoir and in order to eliminate the effect of ambient pressure differences, a vent hole **15** allows the drug reservoir **3** to be exposed, on all sides, to ambient pressure. The check valve **16** also serves to keep saliva from entering the narrow bore tubing **8** while the drug is not flowing. Figures 5C and D show the device with a full drug reservoir **3** and a partially emptied drug reservoir **3**, respectively.

Yet another embodiment of a nonelectric disposable pump including a pressurized fluid and a flow restrictor involves the use of a volume of elastomer in a fixed volume drug reservoir. The elastomer may, optionally, be a closed cell elastomer. The elastomer is compressed and the subsequent controlled expansion of the elastomer provides the force to deliver the drug. In continuous pumping using a gas-including closed-cell elastomer, a drug-including fluid is pumped at an about constant flow rate by maintaining in the fixed volume, oral liquid impermeable reservoir an about constant pressure. For maintaining the about constant pressure in the reservoir a substantially compressible elastomer is placed in the reservoir. The substantially compressible elastomer can be compressed by applying a pressure in the reservoir that is typically less than 10132,5 kPa (100 atm) (for example less than 1013,25 kPa (10 atm)) to a volume of elastomeric material. The volume of the compressed elastomeric material in the pressurized reservoir can be less than about 67%, 50%, or 25% of the volume of the elastomer at about sea-level atmospheric pressure. An exemplary family of such compressible elastomers includes closed cell rubbers, also known as closed-cell rubber foams. Closed cell rubbers have fully rubber-enclosed gas pores, the pores containing a gas, such as N₂, CO₂, or air. At about sea-level atmospheric pressure the density of the closed pore elastomer can be less than 67% of the density of the elastomer without the gas, for example between 67% and 33% of the elastomer without the gas, between 33% and 25% of the elastomer without the gas, between 25% and 12% of the elastomer without the gas, or less than 12% of the density of the elastomer without the gas. The volume percent of the gas in the elastomer at about sea-level atmospheric pressure can be greater than 20 volume %, for example greater than 50 volume %, or greater than 75 volume %. The elongation of the gas-including elastomer can be greater than about 25%, for example between 50% and 200%, between 200% and 450%, or greater than 450%. The gas containing elastomer can be of any shape fitting in the fixed volume drug reservoir. It can be a single piece, such as a block, or an optionally folded sheet, or it can be multiple pieces, such as small gas-filled spheres. Typical gas pore enclosing elastomers can include cross-linked polymers and copolymers for example of dienes (exemplified by isoprene, chloroprene, butadiene); exemplary copolymers include acrylonitrile-butadiene-styrene, acrylonitrile-butadiene, or elastomeric polyacrylates, or elastomeric olefins such as ethylene-propylene rubbers, or elastomeric silicones and fluorosilicones, or elastomeric polyurethanes. In general the less gas permeable, particularly less water vapor permeable elastomers, are preferred.

Drug delivery devices including closed cell elastomeric pumps are preferrably configured and arranged to continuously or semi-continuously administer the drug into the patient's mouth at an average rate for a delivery period of not less than 4 hours and not more than 7 days at a rate in the range of 80% - 120% of the average rate.

During the delivery of the drug-including fluid at a constant flow rate the gas-including elastomer expands such that it occupies most or all of the volume vacated by the already delivered fluid and there are large gas bubbles within the elastomer. In an exemplary method of production and operation of a system delivering the drug at an about constant flow rate, a closed-cell elastomer can be placed in a drug reservoir equipped with an closed outlet or outlets for drug delivery and optionally equipped with a septum for filling the reservoir. The drug reservoir can have walls made of a material that does not substantially deform at the operating pressure in the reservoir, for example the deformation of the wall under the applicable pressure changing the reservoir volume typically by less than 5%, for example by less than 1%. The drug containing fluid can be then injected through the septum, compressing the gas-containing closed cells of the rubber and pressurizing thereby the reservoir. Opening the outlet or outlets initiates the flow of the drug-including fluid, e.g., into the mouth. The about constant pressure in the reservoir during the delivery of the drug can be controlled, for example, by the type of the closed cell rubber.

An advantage of elastomeric infusion pumps for use in the mouth is that it is possible to temporarily stop the drug delivery from the device if the patient wishes to temporarily remove the drug delivery device from the mouth. This can be accomplished, for example, by blocking or closing the flow restrictor, e.g., the orifice, the glass capillary or the narrow bore tubing.

To minimize the change in flow rate when the patient drinks a hot beverage, it is preferred to utilize elastomeric materials whose force is relatively independent of temperature in the range of 37°C - 55°C. For example, the force in a fresh reservoir may increase by less than 30%, 20% or 10% when the temperature is raised from 37°C to 55°C.

*Spring driven pumps.* Positive-pressure spring-powered pumps are powered by energy stored in a compressed spring. In one embodiment, the spring is compressed during the reservoir filling process, as the volume of the solid or fluid in the reservoir increases.

A significant advantage of spring-driven pumps for use in the mouth is that it is possible to temporarily stop the drug delivery from the device if the patient wishes to temporarily remove the drug delivery device from the mouth. This can be accomplished, for example, by retracting the spring, restricting the further expansion or contraction of the spring, or blocking or closing the flow restrictor, e.g., the glass capillary or narrow bore tubing.

The spring of the invention is preferably a constant force spring. To minimize the change in flow rate as the solid or fluid is delivered, it is preferred to utilize sufficiently high tension in the spring such that the difference between the starting and ending force applied by the spring is less than 30%, 20%, or 10% of the starting force.

To minimize the change in drug administration rate when the patient drinks a hot beverage, it is preferred to utilize spring materials whose force is relatively independent of temperature in the range of 37 - 55 °C. For example, the force in a fresh reservoir may increase by less than 30%, 20% or 10% when the temperature is raised from 37 to 55 °C.

The springs of the invention may be any type of spring, including traditional metal springs or a compressible elastomer. For example, the compressible elastomer may be a solid such as isoprene, or it may contain closed gas cells (e.g., neoprene).

An example of a spring-driven, fully disposable, constant-rate medication infusion pump with flow restrictor is the Valeritas V-go insulin patch pump, described in U.S.S.N. 13/500,136The V-go pump includes two powerful springs that expand as they relax; in the event of a structural failure, the springs could cause the device to explode. For spring-driven, intra-oral drug delivery devices it is preferable to use springs that are less powerful and that contract instead of expanding upon relaxation, thereby reducing the risk of an explosive failure.

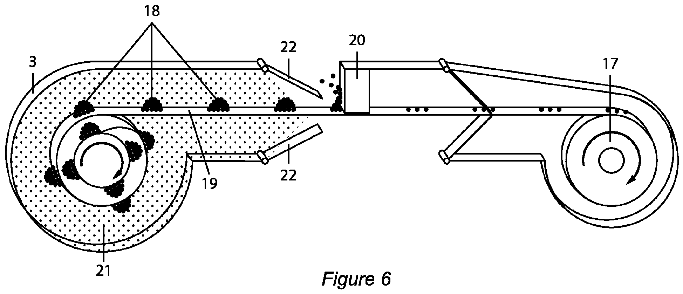

In an embodiment of a spring-driven pump, the mechanical advantage of a wound rotary spring is used as a motor to drive a mechanical system to deliver the drug. For example, Figure 6 illustrates a conveyor-belt driven by a spring motor **17** that delivers discreet solid doses **18** (e.g., pills, granules, pellets, particles, etc.) carried on a backing material **19.** The spring-driven mechanism transports the solid drug out of the dry oral liquid impermeable reservoir on the backing material to a position in which it is exposed, and the drug may then be released from the backing material by interference with a stationary post **20.** The reservoir **3** containing the solid drug on the conveyor-belt can optionally be filled with a non-aqueous fluid **21** that does not interact with the drug, and which would serve to prevent the solid drug from coming in contact with saliva (through the use of a duck bill valve **22**) prior to its intended administration time, which might otherwise clog the system or degrade the drug. A related embodiment includes the use of an edible backing material with the discreet solid doses screen-printed onto it. This embodiment eliminates the need to contain the depleted conveyor belt material. Another embodiment includes the use of a series of compartments filled with drug and a peel-away backing material that exposes each compartment individually. This embodiment eliminates the possibility of drug coming in contact with saliva since only one compartment would be exposed at a time. A further advantage of the use of a constant force radial spring is that, for example, a relatively low force (2,224 N (0.5 IbF)) spring can be used to deliver the drug through a relatively large orifice (-0.4mm diameter). In the event that the device fails while being worn in the mouth, the stored potential energy of the spring, while low, will cause the spring to retract onto itself; compression springs, conversely, will have a stored potential energy that will force the spring to relax to its expanded state, potentially causing harm to the user. An example of a constant force spring is product number SH6F24 manufactured by Vulcan Spring and Mfg Co, (501 Schoolhouse Rd, Telford, PA 18969). Preferred springs deliver forces of less than 44,482, 22,241 or 4,448 N (10, 5, or 1 IbF) and retract upon relaxation.

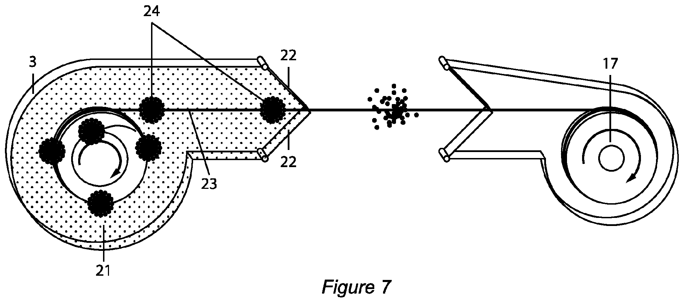

Another embodiment, illustrated in Figure 7, is the use of a spring-driven motor **17** to advance a string **23** to which solid drug is attached (e.g., in the form of multiple discreet solid pills **24**), transporting the solid drug out of the dry oral liquid impermeable drug reservoir **3** to a position in which it is exposed to saliva in the mouth, where the solid drug can dissolve or disperse. Alternatively, the drug reservoir **3** containing the solid pills **24** can be filled with a non-aqueous fluid **21** that does not interact with the drug, and which would serve to prevent the solid drug from coming in contact with saliva, through the use of a valve **22,** prior to its intended administration time, which might otherwise clog the system or degrade the drug. The advantage of using a dispersible solid drug is that the solid drug cannot accidentally be inspired into the lungs, ejected from the mouth, or trapped in a location in the mouth where it will not be exposed to saliva.

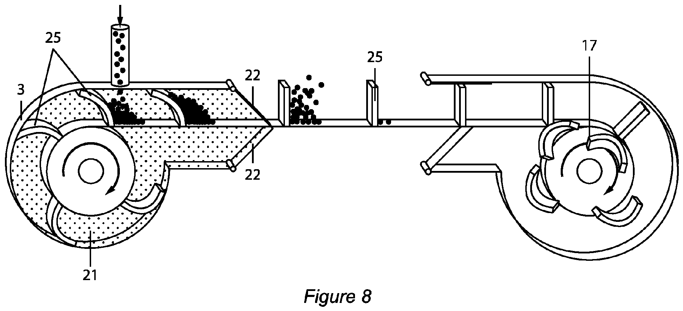

A further embodiment, illustrated in Figure 8, is the use of a spring-driven motor **17** and a series of squeegees **25** that propel the drug from the dry oral liquid impermeable drug reservoir **3**, through a duckbill valve **22**, into the mouth. Alternatively, the drug reservoir **3** containing the solid drug can be filled with a non-aqueous fluid **21** that does not interact with the drug, and which would serve to prevent the solid drug from coming in contact with saliva.

In embodiments in which the drug is delivered into the mouth via a tube or channel, the the oral liquid impermeable drug reservoir may be kept free of oral liquids by using a tube or channel coated with a hydrophobic or non-stick material (e.g. paraffin or PTFE), and/or designed with a diameter that would require a sufficiently high pressure so as to not allow saliva to enter.

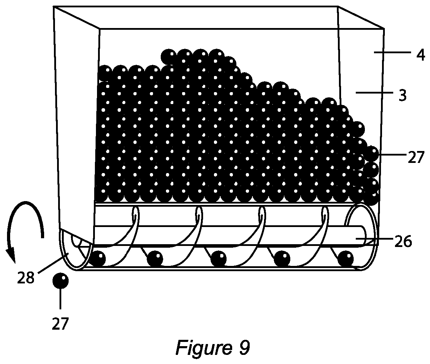

An alternative embodiment for delivery of a solid drug, illustrated in Figure 9, includes the use of a spring-driven auger **26** to capture and deliver a solid drug, e.g., a discreet solid granule, capsule or pill **27.** The auger can be configured so that one end is located within the dry, oral liquid impermeable drug reservoir **3** containing the drug in solid form within the housing **4**, and the opposite end exposed to an opening **28** that allows the drug to be delivered into the mouth at the desired rate. The advantages of the auger **26** are that the motion and materials of the auger prevent the saliva from penetrating into the oral liquid impermeable reservoir, and the considerable force that can be generated by an auger can break any solid particles that may have been exposed to saliva and conglomerated.

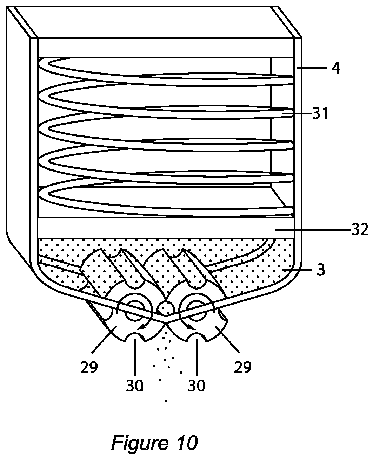

Another embodiment of a spring driven drug pump, illustrated in Figure 10, includes the use of a spring motor to rotate two columnar or conical shaped drums **29** that are attached to the oral liquid impermeable drug reservoir **3** containing a solid drug. The drums **29** are constructed of a hydrophobic or non-stick material, and can be configured with a tight tolerance to prevent introduction of saliva into the reservoir. The rotation of these drums can draw the drug particles from the drug reservoir **3**, through the drums **29,** and into the mouth. The drums can be configured such that a cutout **30** defines the dosage, and the frequency of rotation of the drums **30** defines the drug delivery rate. In another embodiment, the cutout **30** would not be present and the spacing between the drums **29** along with the speed of rotation of the drums **29** would define the drug delivery rate. In order to maintain constant feeding and eliminate the potential for gaps of drug to the drums, a spring **31** and piston **32** are employed within the housing **4.**

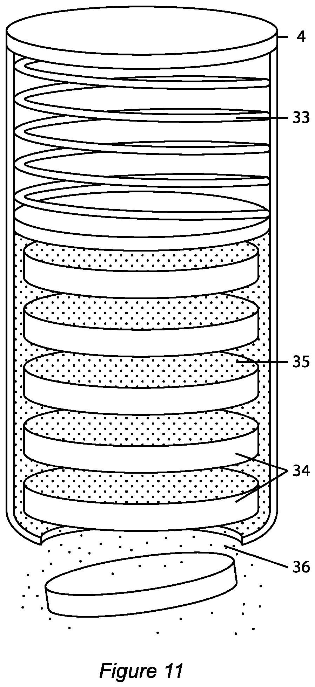

In yet another embodiment, illustrated in Figure 11, a spring **33** pushes a series of solid drug doses in the shape of disks **34,** each dose separated by a thin layer of a material **35** that prevents the saliva from contacting the disks **34** prematurely. The distal drug dose is exposed to saliva in the mouth, where it dissolves or disperses. The material between the drug doses then dissolves or disperses, allowing the saliva to contact the next drug dose. At any time, only the distal drug dose can be exposed to saliva while exiting the orifice **36.**

In yet another embodiment, a series of solid drug doses are arranged in a spiral-shaped cylinder. In this embodiment, a one-way valve is positioned at the exit of the reservoir, serving to protect the pills from exposure to saliva. In this embodiment, the pills are in direct contact with a low friction coefficient wall. Since the force required to push the pills is directly proportional to the normal force of the pills on the wall (and the friction factor) and is a linear relationship, a compression spring of a specific spring rate can be used to deliver the pills at a constant rate. For example, a compression spring governed by Hooke's Law follows the relationship Fs=Kx, where Fs is the spring force, K is the spring rate and x is the distance the spring is compressed. The force required to push the pills through the one-way valve is proportional to the number of pills by the weight of the pills and the frictional force associated to that number of pills. The governing equation for frictional force in this case, is F_{F}= µN, where F_{F} is the frictional force, µ is the coefficient of friction, and N is the normal force, in this case proportional to the weight of the pills. Therefore, a spring with a spring rate proportional to µN/x would deliver the pills at a constant rate.

In a further embodiment of a spring-driven pump, Figures 12A and 12B illustrate an embodiment in which a constant force spring is used to pull a compression plate toward an orifice. A flexible oral liquid impermeable reservoir within a housing contains the drug. The end of the spring rides along a track on the inside of the housing. Figure 12A, shows the location of the spring **37,** spring axle **38** and compression plate **39** when the reservoir **3** is full and the spring **37** is fully extended. Figure 12B shows the location of the compression plate **39** and spring **37** when the retraction of the spring **37** has delivered all of the drug from the reservoir **3..** In a related embodiment, the drug can be contained within the housing itself and the compression plate would create a seal and act as a plunger to deliver the drug in a manner similar to a syringe. In this embodiment, the spring rides inside of the housing and inside of the drug chamber, within a sealed sleeve, protecting the drug from exposure to the spring. In order to eliminate the effect of changes in ambient pressure on the drug delivery rate, a vent hole **15** is present within the device to allow both the drug reservoir **3** and the drug reservoir nozzle **8** to be exposed to ambient pressure, which eliminates the effect of any changes in ambient air pressure (e.g., due to the patient sucking on the device and/or changes in altitude).

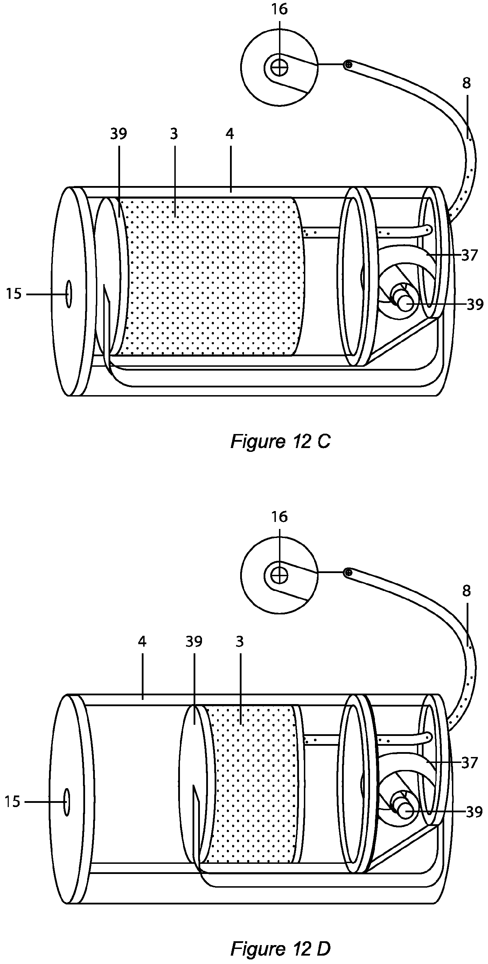

In another embodiment illustrated in Figures 12C and 12D, a constant force spring **37** remains fixed in space; one end of the spring **37** is attached to a compression plate **39,** and pulls the compression plate **39** toward the drug reservoir nozzle **8.** Figure 12C shows the location of the spring **37** and compression plate **39** when the drug reservoir **3** is full and the spring **37** is fully extended. Figure 12D shows the location of the compression plate **39** and spring **37** when the retraction of the spring **37** has delivered all of the drug from the reservoir **3.** Figures 12C and 12D also have a vent hole **15** incorporated into the design, to eliminate any effect of ambient pressure on the drug delivery rate.

In a further embodiment of a solid dose drug delivery, a series of pills **41,** aligned serially, are pulled by a motor **42** toward a feature that causes them to drop out of the housing. This embodiment can use a spring-driven, elastomer-driven, gas-driven, or any other power source taught herein. Figure 13 shows the solid pills 41 arranged along a line, being pulled by a motor **42** attached to a lead **44,** with a pusher **45** on the opposite end. As the motor **42** reels the lead **44,** onto the takeup reel **46,** the solid pills are pulled toward the opening **43** and are ejected into the user's mouth. A valve at the opening **45** protects the micro pills from premature exposure to saliva. A lubricating liquid **47,** allows the pills to move smoothly within the drug reservoir **3** and prevents ingress of saliva into the drug reservoir.

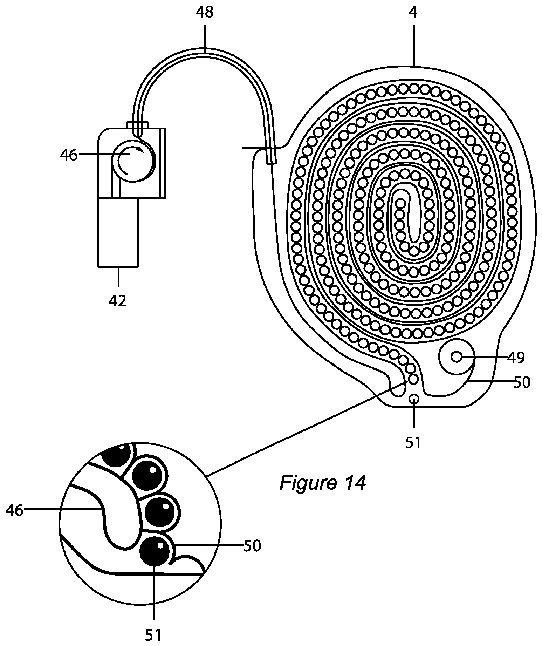

Figure 14 shows another embodiment of solid dose drug delivery in which the drug doses are individually packaged in order to ensure that individual doses of the drug are delivered at a specific frequency and also to ensure that they are protected from premature exposure to saliva. In this embodiment, a motor **42** pulls on the backing material **48** of the sealed package and a radial spring **49** maintains tension on the container **50** in order to separate the drug dose. As the motor continues to pull on the backing material, it is stored on the takeup reel **46.** As each drug dose **51** is exposed, it will be delivered into the user's mouth. In this embodiment, the drug dose **51** can take the form of a single pill, multiple micropills or drug in particle or powder form. While illustrated using a spring-driven motor, this embodiment can use a spring-driven, elastomer-driven, gas-driven, or any other power source taught herein.

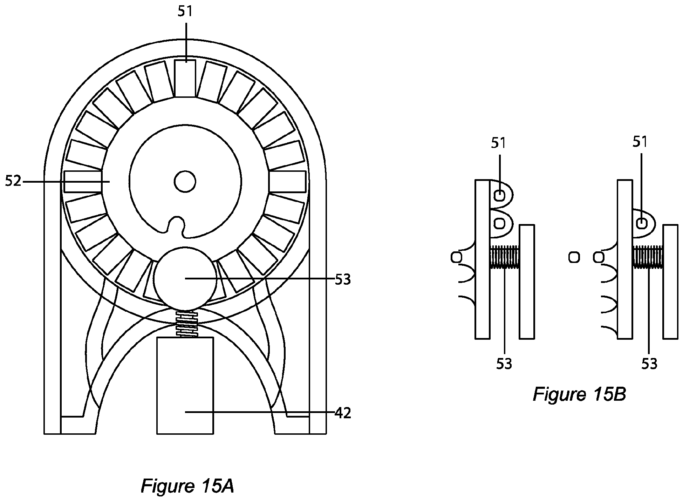

Figures 15A and 15B show another embodiment of solid dose drug delivery. In this embodiment, the drug reservoir is a rotating cartridge **52** containing a plurality of individually packaged drug doses **51** that are delivered individually at a specific frequency by the rotation of the cartridge **52.** The rotation causes the solid drug to be pushed through the packaging and into the user's mouth by a spring-loaded feature **53.** In this embodiment, a motor **42,** rotates the drug reservoir cartridge **52** at a specific rate until a single drug dose **51** contacts a spring loaded feature **53** which imparts a force on the drug compartment backing, forcing the drug **51** through the packaging, and out of the drug reservoir **52.** While illustrated using a spring-driven motor, this embodiment can use a spring-driven, elastomer-driven, gas-driven, or any other power source taught herein.

In a further embodiment of a spring pump, a compression spring can be used to apply an approximately constant force to a piston or plunger that applies that force to the drug reservoir. Using a very long compression spring with a low spring rate, one could apply a force across a short stroke with relatively constant force. As an example, a 25,4 cm (10 inch) long spring with a spring rate of 0,0876 N/cm (0.05 IbF/in) would be compressed to 21,59 cm (8.5 inch) and would apply a force of 1,89 N (0.425 lbF). If the spring were allowed to expand to 19,05 cm (7.5 inches) (a 2,54 cm (1 inch) total stroke), the resulting force would be 1,668 N (0.375 IbF), which is a decrease of 12.5% throughout the stroke. In preferred embodiments, the spring force is in the range of 1,112-44,482 N (0.25-10 IbF) and is preferably less than 44,482, 22,241, or 4,448 N (10, 5, or 1 IbF); the spring rate is in the range of 0,0175-1,751 N/cm (0.01-1 IbF/inch) and is preferably less than 1,751, 0,876, or 0,0876 N/cm (1, 0.5, or 0.05 IbF/inch); the stroke length is in the range of 1,27-2,54 cm (0.5-1 inch) and is preferably less than 5,08, 2,54, or 1,27 cm (2, 1, or 0.5 inch); and the difference between the starting and ending force across the stroke is less than 15%, 10%, or 5%.

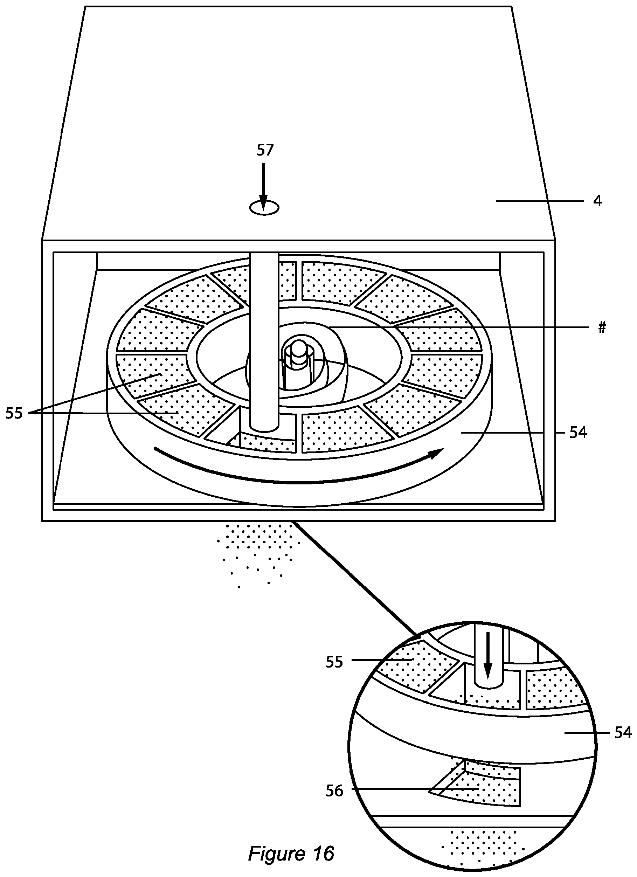

*Pneumatic pumps.* Pneumatic pumps generate a driving force using a pressure head of air. In one embodiment, a diaphragm pump generates a pressure head that pushes a discreet amount of drug, in solid form (e.g., particles, granules or powder), from a reservoir and into the mouth. An example of such a design, illustrated in Figure 16, is a rotating disk **54** that contains compartments filled with drug particles **55** that are injected by an air pressure bolus **57** at a pre-determined rate through an orifice **56** that is fixed in place with respect to the rotating disk **54.** The rotation of the disk **54** exposes a single compartment and the bolus of air **57** delivers the drug from that compartment to the mouth at a specific rate. The housing can be formed from a clear material that would allow the user to observe how much drug remains in the device. In another embodiment, the disk can contain a single compartment that rotates and alternately fills the compartment from the reservoir and delivers the drug with a bolus of air. In this configuration, the air not only delivers the drug material, but also removes any saliva prior to refilling the compartment from the reservoir.

*Negative pressure pumps.* Negative-pressure pumps generate a driving force from the pressure difference across two sides of the pump's low-pressure chamber wall, with one side being at very low pressure (inside a vacuum chamber) and another side being at atmospheric pressure. The very low pressure in the vacuum chamber may be created during the reservoir filling process. Expansion of the oral liquid impermeable reservoir, caused by the addition of fluid to the drug-containing reservoir, causes simultaneous expansion of the reduced pressure chamber, thus creating a significant vacuum. During administration of the solid or fluid drug, pressure on the movable wall plunger is generated by the large pressure difference between its two sides, causing it to move and compress the solid or fluid in the drug-containing chamber.

A significant advantage of negative pressure pumps for use in the mouth is that it is possible to temporarily stop the drug delivery from the device if the patient wishes to temporarily remove the drug delivery device from the mouth. This can be accomplished, for example, by blocking or closing the flow restrictor, e.g., the glass capillary or narrow bore tubing.

*Gas-driven infusion pumps.* In one embodiment, a gas-driven drug delivery device includes two or more compartments, with pressurized gas in at least one compartment and the solid or fluid drug to be administered in at least one separate oral liquid impermeable drug reservoir. The pressurized gas provides the driving force. The two compartments are separated by a movable member that transmits the force from the gas compartment to the solid or fluid.

The housing containing the two compartments is typically constructed to be a fixed volume that does not vary significantly as the drug is dispensed and the internal pressure declines in the compartment containing the pressurized gas. An example is a reservoir in the shape of a syringe barrel including: a fluid dispensing orifice at the distal end of the syringe barrel; a sealed proximal end of the syringe barrel; a mobile rubber or elastomeric plunger in the syringe barrel, which separates the syringe barrel into two compartments; a drug-including fluid located in the distal compartment; and a pressurized gas in the proximal compartment. In another example, the drug compartment may have a bellows shape and may be surrounded by the gas compartment, such that the pressurized gas compresses the bellows and forces the drug-including fluid through a flow restrictor.

Figures 17A, Band C illustrate another embodiment, wherein a first elastomeric drug reservoir **3** is compressed by a second elastomeric compartment **7** containing gas or propellant. In Figure 17A, the drug delivery device includes a housing containing a first, full elastomeric drug reservoir **3**; a second empty, elastomeric compartment **7**; and an optional gas pump **11** and electronics. In one embodiment, which is not according to the invention and present herein for illustration purposes only, air and/or saliva is pumped by the electronic (e.g., piezoelectric) pump **11** into the second elastomeric reservoir **7.** In another embodiment the second elastomeric reservoir **7** contains a compressed gas or propellant, and no pump is required. In either embodiment, the pressure from the second elastomeric reservoir **7** compresses the first elastomeric reservoir containing the drug **3**, forcing the drug out of the reservoir through a flow restrictor **58** at a constant rate. Figure 17B illustrates the system when the drug reservoir **3** is half-full. Figure 17C illustrates the system when the drug reservoir **3** is close to empty.

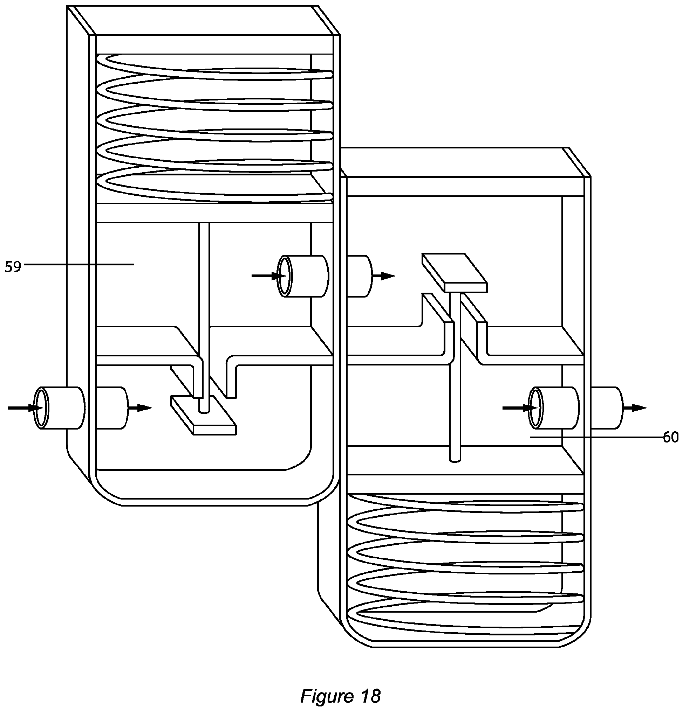

In one embodiment, a gas (e.g., carbon dioxide, nitrogen) is contained in a miniature gas cartridge or cylinder. The gas cartridges have an external volume of less than or equal to 5, 2, or 1 mL and have stored pressures of 689,476-3447,379(100-500), 3447,379-6894,757 (500-1000), 6894,757-2757903 (1000-4000), or greater than 2757903 kPa (4000 PSI). Exemplary gas cartriges are product numbers 40106 (1.00" CO₂ Filled; 0.75 grams) and 40106IIN21750 Nitrogen cylinder (1.00" N₂ Filled; 0.135 grams) manufactured by Leland Gas Technologies (2614 South Clinton Ave, South Plainfield, NJ 07080) and product number SM-2 (5/32" Single Acting, Spring Return, Sub-Miniature Cylinder) manufactured by Clippard Instrument Laboratory, Inc. (7390 Colerain Avenue, Cincinnati, OH 45239). The gas from the miniature cartridge or cylinder can be used to compress the oral liquid impermeable drug reservoir, thereby delivering the drug. The gas-pressurized cartridge can be used in conjunction with a one or two-stage regulator in order to provide a constant pressure gas flow as the drug reservoir is emptied. Figure 18 shows a schematic diagram of a standard commercially available two-stage regulator. Examples of miniature two-stage regulators are the product categories PRD2 and PRD3 manufactured by Beswick Engineering Co, Inc.(284 Ocean Rd, Greenland, NH 03840-2442). A two-stage regulator is used to gradually reduce the pressure from high to very low, in this example from the cartridge to the piston chamber of the pump. The first stage **59** reduces the gas pressure to an intermediate pressure. The gas at that intermediate pressure then enters the second stage **60** and is further reduced by the second stage **60** to the working pressure. In a related embodiment, a gas cartridge contains an optionally reversibly COz-absorbing or adsorbing solid that maintains, e.g. in its Henry region, an about constant CO₂ pressure at about 37°C. The reversibly COz-absorbing or adsorbing solid can be, for example, a high specific surface activated carbon, silica, e.g., silica gel, modified with n-propylamine or with another amine or heterocyclic nitrogen compound. The BET (Brunauer-Emmett-Teller) specific surface of the materials can be greater than 50 m²/g such as, between 50 and 500 m²/g, or greater than 500 m²/g. The materials can contain more than 0.5 millimoles of amine functions per gram, for example between 1 and 5 millimoles of amine functions per gram. Exemplary reversibly COz-absorbing or adsorbing solids are described, for example, by Z. Bacsik, N. Ahlsten, A. Ziadi, G. Zhao, A. E. Garcia-Bennett, B. Martin-Matute, and N. Hedin "Mechanisms and Kinetics for Sorption of COz on Bicontinuous Mesoporous Silica Modified with n-Propylamine" Langmuir 2011, 27, 11118-11128 and in the references cited by Bacsik et al. The materials may also be in the MIL-53 family of soft porous crystals, such as MIL-53(AI), MIL-53(Al)-11.1%NH2, MIL-53(AI)-20%NHz, MIL-53(AI)-50% NH₂, MIL-53(AI)-66.7% NH₂, and MIL- 53(AI)- NH₂, as described by M. Pera-Titus, T. Lescouet, S. Aguado, and D. Farrusseng "Quantitative Characterization of Breathing upon Adsorption for a Series of Amino-Functionalized MIL-53" (J. Phys. Chem. C 2012, 116, 9507-9516). In general, the reversibly COz absorbing amine-modified carbon, zeolite, silica or silica gel adsorbs COz when the silica also contains bound water. The materials may also comprise high surface area carbon or activated carbon as described for example in "Fixed bed adsorption of CO2/H2 mixtures on activated carbon: experiments and modeling" by N. Casas, J. Schell, R. Pini, M. Mazzotti Adsorption (2012) 18:143-161 and "Pure and binary adsorption of COz, H2, and N2 on activated carbon" by J Schell, N Casas, R Pini, M Mazzotti in Adsorption (2012) 18:49-65.

The materials may provide an about constant COz pressure of greater than 101,325 kPa (1 atm), for example between 121.59 (1.2) and 202.65 kPa (2.0 atm), or between 202.65 (2.0) and 506.625 kPa (5.0 atm), or between 506.625 kPa (5 atm) and 2026.5 kPa (20 atm).

In yet another related embodiment the gas cartridge may contain a solid metal hydride, providing at about 37°C an about constant hydrogen pressure. The metal hydride may include an alloy, for example of a rare earth like lanthanum, and a transition metal like nickel, and may also include magnesium.

In some embodiments, the pressurized gas remains in the gaseous state through the temperature range of 0°C - 37°C. A disadvantage of such embodiments is that the drug infusion rate tends to decline as the drug is dispensed because the gas pressure declines as the gas expands. For this reason, it is preferred to utilize sufficiently high gas pressures in the pump such that the difference between the starting and ending gas pressure is less than 30%, 20%, or 10% of the starting gas pressure.

To minimize the change in flow rate when the patient drinks a hot beverage, it is preferred to minimize the volume of the gas relative to the volume of the drug-including fluid. The volume of the gas can be less than 40%, 30%, 20% or 10% of the volume of the drug-including fluid in a fresh reservoir. For example, the force in a fresh reservoir may increase by less than 30%, 20% or 10% when the temperature is raised from 37 to 55 °C.

In another embodiment, the drug delivery device includes a volatile propellant in one compartment and the drug in a second compartment, the propellant boiling at sea level atmospheric pressure at a temperature less than about 37°C C and optionally boiling at sea level atmospheric pressure at a temperature greater than about 15°C, with the propellant under such pressure that it is present in both its liquid and gaseous states at 37°C. In this embodiment, a propellant-driven drug delivery device can include an oral liquid impermeable drug reservoir with a pressure-liquefied propellant, i.e., a propellant-containing compartment within the drug delivery device, such that the pressurized, volatile, propellant liquid and the solid or fluid including the infused drug reside in the different compartments. Optionally, the wall material of the propellant-containing compartment can be an elastomer, allowing for expansion of the propellant-containing compartment as the drug-containing fluid is depleted. Typically, the propellant is a gas at 101,325 kPa (1 atm) pressure at 37°C and maintains an about constant pressure when the drug including formulation is infused in the mouth. In an embodiment shown in Figures 19A and B, the gas compartment is encapsulated by an elastomeric membrane **61** and reside within the oral liquid impermeable drug reservoir **3.** The propellant exerts a nearly constant pressure on the elastomeric membrane **61** as the elastomeric membrane **61** expands and pushes the solid or fluid drug from the oral liquid impermeable drug reservoir **3** through a narrow-bore tubing **8.** The narrow bore tubing may serve as a flow restrictor to control the delivery rate, or there may be a separate flow restrictor. Figure 19A shows the compressed elastomeric compartment **61** containing propellant within the full drug reservoir **3.** Figure 19B shows the nearly empty drug reservoir **3** and the expanded elastomeric compartment **61** containing propellant. The advantage of this embodiment is that the drug delivery rate does not decline as the drug is dispensed.

In a further embodiment, the gas can be contained in a gas-impermeable, non-flexible material, such as metallized Mylar^{®}, which is folded such that the expansion of the gas unfolds the gas compartment and allows the pressurization of the solid or fluid drug to occur. Optionally, the unfolding compartment can be coil or bellows-like.

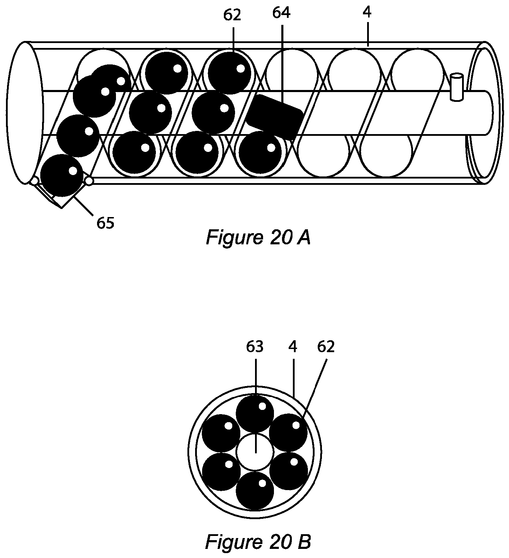

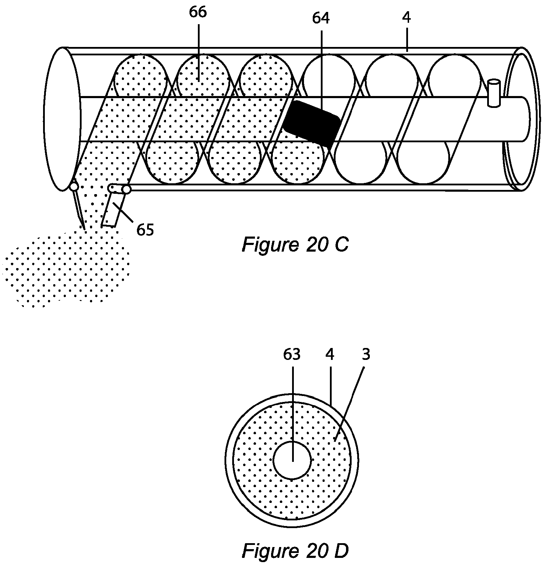

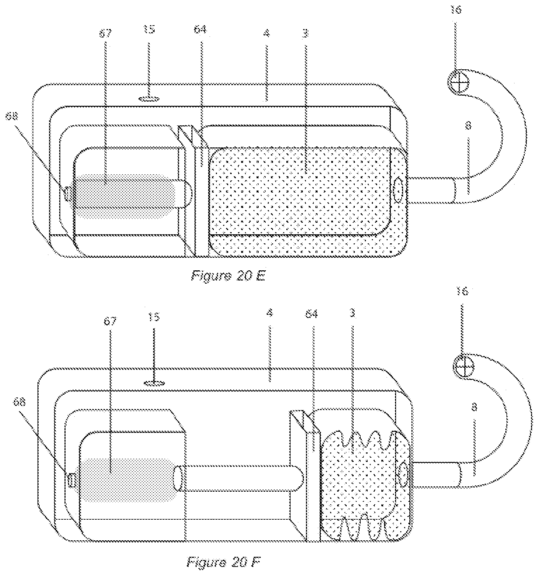

In another embodiment of a gas-driven pump, a propellant can be used to deliver a drug-comprising fluid (e.g., a solution or a suspension) or a series of solid unit drug doses. Figure 20A illustrates an approximately cylindrical drug reservoir **4** containing an ascending spiral of solid doses **62** around the circumference, bathed in an edible oil or other safe, edible fluid. Preferably, the solid unit drug doses and saliva are not substantially soluble in the edible oil fluid. An interior, approximately cylindrical core **63**, shown in Figure 20B, contains the propellant. When placed in mouth and held at a substantially constant temperature, the propellant applies a substantially constant pressure to a plunger **64.** At the distal end of the spiral drug reservoir, the solid unit drug doses **62** are released from the device into the mouth, optionally through a valve **65** that reduces or prevents the entry of saliva into the drug reservoir. For the drug delivery device to deliver drug at a substantially constant rate it is necessary that the piston, plunger or plug move at a constant rate, which requires that resistance to movement remain substantially constant from the time the drug reservoir is substantially full until it is substantially empty. This is problematic in that the resistance to movement will naturally drop as the drug reservoir is emptied. A solution to this problem is to make the resistance to movement of the piston, plunger or plug much greater than the resistance to movement of the column of solid unit drug doses. An example of a means for creating a relatively high resistance to movement of the piston, plunger or plug is to use an elastomeric piston, plunger or plug in a hard-walled spiral drug reservoir. Examples of means for creating a relatively low resistance to movement of the column of solid unit drug doses are to include an edible lubricant (e.g., an edible oil) in the drug reservoir, to make the solid unit drug doses substantially round, and to provide sufficient dimensional clearances the spiral drug reservoir so that the the solid unit drug doses are not held tightly. Preferably, the resistance to flow of the piston, plunger or plug is greater than or equal to about 2, 4, 6, or 10 times the resistance to flow of the solid unit drug doses when using a fresh drug reservoir. It will be apparent that a system of this design can also be used to deliver a drug-comprising fluid. In another similar embodiment, illustrated in Figures 20C and D, the propellant pushes the plunger **64** which alternatively applies a constant pressure to a column of drug in suspension form. The flow rate of the drug suspension **66,** is dictated by the resistance of the drug reservoir **3**, and will change as the drug reservoir **3** is emptied. To address this issue, the resistance of the plunger should be sufficiently greater than the resistance of the suspension maintain the flow rate within the desired tolerance. In another embodiment, a vent within the housing of a propellant driven piston allows the piston to be exposed to ambient pressure, thereby eliminating the effect of changes in ambient pressure on the flow rate of the drug. This embodiment is illustrated in Figures 20E and 20F. Figure 20E shows the drug reservoir **3** in its full state. The piston **64** is positioned against the drug reservoir 3 on one end and within the propellant chamber **67** on its opposite end. The piston **64** forms a seal with the propellant chamber **67** such that the propellant is allowed to pressurize and maintains within the volume created by the propellant chamber **67** and the piston **67.** As the propellant is exposed to body temperature, the propellant pressurizes pushing the piston **64** against the drug reservoir **3.** A vent **15** maintains ambient pressure around the drug reservoir **3.** Figure 20F shows the device after some time has elapsed and the collapsible drug reservoir **3** has emptied some of its contents. A filling septum **68** is located on the opposite end of the piston **64** allowing filling of the propellant chamber **67.**

In a further embodiment, the drug delivery device includes a propellant and a drug together in the same compartment. The propellant typically boils at 101,325 kPa (1 atm) pressure at a temperature greater than about 15°C and less than about 37°C, with the propellant present in both its liquid and gaseous states at 37°C. In this embodiment, a propellant-driven drug delivery device can include an oral liquid impermeable drug reservoir with a pressure-liquefied propellant, i.e., volatile liquid propellant in the reservoir, such that both the pressurized, volatile, propellant liquid and the solid or fluid including the infused drug reside in the same compartment. The propellant maintains an about constant pressure when the drug including formulation is infused in the mouth.

Because separation or segregation of the liquid propellant and the drug formulation could lead to oral delivery of propellant-enriched or prepellant-poor fluid and hence to lesser or greater than intended drug dosing, the liquid propellant can be dissolved or co-dispersed in the drug formulation. The propellant liquid can be present, for example, as an oil-in-water emulsion, formed optionally by adding an emulsifier, such as a lecithin, or by Pickering emulsification, where small solid drug or other particles stabilize the emulsion. In general, the emulsions are stable for at least 24 hours and can be re-formed by agitation, for example by shaking. The optionally oil-in-water emulsions can be foamable or non-foamable and can include an emulsifier such as lecithin, a protein, or a surfactant that can be non-ionic, including for example a glyceryl monoester, like glyceryl monooleate, a Tween or a Polysorbate. Examples of emulsifiers in propellant including mixtures are listed for example in U.S. Patent No. 6,511,655 and in U.S. Patent Publication No. 2003/0049214. Alternatively the liquid propellant can be dissolved in the carrier liquid of a solid drug comprising formulation, e.g. when the carrier liquid is non-aqueous, for example when it is edible oil or medicinal paraffin oil. The propellant dissolving carrier liquid may optionally be a temperature sensitive liquid such as cocoa butter.

As the drug is dispensed and the internal pressure falls in the gas compartment, the volatile compound boils and replaces the lost gas within the gas compartment, thereby maintaining a nearly constant pressure within the oral liquid impermeable reservoir. The advantage of such an embodiment is that the drug infusion rate does not decline as the drug is dispensed.

In a related embodiment, a gas-driven drug delivery device includes an oral liquid impermeable drug reservoir having one or more compartments, with a non-toxic, propellant gas, formed from the optionally substantially immiscible pressurized liquid when the pressure is reduced to about 101,325 kPa (1 atm), and the drug to be infused both present in at least one compartment. The propellant gas provides the driving force. The pressure liquefied gas can be optionally be insoluble in the fluid containing the drug, such that the pressure in the reservoir remains about constant at the about constant body temperature near 37°C in the mouth.

Alternatively, the pressurizing gas can be soluble in the drug or prodrug-including fluid. For example, when the fluid infused in the mouth is aqueous, or when it includes ethanol, and the reservoir is pressurized, the pressurizing gas can be COz. When the fluid infused in the mouth includes an edible oil such as a vegetable oil, a monoglyceride, a diglyceride or a triglyceride, or paraffin oil, and the reservoir is pressurized, the pressurizing gas can be a flurorohydrocarbon, a Freon^{™}, or a saturated hydrocarbon or a non-saturated hydrocarbon such as an olefin. When the pressurizing gas dissolves in the fluid in the oral liquid impermeable reservoir the pressure can be about constant at the constant about 37°C temperature in the mouth, making the flow rate about constant.

Examples of continuously subcutaneously drug infusing compressed air or Freon^{™} pressurized pumps include those described in U.S. Patent Nos. 4,265,241, 4,373,527, 4,781,688, 4,931,050, 4,978,338, 5,061,242, 5,067,943, 5,176,641, 6,740,059, and 7,250,037. When the reservoir is refillable and when the pumping is by pressurization, the reservoir can be pressurized upon its refilling.

An example of a propellant-driven, implanted medication infusion pump is the Codman pump described in U.S. Patent No. 7,905,878, European Patent Nos. EP 2177792 B1 and EP 1527794 B1.

To provide different patients with different dose rates, fluids with different drug concentrations can be placed in the reservoirs, thereby not necessitating modifications to the drug delivery device or to the flow rate. Alternatively, the drug concentration in the reservoir can be held constant and the flow rate can be changed, for example by changing the diameter or length of the flow restrictor.

Exemplary volatile propellant compounds for use in the devices of the invention include hydrocarbons (e.g., pentane; isopentane; 1-pentene; trans-2-pentene; trans-dimethylcyclopropane; ethylcyclopropane; 1,4 -pentadiene; 2-methyl-1,3-butadiene; and methyl-1-butane; 2-butyne); halocarbons (e.g., trichlorofluoromethane; 1,1-dichloro-1-fluoroethane; 2,2-dichloro-1,1,1-trifluoroethane; 1-fluorobutane; 2-fluorobutane; perfluoropentane; 1,1-dichloroethylene; cis-1-chloropropene; and 2-chloropropene); esters (e.g., methyl formate); ethers (e.g., diethyl ether), and hydrofluoroalkanes. Preferred propellants are those approved by the FDA for use in medication inhalers, such as 1,1,1,2 tetrafluoroethane (sold as DuPont^{™} Dymel^{®} I134a/P); and 1,1,1,2,3,3,3 heptafluoropropane, sold as 227ea/P (sold as DuPont^{™} Dymel^{®} 227ea/P). Also preferred are propellants approved by the FDA for topical applications, such as 1,1,1,3,3,3 hexafluoropropane (sold as DuPont^{™} Dymel^{®} 236fa); and propellants approved for use in food and over the counter anticarie drug products, such as octafluorocyclobutane and isopentane, respectively.

Exemplary pressurized liquid propellants and their vapor pressures at 37 °C are listed in Table 1.

**Table 1. Exemplary propellant liquids pressurizing the drug delivery device residing in the mouth and their vapor pressures at 37°C**

| Propellant | Approximate Pressure, kPa (bars) at 37°C |
|---|---|
| diethyl ether | 110 (1.1) |
| 1-fluorobutane | 130 (1.3) |
| isopentane | 140 (1.4) |
| 2-fluorobutane | 160 (1.6) |
| 1,2-difluoroethane | 190 (1.9) |
| neopentane | 240 (2.4) |
| methyl ethyl ether | 300 (3) |
| 2-butene | 320 (3.2) |
| butane | 350 (3.5) |
| 1-fluoropropane | 410 (4.1) |
| 1-butene | 420 (4.2) |
| 2-fluoropropane | 500 (5) |
| 1,1-difluoroethane | 840 (8.4) |
| propane | 1280 (12.8) |
| propene | 1550 (15.5) |
| 1,1,1,2 tetrafluoroethane | 930 (9.3) |
| 1,1,1,2,3,3,3 heptafluoropropane | 460 (4.6) |
| 1,1,1,3,3,3 hexafluoropropane | 400 (4.0) |
| octafluorocyclobutane | 430 (4.3) |

When the pressurized gas and the drug are located in the same compartment, the gas can be selected to be safe, non-toxic, and non-irritating when delivered into the mouth and inhaled into the lungs at the delivery rates of the invention. Furthermore, the gas can be selected so as not to adversely affect the stability of the drug and formulation in the reservoir. Inert gases are therefore preferred.

A source of inaccuracy in propellant pressurized devices is that the pressure, such as the vapor pressure of a liquid propellant, increases with temperature. An important benefit of carrying within the mouth the drug delivery devices of the invention is that the pressure is held nearly constant at about 37°C, thereby minimizing variations in the infusion rate.

In another embodiment, gas is generated by the gas-driven drug delivery device. For example, a very low current electrolyzer may be used to generate hydrogen gas. Exemplary hydrogen gas generating systems are the hydrogen gas generating cells sold by VARTA Microbattery GmbH Daimlerstr. 1, D-73479 Ellwangen/Jagst Germany. The VARTA systems are capable of generating 130 ml, 260 ml or more ultrapure H₂ at high back pressure. An advantage of such a system is that gas or propellant need not be stored in the drug delivery device prior to its use.

A significant advantage of gas-driven infusion pumps for use in the mouth is that it is possible to temporarily stop, or greatly reduce, the drug delivery from the device if the patient wishes to temporarily remove the drug delivery device from the mouth. This can be accomplished, for example, by blocking or closing the flow restrictor, e.g., the orifice, the glass capillary or the narrow bore tubing or by cooling to a temperature below that in the mouth, for example to the typically 20°C -25°C room temperature or by placing the device in a refrigerator typically at 3°C -8°C.

*Osmotic delivery pumps (non-electric).* Osmotic devices that do not require electricity for delivery of drugs are known in the literature.

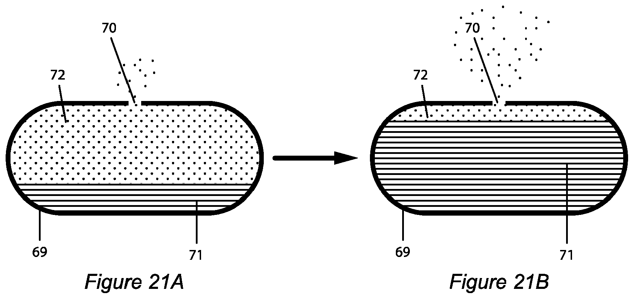

Examples of steady-state zero order osmotic delivery technology are the Swellable Core Technology (SCT) and the Asymmetric-Membrane Technology (AMT) of Bend Research (Bend, OR). As seen in Figures 21A and B, which are not according to the invention and are present for illustration purposes only, SCT tablets are bi-layer tablets coated with an insoluble, dense, semipermeable coating **69** and have a laser-drilled hole **70.** The two layers are a sweller layer **71** and a drug-containing layer **72.** The sweller layer **71** contains hydrophilic swelling polymer(s) and other tablet excipients. Following ingestion, the sweller layer **71** imbibes water and swells to generate hydrostatic pressure that extrudes, or pumps, the solution/suspension contents of the drug layer **72** through the hole **70** in the coating on the drug-layer side. The release rate is primarily controlled by the rate of water permeation through the coating. However, the osmotic, swelling, and viscosity properties of the tablet-core sweller and active layers also contribute to the release rate and are important in ensuring that the entire active layer is delivered from the tablet. SCT tablets are manufactured using conventional bi-layer tableting, film-coating, and laser-drilling. The tablet sweller layer **70** is typically a direct-compression formulation. The active layer, depending on the API properties and dose, may be formulated by direct compression, wet granulation, or dry granulation. The membrane is formed by solvent film-coating in a conventional pan coater and the delivery orifice is created on the drug-layer side of the tablet with a laser drill, using either a batch array or continuous tablet feeding.

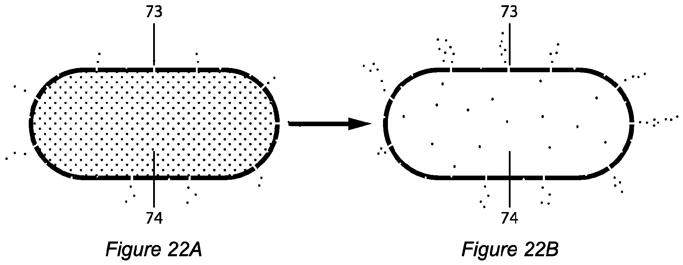

As seen in Figures 22A and B, which are not according to the invention and are present for illustration purposes only, AMT tablets are single-layer tablets film-coated with a porous, semipermeable membrane. Soluble tablet-core ingredients, including the drug, generate an osmotic pressure gradient across the coating. As water volume increases within the tablet, hydrostatic pressure develops and forces drug solution out through the microporous coating **73.** The release rate is controlled by the water permeability of the coating and the osmotic pressure of tablet core **74.** Coating porosity is achieved using a phase-separation process dictated primarily by the polymers and co-solvent system. High-porosity AMT coatings can permit higher water fluxes, shorter lag times and faster release than SCT systems. Importantly, the interconnected pores serve as the delivery medium so AMT tablets do not require laser-drilled orifices. AMT tablets are manufactured using conventional tableting and film-coating technologies. The tablet core **74** is compressed, depending on API properties, using direct-compression, wet-granulation, or dry-granulation techniques. The semipermeable membrane polymers are dissolved in solvent and film-coated using conventional pan coaters.

Other exemplary embodiments of osmotic delivery devices, including those for the delivery of medications for the treatment of Parkinson's disease, are described in U.S. Patent Nos. 4,142,526, 5,192,550, 5,266,332, 5,776,493, 5,021,053, 6,217,905, and 6,773,721.

A significant disadvantage of existing non-electric osmotic pumps is that once the devices have been wetted and drug delivery has been started, it is not possible to temporarily stop the drug delivery from the device if the patient wishes to temporarily remove the drug delivery device from the mouth. Another significant disadvantage is that the flow rate of these devices is often very sensitive to temperature.

*Controlled release drug delivery patches.* Modified versions of zero order transdermal drug delivery technologies can be used for drug administration in the mouth. In transdermal drug delivery, the drug delivery devices often include either a reservoir-type or a matrix-type device. In a reservoir-type device, the device includes an impermeable backing film on the outer side, followed by a reservoir containing the drug, then a semipermeable, rate-controlling membrane, followed by an adhesive layer for attachment to the skin, and a final protective, removable inner film. Alternatively, for a matrix-type device, the drug can be dispersed in a polymeric matrix, laminated to the backing film and coated with an adhesive layer, followed by a protective, removable inner film.

For drug administration into the mouth, the order of the layers may be changed such that the adhesive layer and the impermeable backing film are proximate the mucous membrane, and drug is released toward the space of the oral cavity rather than toward the surface of the mouth.

A significant disadvantage of existing controlled-release drug delivery patches designed for use in the mouth is that once the patches have been wetted and drug delivery has been started, it is not possible to temporarily stop the drug delivery from the patch if the patient wishes to temporarily remove the drug delivery patch from the mouth. Another significant disadvantage is that the flow rate of these devices is often very sensitive to temperature, food and drink.

### Ambient-Pressure and Suction Independent Pump Designs

The invention includes intra-oral drug delivery devices whose rates of drug delivery are substantially independent of increases or decreases in ambient pressure in the mouth and/or in the atmosphere, e.g., devices that do not deliver clinically significant, undesired boluses as the ambient pressure changes. A source of inaccuracy in many device designs, including many pumps pressurized by a spring, a propellant or by compressed gas is that the rate of drug delivery can vary as (a) the ambient air pressure changes, e.g., at sea level (101,353 kPa (14.7 psia)) versus at 2133,6 m (7,000 feet) elevation or in an airplane (both about 77,911 kPa (11.3 psia)), and (b) the patient sucks on the drug delivery device. The invention includes pressure-invariant pumps whose drug delivery rate is substantially insensitive to changes in atmospheric pressure. The invention also includes suction-induced flow limiters that substantially reduce or eliminate the delivery of a drug bolus when a patient sucks on the drug delivery device.

While at first glance it might seem preferable to hermetically seal the spring or propellant compartment so that the components are not exposed to saliva, food, liquids, and potentially deleterious conditions (e.g., acids, bases, alcohols, oils, and solvents in the mouth), preferred drug delivery devices of the invention comprise spring or propellant-pressurized surfaces in the spring or propellant compartments that are in fluidic (gas and/or liquid) contact with the ambient atmosphere via one or more ports or openings in the housing of the drug delivery device. Highly preferred designs for ambient pressure independent spring-driven and propellant-driven pumps are those in which both the drug outlet and the spring or propellant-pressurized surface (e.g., a pressure plate or plunger) are exposed to the ambient pressure, i.e., the pressurized surface is not enclosed within an hermetically sealed chamber. With such a design, any changes in the ambient pressure will be equal on both the drug outlet and on the pressurized surface, resulting in no change to the rate of drug delivery.

In another embodiment, the system can be designed to keep the change in the rate of drug delivery within a desired limit by using a sufficiently high pressure inside the device. For example, for the flow rate to vary by less than 10% across the range of 101,353 (14.7) to 77,911 kPa (11.3 psia) (sea level to 2133,6 m (7,000 feet)) the system can be calibrated such that it delivers drug at its target rate at the pressure midpoint, i.e., 89,632 kPa (13.0 psia). Then, for a 11,721 kPa (1.7 psia) ambient pressure change to cause less than a 10% change in the rate of drug delivery it is necessary for the drug delivery device to have an internal pressure of greater than about 101,353 (14.7) / .1 = 1013,53 kPa (147 psia), or about 1013,25 kPa (10 atm). In such a manner it is possible to achieve any desired accuracy across a specified ambient pressure change. For example, it is possible to achieve an accuracy of equal to or less than 20%, 15%, 10%, 5%, or 3% across the ambient pressure range of 101,353 (14.7) to 77,911 kPa (11.3 psia). Preferred spring-driven, gas-driven, or propellant-driven drug delivery devices of the invention maintain an internal pressure of greater than or equal to about 405,3, 607,95, 810,6, 1013,25 or 1519,875 kPa (4, 6, 8, 10, or 15 atm).

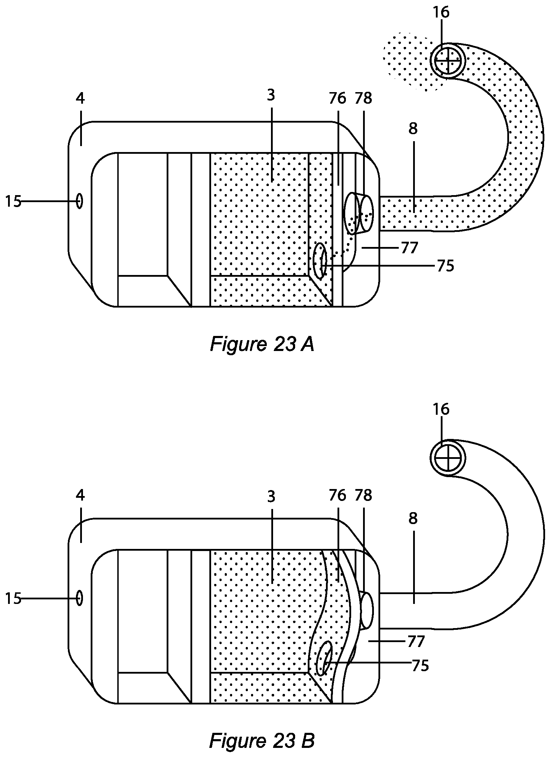

A low pressure condition can be created within the mouth if the patient sucks air out of the mouth or sucks directly on the drug delivery device. Humans are able to draw a negative pressure of up to about 13,790 kPa (2 psi) in the mouth. The low pressure can cause a drug bolus to be delivered from the drug reservoir into the mouth. In embodiments where a liquid or solid form of the drug is delivered, it is necessary to provide a means whereby the suction created within the mouth does not cause the drug to be evacuated from the drug reservoir prematurely. One example of a means to address this issue is to employ a fluidic channel that is designed such that when the drug is being infused via a pressure head, the fluidic channel inflates and when low pressure is created by the mouth, the fluidic channel collapses, causing it to kink and temporarily halting the infusion of the drug. In another embodiment, low ambient pressure in the mouth causes a diaphragm to deflect and block the drug flow channel, examples of which can be seen in Figures 23A and 23B. Figure 23A shows drug delivery during normal operation. Drug from the drug reservoir **3** is pushed through the orifice **75** in the diaphragm **76** and into a chamber **77** prior to entering the nozzle tube **78** and then out the nozzle with one-way valve **16.** In Figure 23B, an external vacuum is applied to the environment that the device occupies. This causes the diaphragm **76** to be displaced, blocking the orifice **75** from flow and halting flow through the nozzle **78.** Another example of a means of addressing the issue of bolus delivery of the drug due to low pressure in the mouth is the use of an inline vacuum-relief valve, such as a float valve that closes the fluidic channel when a vacuum is created and releases the fluidic channel once the vacuum is released.

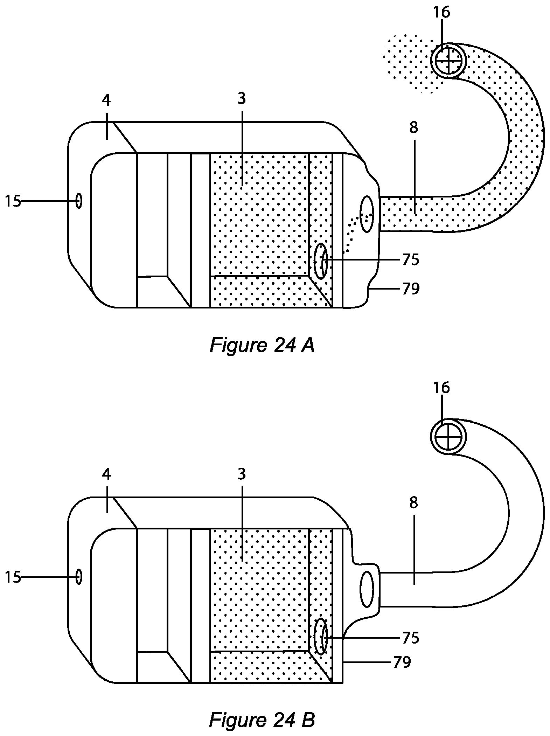

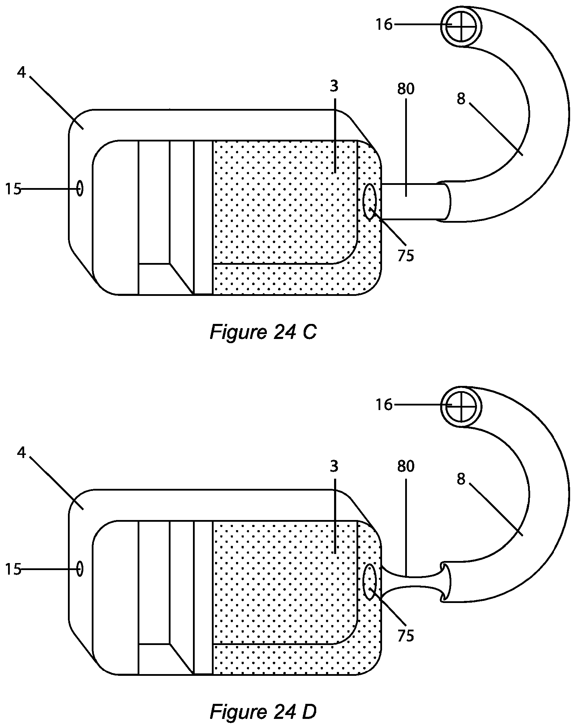

In another embodiment, the drug delivery device comprises a compliant accumulator reservoir downstream of the drug reservoir. This accumulator comprises a compliant material that collapses and plugs the outlet port from the drug reservoir when the ambient pressure decreases below a specified level. Figure 24A and B illustrates the mechanism of operation of the accumulator. Figure 24A shows the concept during normal operation. Drug from the drug reservoir **3** is pushed through an orifice **75** and into the accumulator **79** prior to entering the nozzle tube **8** and then exiting the nozzle via one-way valve **16.** In Figure 24B, an external vacuum is applied to the environment that the device occupies. This causes the accumulator **79** to collapse, blocking the orifice **75** from flow and halting flow through the nozzle **8.** Another embodiment is a compliant member that collapses with external vacuum pressure. A compliant tubing **80** is placed in line and is in fluid communication with the drug reservoir **3** and the ambient environment. Figure 24C shows the device in normal operation. Figure 24D shows the collapsed compliant tubing **80** when an external vacuum pressure is applied to the system, collapsing the compliant tubing **80** and blocking flow from exiting the one-way valve **16.**

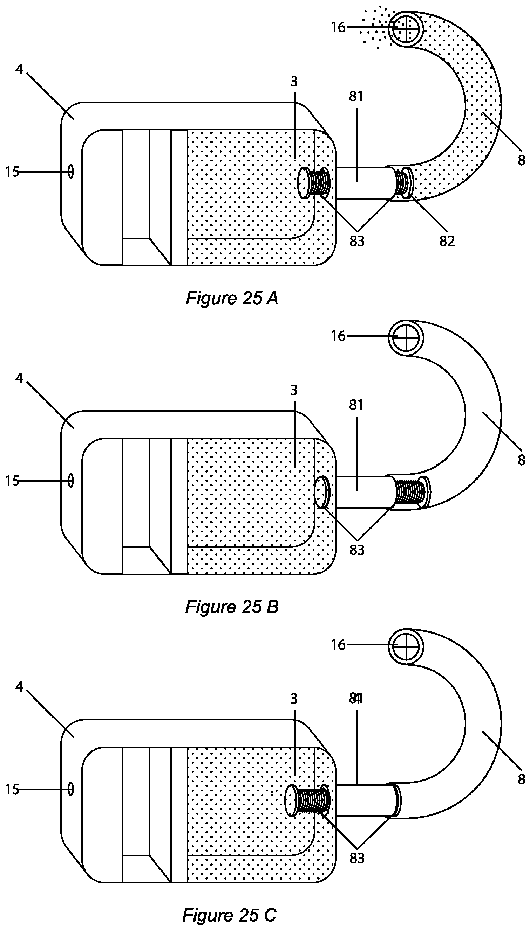

Figures 25A, B and C illustrate an additional mechanism that prevents bolus delivery in the mouth when a patient sucks on the drug delivery device, and changes in drug delivery rate when the ambient pressure changes. Figure 25A shows the concept during normal operation. Drug from the drug reservoir 3 is pushed through an orifice tube **81** prior to entering the nozzle tube **8** and then exiting the nozzle with one-way valve **16.** In Figure 25B, an external vacuum is applied to the environment that the device occupies. This causes the float valve **82** to compress the spring **83** and move in the direction of blocking flow from entering the orifice tube **81** and halting flow through the one-way valve **16.** In Figure 25C, an external positive pressure is applied to the environment that the device occupies. This causes the float valve **82** to compress the spring **83** and move in the direction of blocking flow from exiting the orifice tube **81.**

In preferred embodiments of these designs for substantially ambient-pressure independent drug delivery devices, the drug delivery device is configured to deliver a bolus of less than about 5, 3 or 1% of the contents of a fresh drug reservoir, when the device is sucked on by a patient for a period of about one minute, as compared to an identical drug delivery device at atmospheric pressure; or when the ambient pressure drops by 13,790 kPa (2 psi) for a period of one minute.

### Ambient-Temperature Independent Pump Designs and Methods

While the flow rate of electric pumps is typically substantially independent of the ambient temperature the same is not true of passive pumps, such as elastomeric, spring-driven, gas-driven, propellant-driven, or osmotic pumps. The invention includes designs and methods of achieving accurate drug delivery as the ambient temperature surrounding the drug delivery device increases or decreases, i.e., devices that do not deliver clinically significant, undesired boluses as the ambient temperature changes. Osmotic pumps, drug delivery patches and other diffusion-based drug delivery systems are particularly sensitive to changes in the ambient temperature, and transient temperature excursions may permanently change the drug transport characteristics of the diffusion-controlling membranes or pores in these devices. In a preferred embodiment the drug delivery devices of the invention do not substantially change their average long-term rate of drug delivery after exposure to a transient temperature excursion. In preferred embodiments, the invention includes one or more temperature-induced flow limiters which substantially reduce or eliminate the delivery of a drug bolus when a patient consumes a hot drink.

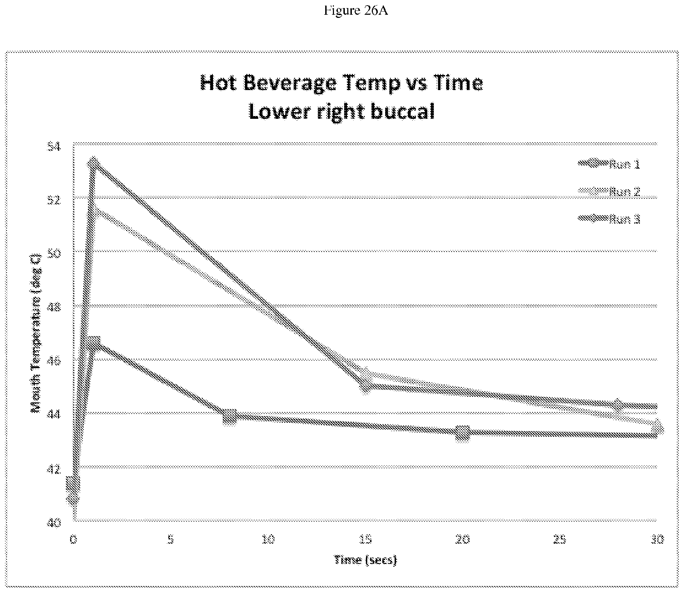

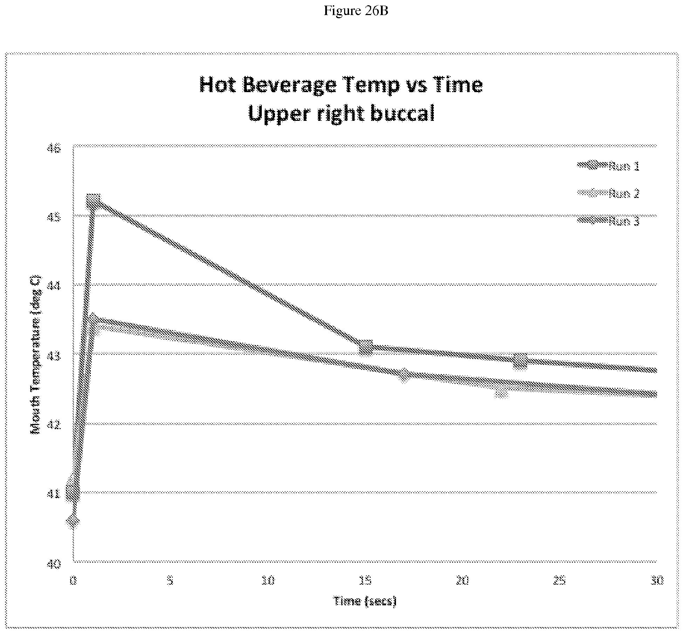

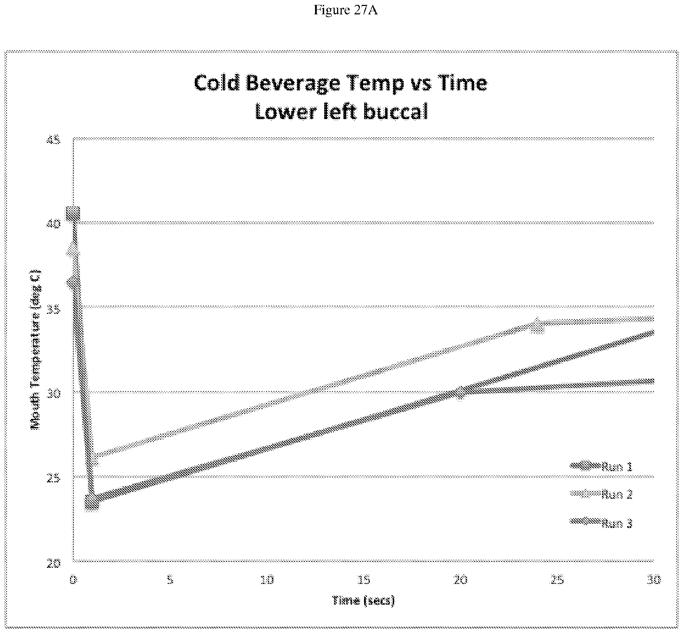

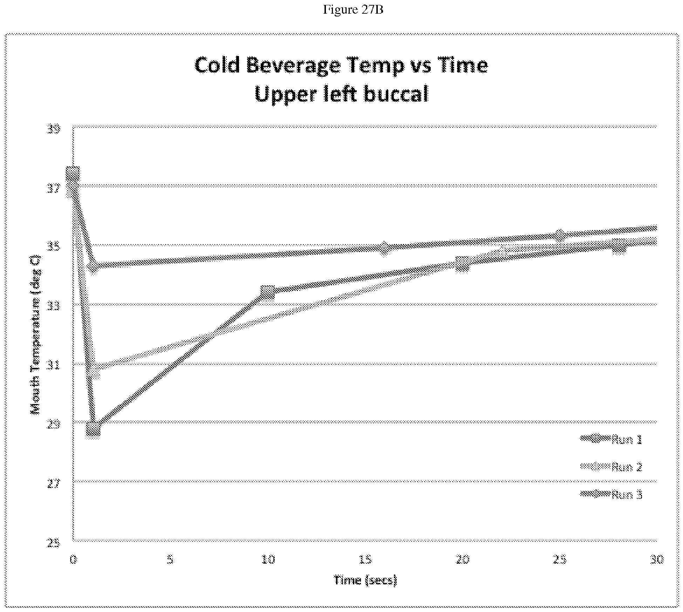

Figure 26A shows the temperature-time profile in the lower buccal vestibule when a hot drink is sipped. Figure 26B shows the temperature-time profile in the upper buccal vestibule when a hot drink is sipped. Figure 27A shows the temperature-time profile in the lower buccal vestibule when a cold drink is sipped. Figure 27B shows the temperature-time profile in the upper buccal vestibule when a cold drink is sipped. All experiments were performed in a single male patient. A thermocouple was placed in the vestibular space to obtain baseline oral temperature. A beverage was held in the mouth and swished over the location of the thermocouple for approximately three seconds. The data demonstrate that transient temperature excursions routinely occur in the mouth when a hot or cold beverage is consumed, with excursions possible of over about 53°C and below about 24°C. The data also demonstrate that temperature excursions tend to be significantly reduced in the upper buccal vestibule than in the lower buccal vestibule, with a maximum temperature recorded of about 45°C vs. 53°C and a minimum temperature recorded of 29°C vs. 24°C. Consequently, in a preferred embodiment the drug delivery devices of the invention are located in the upper buccal vestibule rather than in the lower buccal vestibule.

Generally, it is a greater concern when the intra-oral temperature increases rather than decreases, because many non-electric pumps will provide an undesired drug bolus that may be clinically signficant. When the temperature decreases, many non-electric pumps will provide a transient reduction in drug delivery that is generally not clinically significant.

In a preferred embodiment, the solid or fluid drug delivery device is configured to deliver a bolus of less than 5% of the contents of a fresh drug reservoir, when immersed for five minutes or for one minute in a stirred physiological saline solution at about 55 °C, as compared to an identical drug delivery device immersed for the same duration in a physiological saline solution of pH 7 at 37 °C. In another preferred embodiment, the solid or fluid drug delivery device is configured to change its average rate of drug delivery over a period of one hour in a physiological saline solution of pH 7 at 37 °C by less than about 5% after immersion for five minutes or for one minute in a stirred physiological saline solution at about 55 °C, as compared to its average rate of drug delivery immediately prior to exposure to the temperature excursion.

For elastomeric pumps, to minimize the change in flow rate when the patient drinks a hot beverage, it is preferred to utilize elastomeric materials whose force is relatively independent of temperature in the range of 37°C - 55°C. For example, the force in a fresh reservoir may increase by less than 30%, 20% or 10% when the temperature is raised from 37°C to 55°C. Examples of elastomeric materials whose mechanical properties change very little within these temperature ranges are natural rubbers, such as highly cross-linked polyisoprene and synthetic elastomers such as neoprene.

For spring-driven pumps, to minimize the change in drug administration rate when the patient drinks a hot beverage, it is preferred to utilize spring materials whose force is relatively independent of temperature in the range of 37°C - 55°C. For example, the force in a fresh reservoir may increase by less than 30%, 20% or 10% when the temperature is raised from 37°C to 55°C. Examples of materials with low sensitivity to temperature changes in this range, that are safe for use in the oral anatomy are 300 series stainless steels, such as 301, titanium, Inconel and fully austenitic Nitinol (above its austenite finish temperature).

For gas-driven pumps, to minimize the change in flow rate when the patient drinks a hot beverage, it is preferred to minimize the volume of the gas relative to the volume of the drug-including fluid. The volume of the gas can be less than 40%, 30%, 20% or 10% of the volume of the drug-including fluid in a fresh reservoir. For example, the force in a fresh reservoir may increase by less than 30%, 20%, or 10% when the temperature is raised from 37°C to 55°C.

For propellant-driven pumps, it is preferred to use propellants whose pressure increases by less than about 80%, 60%, or 40% when the temperature is raised from 37°C to 55°C. As examples, the pressure of Dupont Dymel HFC-134a (1,1,1,2-tetrafluoroethane) increases from 938 kPa (absolute) at 37°C to 1,493 kPa (absolute) at 55°C, an increase of 59%. The pressure of Dupont Dymel HFC-227ea/P (1,1,1,2-tetrafluoroethane) increases from about 700 kPa (absolute) at 37°C to 1,000 kPa (absolute) at 55°C, an increase of 42%. In order to minimize the effect of temperature fluctuations on the propellants, a number of methods can be employed. In one embodiment, an insulating material can be used to decrease the sensitivity to changes in ambient temperature by insulating the propellant and drug reservoirs with materials of low thermal conductivity. Materials such as closed cell neoprene foams, can be used in this embodiment. In another embodiment, a material with very low thermal conductivity can be utilized, such as a ceramic.

### Pump Automatic Stop/Start Safety Feature

When the pump is removed from the mouth, it is preferred that the drug delivery be temporarily stopped. This is desirable so that drug is not wasted and, more importantly, so that dispensed drug does not accumulate on the surface of the device. Such an unquantified accumulation of drug on the surface of the device might lead to the undesired delivery of a bolus of an unknown quantity of drug to the patient when the device is reinserted in the mouth. In preferred embodiments, the drug delivery device comprises one or more automatic stop/start elements.

In one embodiment, the drug delivery device has an on/off switch or other mechanism for use by the patient. In a preferred embodiment, the drug delivery device automatically stops delivering drug when (1) the drug delivery device, the pump, and/or the oral liquid impermeable reservoir are removed from the mouth; (2) the drug delivery device, the pump, and/or the oral liquid impermeable reservoir are disconnected from their attachment to the interior surface of the mouth, either directly (e.g., when secured to the teeth), or indirectly (e.g., when secured to a fastener which is secured to the teeth); or (3) the oral liquid impermeable reservoir is disconnected from the pump or from the reusable component (e.g., the fastener). In another preferred embodiment, the drug delivery device automatically starts delivering drug when (1) the drug delivery device, the pump, and/or the oral liquid impermeable reservoir are inserted into the mouth; (2) the drug delivery device, the pump, and/or the oral liquid impermeable reservoir are connected to their attachment to the interior surface of the mouth, either directly (e.g., when secured to the teeth), or indirectly (e.g., when secured to a fastener which is secured to the teeth); or (3) the oral liquid impermeable reservoir is connected to the pump or to the reusable component (e.g., the fastener).

In another embodiment, the flow of drug begins when a cap is removed from the orifice from which the drug is delivered into the mouth and halts when the cap is placed back onto the orifice. In a different embodiment, a clip can be placed over the fluidic channel carrying the drug, causing a kink or blockage, thereby halting the flow of drug to the patient. The flow of drug is restored to the patient once the clip is removed. In yet another embodiment, the flow of drug is halted due to the release of a pressure sensitive switch that breaks the circuit of power to the pump, halting the flow of drug when the device is removed from the mouth. The act of replacing the device back onto the dentition closes the pressure sensitive switch, restoring power to the pump and the flow of drug to the patient. In a further embodiment, the fluidic channel kinks, halting the flow of drug, when the device is removed from the patient due to a change in the radius of curvature of the fluidic channel.

In another embodiment, illustrated in Figures 12E and 12F, a protrusion **84** in the drug delivery device is attached to a spring loaded clutch mechanism **85** employed in the device that engages the piston **39** to inhibit the force transmission to the drug reservoir **3** prior to use. This protrusion **84** is depressed when the drug delivery device is placed onto the tooth or teeth, releasing the piston **39** and allowing the piston **39** to transmit force to the drug reservoir **3.** When the device is removed from the mouth, the protrusion **84** is disengaged, which again engages the clutch mechanism **85**, stopping the piston **39** from applying force to the drug reservoir **3.**

In another embodiment, a sensor detects when the device is placed in the mouth. For example an optical sensor can send a signal to turn the device off, halting flow from the pump. In another example, a moisture sensor can send a signal to turn the device on, initiating flow from the pump.

### CONCENTRATED DRUG FORMULATIONS

Formulations of drugs to be delivered via the drug delivery devices of the invention (such as LD, LD prodrugs, DDC inhibitors, and other drugs) may include non-toxic aqueous or non-aqueous carrier liquids, such as water, ethanol, glycerol, propylene glycol, polyethylene glycols, ethyl lactate and edible oils such as vegetable oils, lipids, monoglycerides, diglycerides or triglycerides, paraffin oil, and their mixtures. The liquids or their infused mixtures melt or sufficiently soften for pumping typically below about 37°C. The formulations may comprise any fluid taught herein, such as true solutions, colloidal solutions, emulsions or suspensions. Solid drug formulations with or without carrier liquids can also be semi-continuously delivered.

In some embodiments the infused fluid can include drug-containing micelles or liposomes; the fluid can be a water-in-oil emulsion or an oil-in-water emulsion and the drug can be mostly in one of the phases, e.g., in the oil phase of the emulsion, or in the aqueous phase of the emulsion. Exemplary oil phases include edible oils, such as vegetable oils, monoglycerides, diglycerides or triglycerides and paraffin oil.

In different embodiments the fluid infused in the mouth can be aqueous, non-aqueous (e.g., an oil based suspension), or a mixed aqueous-non-aqueous suspension, viscous gel or a colloid. The suspension, gel or colloid can include in these embodiments small solid particles and the particles can include the drug.

The average diameters of the suspended solid particles in the carrier liquids can be typically less than 100 micrometers, for example less than 50 micrometers, less than 10 micrometers, less than 5 micrometers, less than 1 micrometer, less than 500 nanometers, less than 100 nanometers, less than 50 nanometers, less than 10 nanometers or less than 5 nanometers.

Nanosuspensions, also known as colloids, are suspensions in which particles are in the submicron size range. Particles in this size range tend not to settle out since their Brownian motion is sufficient to overcome gravitational acceleration. Because of the limitations of dry powder particle size reduction techniques, nanosuspensions are typically generated either by controlled precipitation or by wet milling.

Despite the attractiveness of their physical stability, nanosupensions do have some significant limitations related to the high specific surface area and the tight curvature of the particles. On the molecular level, curvature is energetically unfavorable since it decreases the ratio of crystal lattice to interfacial associations, resulting in migration of molecules from smaller to larger particles (Ostwald ripening). Increased specific surface area is potentially detrimental due to increased potential for chemical reactivity as well as the tying up of solvent molecules tightly bound to the surface, making it difficult to reach high drug concentrations, i.e., to reduce the volume of the formulated drug enough for the system to comfortably fit in the mouth without impeding speech or swallowing.

Suspensions in non-aqueous liquid vehicles can have stability advantages over aqueous suspensions. Some non-aqueous vehicles have the benefit of the drugs having very low solubility in them. Very low solubility may slow chemical degradation and will also slow Ostwald ripening, a phenomenon in which particles grow overtime due to dissolution from highly curved (and therefore highly energetic) particle surfaces to surfaces with lower curvature.

Temperature sensitive, typically non-aqueous, liquid vehicles can be particularly advantageous. A temperature-sensitive emulsion or suspension can be solid or semi-solid at its storage temperature but is fluid at body temperature. Advantages of temperature sensitive emulsions or suspensions include improved physical stability during storage, since the settling, i.e. the sedimentation rate, of suspended solid particles dispersed in a solid vehicle can be slow or negligible. Temperature-sensitive emulsions and suspensions also offer better chemical stability when their chemical degradation reaction rate is bimolecular and diffusion dependent, the diffusion being much slower in the solid than in the liquid state. For example, reactions of drugs with dissolved oxygen are typically diffusion dependent and their rates are much slower, or may even approach nil, in solids. Examples of such reactions include oxidations of LD, CD, benserazide and COMT inhibitors like tolcapone and entacapone. Their oxidation by dissolved O₂ can result not only in loss of active drug, but also in the formation of toxic products, as is the case of CD, where hydrazine is produced upon air-oxidation. Accumulation of hydrazine can limit the shelf life of liquid CD containing products like the DuoDopa gel to less than 4 months. As disclosed here the shelf life can be extended to greater than 6 months, e.g., more than a year or even more than 2 years through use of a temperature sensitive carrier, exemplified by cocoa butter.

In preferred embodiments, the intra-orally administered formulation comprises a suspension at body temperature, the suspension comprising solid drug particles of a concentration greater than or equal to 2 M, such as greater than 3 M, for example greater than 4 M, such as 4.5 M or greater. The suspensions can remain free of sedimented solid drug for about 1 month or more or for about 1 year or more at about 25°C. The drug may be LD and/or CD, and may optionally further comprise a COMT inhibitor. The suspensions may have a shear (kinematic) viscosity greater than 10 Pa·s (100 Poise). The weight fraction of solid drug particles having maximal diameters that are smaller than 5 micrometers and that are larger than 0.5 micrometers can be greater than ½. The maximal solid drug particle diameters may be bimodally or multimodally distributed.

### LEVODOPA FORMULATIONS

LD is poorly soluble in most non-toxic solvents, including water and alcohols. For example, we have found that in a citrate buffered solution of about pH 4.5 the solubility of LD at 25°C is only about 0.68 g/100 mL, or 34 mM. LD is even less soluble in alcohols. To deliver a typical daily dose of 1,000 mg approximately 150 mL of saturated LD aqueous solution would be required, which is incompatible with the volume requirements for a drug delivery device placed in the mouth.

DDC inhibitors such as carbidopa are typically co-administered with LD, and it would be desirable to co-infuse LD and CD. CD is also poorly soluble in non-toxic solvents such as water, further increasing the required volume of infused solution.

The invention features combinations of (1) pharmaceutically acceptable, viscous fluids including highly concentrated LD, and (2) miniature but powerful pumps that are placed in the mouth and which can administer viscous fluids including LD into the mouth. Preferred formulations include LD and one or more additional drugs for the treatment of Parkinson's disease, such as a DDC inhibitor, a COMT inhibitor, a drug to treat gastroparesis, a MAO-B inhibitor, adenosine A2 receptor antagonists, or a dopamine agonist.

Described herein are formulations of optionally viscous fluids in which precipitation of LD and/or CD may be retarded and in which greater than or equal to 1,000 mg of LD and/or CD is contained in a volume of less than 10, 7.5, 5 or preferably 3 mL (LD concentrations of 0.5, 0.67, 1.0, and 1.67M, respectively). The viscosities of the formulations delivered into the mouth at 37°C are typically in the range of 0,0012-200 Pa·s (1.2-200,000 cP), e.g., 0,005-0,05 (5-50), 0,05-0,1 (50-100), 0,1-1 (100-1,000), 1-10 (1,000-10,000), 10-50 (10,000-50,000), 50-100 (50,000-100,000), or greater than 100 Pa·s (100,000 cP). An exemplary precipitation retarding thickener that increases the viscosity is carboxymethylcellulose, e.g., as its sodium salt. Concentrated sugar solutions may be used to increase the viscosity of the fluid. For example, the drug may be added to a sugar or sugar mixture (e.g., sucrose, dextrose, glucose) solution that is 40% - 70% sugar by weight, e.g., 40 - 50% sugar by weight, 50 - 60% sugar by weight, or 60 - 70% sugar by weight. As previously discussed, the LD and CD formulations may comprise multimodal particle size distributions.

*Solutions.* While generally insoluble in water, the solubility of LD in aqueous solutions increases substantially at a pH below about 2 and above about 9, such as pH 1 or pH 9.5, allowing dissolution of a daily dose of 1,000 mg of LD in 10 mL or less of the acidic or basic aqueous solution. These solutions may be converted to colloidal LD solutions of a pH typically between about 2.5 and 8.5 when the viscosity is raised by adding a sugar or sorbitol or glycerol or preferably a thickener like carboxymethyl cellulose, or adding crystal growth and/or precipitation retarder, for example polyvinyl pyrrolidone or polyethylene oxide, and/or a surfactant. Edible surfactants that can be used to stabilize emulsions or form suspensions for infusion into the mouth include monoglycerides, lecithins, glycolipids, fatty alcohols and fatty acids. Among these, the non-ionic surfactants are particularly useful when the infused suspensions are acidic. They include, for example, surfactants with glycerol, sugar polyethylene glycol based polar head-groups, and usually have long aliphatic carbon chains, including 10-24 carbon atoms, for example 12-18 carbon atoms.

To mask the potentially unpleasant taste of the solution, sweeteners, flavors or taste masking agents may be added (e.g., sucrose, sorbitol, citric acid). When the colloidal LD solution is of a pH between 2.5 and 5.0, to reduce the possibility of degradation of the teeth the fluid could be infused into the mouth at least 0.25, 0.5, or 0.75 cm distant from the teeth.

Basic amino acid salts of LD and CD are soluble in aqueous and non-aqueous solutions, as described in U.S. Pat. No. 7,863,336. Concentrated LD and/or CD solutions or gels of such basic amino acid salts (e.g., arginine salts) may be infused into the mouth using the devices and methods of this invention. Such solutions may have a pH of 8-10. The composition may have a molar ratio of about 1:1.5 to about 1:2.5 of LD:arginine. Such solutions may be aqueous or non-aqueous. The solutions are stable for both shelf life and operational use. To reduce the potentially unpleasant taste of the basic solution, flavors or taste masking agents may be added (e.g., sucrose).

*Suspensions.* Concentrated fluids including LD and CD may be continuously or semi-continuously administered into the mouth as suspensions. Suspensions of solid drugs, such as solid LD and CD, are optionally made of particles, such that both their average and mean diameters are less than about 50 µm, 20 µm, 10 µm, 5 µm or 1 µm. They may substantially sediment only in a day or longer, for example in more than 3 days, 1 week, 2 weeks, a month, 3 months, 6 months or a year. In general, the sedimentation rate decreases when the size of the particles is smaller, making particles smaller than 10 µm preferred, smaller than 5 µm more preferred and particles of 1-3 µm most preferred. The sedimentation rate also decreases when the density of the particle suspending liquid vehicle, which is typically lower than that of a solid drug like LD or CD, is increased by dissolving in the liquid a higher density additive, exemplified by a sugar when the liquid is water, the density of the liquid vehicle increasing with the concentration of the additive. Aqueous solutions of edible sugars of densities greater than 1.2 g/mL, such as greater than 1.3 g/mL are useful for reducing the sedimentation rate. An exemplary high density aqueous solution is 65 weight % sugar, with a density of about 1.32 g/mL at about 25°C. Because sedimentation is also decreased when the viscosity is higher, the suspensions can be formulated with agents increasing their viscosity.

Solid drug particle containing aqueous suspensions can be stabilized with simple syrup (e.g., with typical sucrose to water weight ratio from about 1:1 to about 2:1); glycerol; or sorbitol. Alternatively, the suspensions can be stabilized with polymers that can be cellulose derived, e.g., microcrystalline cellulose, methylcellulose, carboxymethylcellulose (CMC), e.g., as its sodium salt, or hydroxypropylmethylcellulose (HPMC) also known as hypromellose; or they can be stabilized with mucilage or tragacanth or xanthan gum. They may contain preservatives and antimicrobial agents such as methylparaben, propylparaben, potassium sorbate, methyl hydroxybenzoate, or propyl hydroxybenzoate; and/or sweeteners like saccharine sodium, flavorings like citric acid, sodium citrate, and antifoaming or defoaming agents like polydimethylsiloxanes and their combinatons. They may also include poly-N-vinylpyrrolidone, polyethylene glycol, surfactants typically with non-ionic polar headgroups, including for example alcohol and ether functions covalently bound to a 12-20 carbon atom chain.

Sedimentation-slowing, viscosity-increasing, and/or other additives retarding precipitation are described, for example, by Volker Bühler, Pharmaceutical Technology of BASF Expedients, Third Edition, particularly in Chapter 5, "Suspensions" and Chapter 6, "Semisolid Dosage Forms" June 2008. The suspension forming liquid, i.e., the vehicle or carrier, can be aqueous or non-aqueous, for example an edible oil, a temperature sensitive butter like cocoa butter, or it can be medicinal paraffin oil, propylene glycol or glycerol or ethanol. As disclosed above the suspensions can be colloidal such that the solid drug particles are too small to scatter visible light sufficiently for opacity; they can be, for example, translucent gels. More typically they can, however, be opaque, the drug particles approaching or exceeding in their dimensions the wavelengths of visible light.

Suspensions suitable for delivery of LD and CD are also described, for example, in the book "Pharmaceutical Emulsions and Suspensions: Second Edition, Revised and Expanded (Drugs and the Pharmaceutical Sciences) Edited by Francoise Nielloud and Gilberte Marti-Mestres, which is Volume 105 of the series Drugs and the Pharmaceutical Sciences, James Swarbrick, Executive Editor, published by Marcel Dekker.

When flow is controlled by a flow-limiting tube or orifice, the peak diameter of the largest particles of the unimodal, bimodal or multimodal particle size distributions is typically smaller than 1/5^{th} of the inner diameter of the tube or orifice, such as less than 1/10^{th} of its diameter, in order to avoid blockage. Consequently, the peaks for largest particles of the distribution can be of 100 µm or less, for example 30 µm or less or 10 µm or less, or 3 µm or less. In a bimodal distribution the peaks for the smaller particles might be correspondingly about 20 µm or less, 6 µm or less, 2 µm or less or 0.6 µm or less, respectively.

Solid drug particle containing aqueous suspensions can be stabilized with simple syrup (e.g., with typical sucrose to water weight ratio from about 1:1 to about 2:1); glycerol; or sorbitol. Alternatively, the suspensions can be stabilized with polymers that can be cellulose derived, e.g., microcrystalline cellulose, methylcellulose, carboxymethylcellulose (CMC), e.g., as its sodium salt, or hydroxypropylmethylcellulose (HPMC) also known as hypromellose; or they can be stabilized with mucilage or tragacanth or xanthan gum. They may contain preservatives and antimicrobial agents such as methylparaben, propylparaben, potassium sorbate, methyl hydroxybenzoate, or propyl hydroxybenzoate; and/or sweeteners like saccharine sodium, flavorings like citric acid, sodium citrate, and antifoaming or defoaming agents like polydimethylsiloxanes and their combinations. They may also include poly-N-vinylpyrrolidone, polyethylene glycol, surfactants typically with non-ionic polar headgroups, including for example alcohol and ether functions, covalently bound to a 12-20 atom long carbon atom chain.

In general, for suspensions continuously delivered in the mouth, a high volume fraction of solids can be advantageous both because the volume is reduced and because settling, i.e., sedimentation, leading to an undesired solid drug concentration difference, is slowed. The inventors have discovered that orally deliverable oil-based suspensions, such as vegetable oil based suspensions, can contain more than 600 mg LD per mL, such as more than 700 mg per mL, for example 800 mg LD per mL or more, yet the suspensions can be pumped. Their apparent viscosity can be lower than that of water-based suspensions with similarly high LD concentrations. For example a suspension of about 800 mg/mL levodopa in edible oil can be poured, and it can be honey-like in its apparent viscosity at about 25°C. Because LD is more soluble in water than in oils, oil-based LD suspensions have the additional advantage of their solid or dissolved LD being less saliva-extracted than LD in suspensions made with water or aqueous solution. For example, when an oil based suspension flows into the mouth through an orifice the risk of leaching by saliva of yet undelivered LD is reduced. The oil-wetted drug is shielded against extraction by saliva, reducing the risk of excess dosing or accidental overdosing.

Optionally the suspensions can also comprise solid carbidopa. When containing solid carbidopa, the sum of the weights of levodopa and carbidopa per mL can be greater than 600 mg per mL, such as more than 650 mg per mL, for example more than 800 mg per mL. The weight fraction of the solid drug or drugs in the suspension can be greater than 0.6. When made with an edible oil, or paraffin oil, or a butter like cocoa butter that is solid at 25°C but is liquid at 37°C, concentrated solid drug suspensions, e.g., of LD, or of LD and carbidopa, can have low apparent viscosities. Because of the typically greater than 3 M suspended solid drug concentration, such as greater than 4 M suspended solid drug concentration, the volume of the drug suspension in the reservoir in the mouth can be small; for example, a daily dose of 1,000 mg of LD can be accommodated in a reservoir of less than 1.25 mL. Because oil can lubricate, i.e., reduce the friction, between flowing solid drug particles suspended in the oil, and also between the particles and the wall of a flow-channel, use of oil-based suspensions can reduce the pressure required for pumping at a particular flow rate. Typical flow rates for the edible oil, paraffin oil, or molten cocoa butter based suspensions can be between about 0.03 mL per hour and about 0.25 mL per hour.

Oil based suspensions can be also physically stable, i.e., sufficiently slowly sedimenting and maintaining the uniformity of their solid drug concentrations for at least 16 hours at 37°C, and can be fluid enough to allow their re-suspension for re-establishing a uniform solid drug concentration after 3 months, 6 months, or longer than 6 month storage at about 25°C. Particularly stable are dispersions of solid drug particles in lipids, including butters like cocoa butter, that are solid at their about 25°C storage temperature, while they are fluid when heated to within the melting range of their mixture of constuents after being placed in the mouth where the temperature is about 37°C.

Adding of lubricants to suspensions, e.g., where the weight fraction of the solid drug is greater than about 0.6 can facilitate the movement of the suspension. The suspensions can be pumped, for example, by slippage or by a combination of flow and slippage. Slippage means that parts of the suspension, or even all of the suspension move, e.g., through a flow-controlling tube or orifice as a unit or as multiple units, each unit a plastically deformable block such as a cylindrical block. The movement, i.e., flow of the block or blocks can be retarded by friction between the moving block and the wall of the flow-controlling tube. The lubricant can reduce the friction and facilitate the flow. To facilitate the flow, a surface active food additive can be added. The surface active food additive lubricant can have a polar or a non-polar head and a long non-polar carbon chain, typically comprising between 8 and 22 carbon atoms. The surface active food additive lubricant can include, for example, a fatty acid monoester of glycerol, such as glyceryl monooleate or glyceryl stearate, or stearyl alcohol or cetyl alcohol.

The non-aqueous, e.g., oil-based, formulations of the invention require that the suspension-contacting components of the drug delivery device utilize materials that are compatible (e.g., dimensionally stable, non-softened, non-leachable, non-extractable) with the non-aqueous formulation. As illustrated in Example 1, this is not today the case for some commonly used pump components. For example, neoprene can be used to replace the non-compatible rubber in a piston or plunger.

*Supersaturated solution.* Concentrated fluids including LD and CD may be administered as supersaturated solutions. Supersaturated solutions are solutions of a drug where the concentration of the drug exceeds its solubility. Solutions of the drug can be supersaturated because the rate of nucleation is retarded or because the growth of nuclei is slowed. Nucleation can be retarded, for example, by exclusion of nucleating solid non-drug particles and drug particles by filtration through filters, for example filters of pore sizes smaller than 0.2 µm, 0.1 µm, 0.05 µm. Growth of nuclei of the drug can be slowed by increasing the viscosity, for example by dissolving a polymer or a compound forming multiple hydrogen bonds like glycerol, a polyol, or a sugar.

*Emulsions.* Concentrated fluids including LD and CD may be administered as emulsions. Pharmaceutical Emulsions and Suspensions, typically including an emulsifying surfactant, are described, for example, in the book "Pharmaceutical Emulsions and Suspensions: Second Edition, Revised and Expanded (Drugs and the Pharmaceutical Sciences) Edited by Francoise Nielloud and Gilberte Marti-Mestres, which is Volume 105 of the series Drugs and the Pharmaceutical Sciences, James Swarbrick, Executive Editor, published by Marcel Dekker. Oil in water and water in oil emulsions are exemplary emulsions. Edible surfactants can be used to form the emulsions for infusion into the mouth, such as monoglycerides, lecithins, glycolipids, fatty alcohols and fatty acids. Among these, the non-ionic surfactants are particularly useful when the infused emulsions are acidic. They include, for example, surfactants with glycerol, sugar polyethylene glycol based polar head-groups, and usually have long aliphatic carbon chains, including 10-24 carbon atoms, for example 12-18 carbon atoms.

*Liposomes.* Concentrated fluids including LD and CD may be delivered as emulsions. Liposome including fluids and formulations are well known in the art. The liposomes may include edible surfactants such as monoglycerides, lecithins, glycolipids, fatty alcohols and fatty acids. Among these, the non-ionic surfactants are particularly useful. They include, for example, surfactants with glycerol, sugar polyethylene glycol based polar head-groups, and usually have long aliphatic carbon chains, including 10-24 carbon atoms, for example 12-18 carbon atoms.

*Solids.* Drugs such as LD, CD and their prodrugs, may be continuously or semi-continuously delivered as solids. The solid may be in the form of small spheres, pills, tablets, pellets, capsules particles, microparticles (e.g., made by extrusion/spheronization), granules, powders, or other similar solid dosage forms known in the art. The solids can be continuously or semi-continuously delivered as coatings of nontoxic polymeric strips or ribbons, such as cellulosic polymer or polylactic acid strips or ribbons. The solid drug formulation may include additional excipients, such as binders, disintegrants, glidants, lubricants, taste modifiers, etc. The solid drug formulation may include a single solid, multiple disceet solids, or a large number of discreet solids (e.g., a powder). For example, to dose LD/CD every 15 minutes over a period of 16 hours, the solid may include 64 individual solid pills, tablets or capsules, with one solid administered at each dosing. The solid may be delivered into the mouth every 1-5, 5-10, 10-15, 15-20, 20-30, 30-60, 60-120, 120-240 minutes. The solids of the invention may include 1 - 1,000 discreet solids, e.g., 1, 2, 3, 4, 2-10, 11-50, 51-100, 101-500, 501-1,000, or 4-1,000 discreet solids. In the case of a powder, the solids of the invention may include greater than 1,000 discreet solids. To minimize the volume of the delivered solids, in preferred formulations the one or more drugs (e.g., LD/CD) includes greater than 50%, 60%, 70%, 80%, 90%, or 95% by weight of the solid, with other excipients making up the balance.

### LEVODOPA PRODRUG FORMULATIONS

*Stable, concentrated LD prodrug solids and fluids.* LD prodrugs are highly soluble in aqueous or non-aqueous solutions enabling their delivery in concentrated aqueous fluids and non-aqueous fluids, of generally lower viscosity than the fluids comprising high solid LD concentrations. Exemplary LD prodrug formulations of the prior art are provided in U.S. Patent No. 5,607,969, and in patent applications WO 2012/079072 and WO 2013/184646.

The preferred prodrugs for administration into the mouth include highly soluble levodopa amides, levodopa esters, levodopa carboxamides, levodopa sulfonamide, levodopa ethyl ester, levodopa methyl ester, and their salts, which can be rapidly hydrolyzed in the body, typically in an enzyme catalyzed reaction, to form LD, yet can be stored at least for the duration of the intended administration period, for example at least 8 hours, 16 hours, 24 hours, 48 hours, 72 hours, in a reservoir of the drug delivery device.

The LD prodrug, or any combination of the prodrug with CD, or CD prodrug, or benserazide, or COMT inhibitor may be dissolved or dispersed in a temperature-sensitive solid or semi-solid carrier, such as cocoa butter, which is solid or semi-solid at about 25°C and is a liquid at about 37°C. The resulting drug solution, suspension or emulsion is stored at ambient temperature, e.g., at about 25°C or below, where it is solid or semi-solid; the solution, suspension or emulsion becomes fluid in the mouth where the temperature is at about 37°C. Dissolved LD prodrugs, CD, CD prodrugs, benserazide, and COMT inhibitors are rapidly oxidized by dissolved air, i.e., by dissolved Oz, but are much less rapidly oxidized in the stored solid or semisolid, where the viscosity is greater and the diffusion of Oz is slower. Oxidation of CD and CD prodrugs by dissolved Oz results not only in loss of active drug, but also in the formation of toxic hydrazine, its accumulation limiting the shelf life of CD or CD prodrug containing products. The shelf life can be extended to greater than 6 months, e.g., more than a year or even more than 2 years through use of a temperature sensitive carrier, exemplified by cocoa butter.

LD prodrugs and/or CD prodrugs may be stored in solid form in an oral liquid impermeable drug reservoir and administered by the drug delivery device into the mouth, where the solid is rapidly dissolved.

### LEVODOPA/CARBIDOPA FORMULATIONS MINIMIZING HYDRAZINE FORMATION

Stored CD is known to degrade to hydrazine. In animal studies, hydrazine shows notable systemic toxicity, particularly by inhalation exposure. These studies report that hydrazine is hepatotoxic, has CNS toxicities (although not described after oral treatment), and is genotoxic as well as carcinogenic. Consequently, it is important to minimize hydrazine formation during storage of CD or LD/CD formulations.

Duodopa, a LD/CD suspension for continuous intraduodenal infusion, produces hydrazine during storage. The average recommended daily dose of Duodopa is 100 ml, containing 2 g levodopa and 0.5 g carbidopa. The maximum recommended daily dose is 200 ml. This includes hydrazine at up to an average exposure of 4 mg/day, with a maximum of 8 mg/day. In order to meet these exposure limits, Duodopa's labeling states that its refrigerated, unopened shelf life is just 15 weeks, and that once removed from the refrigerator and opened the product may only be used for up to 16 hours. The concentrations of LD and CD in Duodopa are 20 mg/mL and 5 mg/mL, respectively.

A stable fluid formulation of CD that does not contain high levels of hydrazine and that can be stored unrefrigerated for extended periods of time is desirable. Hydrazine is produced almost entirely by oxidation of CD in solution; as more of the dissolved CD is degraded over time, more of the suspended CD is dissolved and is itself degraded. In this way significant amounts of hydrazine can be accumulate overtime. Hydrazine is not produced in significant quantities by oxidation of suspended CD particles. Therefore, the amount of hydrazine produced can be dramatically reduced by simultaneously minimizing the amount of aqueous or non-aqueous liquid in which the hydrazine can dissolve, and maximizing the concentration of the suspended solid CD. Such an approach maximizes the ratio of the suspended solid CD to the dissolved CD. The invention includes an oral liquid impermeable reservoir containing a suspension of CD in a fluid volume of 0.20 - 5.0 mL, wherein the concentration of solid CD suspended in the fluid is 50 - 500 mg/mL. The invention features a CD suspension including less than about 4, 1, or 0.25 mg of hydrazine per 500 mg of CD when the suspension has been stored at 5 °C for 1 year, or at 25 °C for 3 months, 6 months, 12 months, or 24 months. The invention features a CD suspension including less than about 1 ppm of hydrazine when the drug reservoir has been stored at 5 °C for 1 year, or at 25 °C for 3 months, 6 months, 12 months, or 24 months. Preferred reservoirs are substantially free of oxygen and are substantially impermeable to oxygen. Preferably, LD is also present in the drug reservoir. Preferred aqueous or non-aqeuous fluids are those in which CD has a very low solubility, such as water. As an example, in an aqueous suspension containing 2,000 mg of LD and 500 mg of CD in a volume of 3 mL, the rate of hydrazine formation is expected to be reduced by a factor of greater than 30 versus its rate of formation in Duodopa. The shelf life of the suspension would therefore be at least 30 times greater than that of Duodopa, as well.

CD or CD prodrug, optionally combined with LD, LD prodrug, or a COMT inhibitor, may be dissolved or dispersed in a temperature-sensitive solid or semi-solid carrier, such as cocoa butter, which is solid or semi-solid at about 25°C and is a liquid at about 37°C. The resulting drug solution, suspension or emulsion is stored at ambient temperature, e.g., at about 25°C or below, where it is solid or semi-solid; the solution, suspension or emulsion becomes fluid in the mouth where the temperature at about 37°C. CD and its prodrugs dissolved in a liquid where O₂ diffuses rapidly can be rapidly oxidized by dissolved air to products including toxic hydrazine, but are much less rapidly oxidized in the stored solid or semisolid, where the viscosity is greater and the diffusion of Oz is slower. Oxidation of CD and CD prodrugs by dissolved Oz results not only in loss of active drug, but also in the formation of toxic hydrazine, its accumulation limiting the shelf life of CD or CD prodrug containing products. The shelf life can be extended to greater than 6 months, e.g., more than a year or even more than 2 years through use of a temperature sensitive carrier, exemplified by cocoa butter.

### PUMP-DRIVEN SUSPENSION SEPARATION

The inventors observed that some suspensions with high solid drug concentrations maintain their uniformity of composition, i.e. may not show sedimentation upon storage at about 25°C, for at least two days, yet when a flow-causing pressure is applied the suspensions can become non-uniform. The invention includes compositions and methods for preventing pressure-induced separation of pumped, viscous suspensions. When viscous suspensions are pumped under pressure, separation of the solids from the liquid carrier is often observed. Typically, the pump delivers a fluid that contains a reduced amount of solids and the solids accumulate behind the orifice and are not delivered to the patient. In preferred embodiments, the drug delivery devices of the invention comprise one or more suspension flow-enhancement elements that substantially prevent pressure-induced separation of pumped, viscous suspensions.

For example, this phenomenon was observed during an experiment to deliver a suspension of LD and water with a viscosity of approximately 50 Pa·s (50,000 cP). The driving pressure was approximately 104,14 cm (41 inches) H₂O through a nozzle with an inner diameter of 0.603 mm. The suspension separated and a murky fluid dripped from the end of the nozzle. As the pressure was increased to 152,4 (60) and then 203,2 (80) in HzO, the separation persisted, with increasing clarity of the exuding fluid. As the pressure was decreased by increasing the nozzle diameter, the effect was lessened, but was not eliminated.

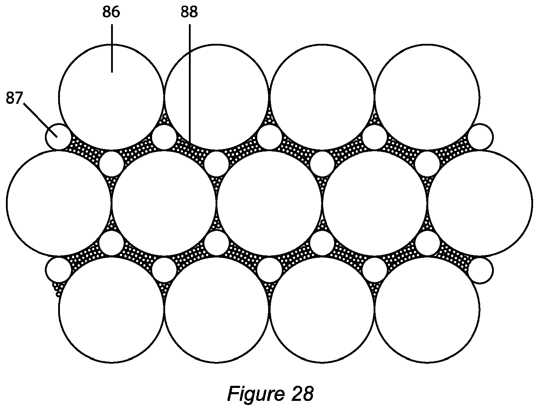

The experiments showed that pressure induced flow can cause formation of a filtering plug, the plug passing more of the carrier fluid and less of the solid drug. Such pressure or flow-induced sedimentation, i.e., filtering-plug formation, makes it difficult, if not impossible, to maintain a fixed dose rate by controlling the flow. Sedimentation leading to filtering may be alleviated when the suspended particle sizes are bimodally or multimodally distributed. Suspensions with multimodal particle size distributions tend to have superior flow characteristics over particles with unimodal particle size distributions, thereby reducing or eliminating the of separation or sedimentation of the solids from the liquid carrier that can occur when a suspension is pumped. Filtering could be reduced or avoided by increasing, through the bimodally or multimodally distributed particle sizes, the volume fraction, i.e., packing density, of the suspended solid drug, typically to greater than about 0.64, for example to between 0.65 and 0.69. A two-dimensional example of an optimal trimodal distribution of particle sizes is illustrated in Figure 28. The largest particle **86** is shown packed with a second smaller particle **87** and a further smaller third particle size **88**. Particle **88** is approximately 1/5^{th} the diameter of **87** and particle **87** is approximately 1/5^{th} the diameter of the particle **86.**

The invention comprises suspensions for infusion into the mouth comprising bimodal or multimodal particle size distributions, preferably wherein the ratio of the average particle diameters for the peaks is in the range of 3:1 to 7:1, e.g., about 3:1, 4:1, 5:1, 6:1, or 7:1. In the bimodal or multimodal distributions particle sizes can peak, for example, between 0.5 µm and 100 µm, such as between 1 µm and 50 µm, or between 1 µm and 30 µm, or between 1 µm and 15 µm. In general, proximal particle sizes at the maxima of the bimodal or multimodal distribution differ twofold or more, for example between two and fourfold, or between four and six-fold. In an exemplary bimodal distribution the weight-based amount of the larger particles can equal or be greater than that of the smaller particles. Typically the large particle: small particle weight ratio is typically greater than 1; it can be, for example, between 1 and 2, such as between 1.2 and 1.8, such as about 1.5.

The invention comprises reduction or elimination of pump-driven suspension separation in the intra-oral drug delivery devices by use of one or more of the following suspension flow enhancement elements:
1. Formulation of pumped suspensions with multimodal particle size distributions that increase the volume fraction of solids. As previously described, the invention comprises suspensions for infusion into the mouth comprising multimodal particle size distributions, preferably wherein the ratio of the average particle diameters for the peaks is in the range of 3:1 to 7:1.
2. Use of excipients (e.g., lubricants, glidants, anti-adhesives, wetting agents, etc.) in the formulation that enhance the flow of the particles through the orifice or tube, exemplified by surfactants used as food additives, such as monoesters of glycerol and fatty acids like glyceryl monooleate or glyceryl monostearate, or a polysorbate like Polysorbate 80, 65, 60 or 20.
3. Use of excipients in the formulation that modify the surface properties of the orifice material to enhance the flow of particles through the orifice or tube, such as a fatty acids, or coating the orifice with a perfluorinated polymer, exemplified by Teflon.
4. Flaring of the orifice to enhance the flow of particles through the orifice or tube.
5. Use of an orifice inner diameter of at least 10 or preferably 20 times the maximum effective particle size.
6. Selection of a formulation viscosity, concentration, and flow rate, and an orifice inner diameter, such that the pressure on the fluid is less than 1000 kPa (10 bars), and preferably less than 500 kPa (5 bars).

The invention features combinations of these designs and methods such that the drug concentration in the suspension delivered by the drug delivery device varies by less than 20%, 10%, 5%, and preferably 3% from the average during each one hour interval over a period of 8, 16 or 24 hours.

### ORAL LIQUID IMPERMEABLE DRUG RESERVOIRS

Solid or fluid drug formulations that are susceptible to oxidation, such as LD, CD and LD prodrugs, benserazide, and COMT inhibitors are preferably stored in containers that are substantially free of oxygen.

Solid drugs, such as LD, CD and LD prodrugs are preferably stored in containers that are substantially free of water. Non-aqueous formulations of LD containing fluids, and non-aqueous formulations of LD prodrug containing fluids, are also preferably stored in containers that are substantially free of water.

The preferred drug reservoirs of the invention are oral liquid impermeable reservoirs. For such oral liquid impermeable drug reservoirs, 1, 4, 8, 16, 24, 48 or 72 hours after placing a drug delivery device including a fresh reservoir in a patient's mouth and initiating the administration, less than 5%, 3%, or 1% by weight of the drug-including solid or drug-including fluid in the reservoir includes an oral liquid (e.g., less than 1% after 1 hour, less than 1% after 24 hours, less than 3% after 8 hours, less than 5% after 4 hours, less than 5% after 72 hours). The oral liquid impermeable reservoirs may contain one or more drugs in solid form or in fluid form. Oral liquids include saliva, water, water-diluted alcohol and other fluids commonly found in the mouth or that are drunk by the patient. Exemplary oral liquid impermeable reservoirs can be made of a metal, or a plastic that can be elastomeric. Metallic reservoirs can include, for example aluminum, magnesium, titanium or iron alloys of these. When made of a plastic it can have a metallic barrier layer; or a non-metallized plastic or elastomer used for packaging of food, or for drink-containing bottles, or in a fabric of washable clothing (e.g., Nylon or Dacron), or in stoppers or seals of drink containing bottles, or in septums of vials containing solutions of drugs. Ingress of oral liquids into openings in the reservoir can be prevented or minimized by the use of one or more valves, squeegees, baffles, rotating augers, rotating drums, propellants, pneumatic pumps, diaphragm pumps, hydrophobic materials, and/or hydrophobic fluids. In some embodiments, multiple doses of fluid or solid drug are contained within multiple, impermeable reservoirs or compartments.

While the flow of a highly viscous, low solubility material substantially decreases the potential for saliva ingress, other methods that substantially prevent the ingress of saliva can be utilized. Saliva ingress can be primarily associated with capillary action, or the interaction between the surface tension of the liquid, in this case saliva, and the adhesive forces between the saliva and the device. Capillary action occurs when the adhesive forces between the surface of the tubing and the saliva are stronger than the cohesive forces (surface tension) of the saliva. One method to eliminate the capillary action is to reduce the cohesive forces by utilizing a large diameter tubing between the drug reservoir and the exit orifice. Another method to eliminate capillary action is to reduce the adhesive force of the surface of the tubing through the use of a hydrophobic coating on the inner surface of the tubing. The goal of the coating is to prevent wetting of the tubing. By decreasing the surface energy of the tubing to achieve a substantially greater than 90 degree contact angle between the saliva and the inner surface of the tubing, capillary action is eliminated. Hydrophobic coatings such as parafilm and Teflon are examples of hydrophobic coatings. Another method of reducing capillary action is the use of a non-aqueous or hydrophobic carrier in the drug suspension. Non-aqueous or hydrophobic carriers will repel saliva and only those particles at the very surface of the flow front will be exposed to saliva. Examples of hydrophobic carriers are oils and waxes. Another method to limit ingress of saliva is the use of a check valve **16** (illustrated in Figures 23A and 23B). In times where the flow is halted or paused, the pressure gradient across the check valve **16** is eliminated, closing the valve and preventing the flow of drug and the ingress of saliva.

### METHODS OF USE AND METHODS OF TREATING DISEASE

The drug delivery devices of the invention can be used to orally administer drugs to patients in therapeutically effective amounts. Similarly, the formulations of the invention can be administered to patients in therapeutically effective amounts. For example, an amount is administered which prevents, delays, reduces, or eliminates the symptoms of a disease, such as PD, bacterial infections, cancer, pain, organ transplantation, disordered sleep, epilepsy and seizures, anxiety, mood disorders, post-traumatic stress disorder, cancer, arrhythmia, hypertension, heart failure, spasticity, and diabetic nephropathy. Using the drug delivery devices of the invention, a drug appropriate for the treatment of a given disease to be treated can be formulated and administered using the methods, compositions, and devices described herein.

Many drugs with narrow therapeutic indices benefit from drug delivery devices and methods that result in small fluctuation indices. For example, Table 2 summarizes the fluctuation indices of extended release tablet formulations of anti-epileptic drugs reported in various studies (from "Extended-release antiepileptic drugs: A comparison of pharmacokinetic parameters relative to original immediate-release formulations", Ilo E. Leppik and Collin A. Hovinga, Epilepsia, 54(1):28-35, 2013).

**Table 2**

| Drug | Fluctuation Index (SD) |
|---|---|
| Carbamazepine | 0.31 (0.1) |
| | 0.26 (0.1) |
| | 0.47 |
| | 0.49 |
| Divalproate sodium | 0.39 (0.15) |
| | 0.67 (0.16) |
| | 0.34 (0.15) |
| | 0.67 (0.17) |
| | 0.59 (0.27) |
| | 0.46 (0.16) |
| | 0.71 (0.20) |
| Lamotrigine | 0.341 |
| | 0.817 |
| | 0.209 |
| | 0.545 |
| | 0.986 |
| | 0.318 |
| Oxcarbazepine | 0.39 (0.08) |
| | 0.54 (0.09) |
| Levetiracetam | 1.19 |
| | 1.27 |

Described herein is also a method of treating a disease or medical condition using any of the devices, drugs, formulations, and methods disclosed herein, wherein the fluctuation index is less than or equal to 2.0, 1.5, 1.0, 0.75, 0.50, 0.25, or 0.15. For example, the disease or medical condition to be treated may be Parkinson's disease, bacterial infections, cancer, pain, organ transplantation, disordered sleep, epilepsy and seizures, anxiety, mood disorders, post-traumatic stress disorder, cancer, arrhythmia, hypertension, heart failure, spasticity, dementia, diabetic nephropathy, gastroparesis, xerostomia, and dementia.

Drug dosages administered using the methods of the invention may be higher or lower than those administered using traditional, infrequent dosing regimens. A lower daily dose is possible without loss of efficacy when continuous or semi-continuous administration reduces troughs in the drug's steady state circulating plasma concentration, enabling the drug's plasma concentration to remain above the minimum effective plasma concentration without the need for high peak concentrations. A higher daily dose is possible without increased side effects when continuous or semi-continuous administration reduces peaks in the drug's steady state circulating plasma concentration, enabling an increase in the drug's average plasma concentration without the need for high peak concentrations.

The methods of the invention provide a dosing regimen having an improved safety profile as adverse events associated with peak plasma concentrations (i.e., a Cₘₐₓ characteristic of oral unit dosage forms) are eliminated. Thus, the methods, compositions, and devices of the invention can be used to deliver drugs having a narrow therapeutic window in the patient population being treated (i.e., patients refractory to standard therapeutic regimens). Details provided below for the treatment of PD can be applicable to the formulation and administration of drugs for the treatment of other diseases.

### Treatment of PD

For the treatment of PD, typical administered dose ranges are from about 20 µmole/kg to about 200 µmole/kg of LD or LD prodrug per day. The typical daily dose of the optionally co-administered DDC inhibitor is between about 5 µmole/kg and about 50 µmole/kg. For example, the typical daily dose for a patient weighing 75 kg is from about 1.5 millimoles to about 15 millimoles of LD or LD prodrug. Optionally, a molar amount of a DDC inhibitor between about 10 % and about 40 % of the molar amount of the LD or LD prodrug, for example between 15 % and 30 %, may be added.

Preferred modes of administration of the drug-including solid or fluid are via drug delivery devices that are removably secured in the mouth, and which administer the drug into the mouth or into the nasal cavity for a period of at least 4 hours. The drug may be administered at a variable rate, although constant rate administration is preferred. Administration is preferably continuous or semi-continuous.

The administration into the mouth can be for 24 hours daily or it can be limited to the awake period, typically about 16 hours. When limited to the awake period it can be advantageous to administer a morning bolus to more rapidly raise the plasma concentration of the LD than a constant rate administration would. The morning bolus can be delivered, for example, through an orally taken pill or pills of LD and a DDC inhibitor or it can be through administration of a solid or fluid drug into the mouth using the drug devices of the invention. Alternatively, the exterior of the drug delivery device may include a drug, such that a bolus of the drug is delivered into the mouth when the device is first inserted into the mouth.

The invention includes pharmaceutical compositions as characterized in the claims useful in methods of administering into the mouth one or more drugs (e.g., LD and CD) from one or more drug reservoirs residing in the cavity of the mouth including a total volume of 0.1 - 10 mL of drugs, e.g., 0.1 - 1.0, 1.0-2.0, 2.0-3.0, 3.0-4.0, 4.0-5.0, 5.0-6.0, 6.0-7.0, 7.0-8.0, 8.0-9.0, or 9.0-10 mL. The methods of administering the one or more drugs (in either solid or fluid form) can be performed at a rate in the range of 0.03 - 1.25 mL/hour, e.g., 0.03 - 0.10, 0.10-0.20, 0.20-0.30, 0.30-0.40, 0.40-0.50, 0.50-0.60, 0.60-0.70, 0.70-0.80, 0.80-0.90, 0.90-1.0, 1.0-1.1, or 1.1-1.25 mL/hour. The methods of administering the one or more drugs can be performed at an average rate of less than 1 mg per hour, 1-10 mg per hour, 10 - 25 mg per hour, 25 - 50 mg per hour, 50 - 75 mg per hour, 75 - 100 mg per hour, 100 - 1025 mg per hour, or greater than 125 mg per hour. The methods of administering one or more drugs can be performed via continuous and/or semi-continuous administration. Preferably, the method includes holding the average administration rate constant or near constant for a period of 4, 8, 12, 16 or 24 hours during the day. For example, the volume administered every hour may vary from the average hourly administration rate during the infusion period by less than ±10% or ± 20% per hour, or by ±10% or ± 20% per 15 minute period. methods of administering one or more drugs into the mouth can be performed using any of the drug delivery devices described herein.

Continuous or semi-continuous administration using the drug delivery devices and formulations of the invention can reduce concentration fluctuations of the therapeutic drug in body fluid, for example in blood, plasma or serum. It can provide, for example, a plasma concentration profile where the difference between peak concentrations and nadir concentrations of the therapeutic drug is less than ±70% of the average concentration through a period in which the drug is administered, for example it can be less than ±50%, less than ±30%, less than ±20%, or less than ±10% of the time averaged concentration over a period of greater than or equal to 4 hours (e.g., 8, 12, 16 or 24 hours).

Described herein is a method of treating a disease in a patient, the method including: (a) inserting a drug delivery device into the patient's mouth; (b) starting a drug administration from the device; (c) administering into the patient's mouth one or more drugs, using continuous or semi-continuous administration, for a period of 4 hours to 7 days at an hourly rate in the range of 0.015 - 1.25 mL/hour or 1-125 mg/hour; and (d) removing the drug delivery device from the mouth; wherein the drug delivery device includes a oral liquid impermeable reservoir of 0.1-5 mL volume (e.g., 0.1-1 mL, 0.5-3 mL, or 3-5 mL), and the reservoir includes a solid or fluid including a drug. Optionally, the method may also include the optional step of: (e) stopping the drug delivery from the device. The invention further includes a method wherein steps a, b, c, d and e are performed at least twice over a period of 4 hours to 7 days. The drug may include a total of greater than 1 millimole of LD or LD prodrug.

Described herein is a method of treating a disease in a patient, the method including: (a) inserting a drug delivery device into the patient's mouth; (b) starting a drug administration from the device; (c) administering into the patient's mouth one or more drugs, using continuous or semi-continuous administration, for a period of 4 hours to 7 days at an hourly rate in the range of 0.015 - 1.25 mL/hour or 1-125 mg/hour; (d) removing the drug delivery device from the mouth; and (e) stopping the drug delivery from the device, wherein: (1) the drug delivery device includes a reservoir of 0.1-5 mL volume (e.g., 0.1-1 mL, 0.5-3 mL, or 3-5 mL), and the reservoir includes a solid or fluid including a drug, and (2) steps a, b, c, d and e are performed at least twice over a period of 4 hours to 7 days. The drug may include a total of greater than 1 millimole of LD or LD prodrug.

The invention features a drug delivery device for treating Parkinson's disease in a subject as characterized in the claims useful in a method for treating Parkinson's disease in a patient, the method including: (a) removably inserting a drug delivery device into the patient's mouth, the drug delivery device including an oral liquid impermeable reservoir of 0.1-5 mL volume (e.g., 0.1-1 mL, 0.5-3 mL, or 3-5 mL), and the reservoir including a solid or fluid including a total of greater than 1 millimole of LD or a LD prodrug; (b) administering into the patient's mouth the solid or fluid for a period of at least 8 hours at an hourly rate in the range of 0.03 - 1.25 mL/hour or 10- 125 mg/hour, such that a circulating plasma LD concentration greater than 400 ng/mL and less than 7,500 ng/mL is continuously maintained for a period of at least 8 hours during the administration; and (c) removing the drug delivery device from the patient's mouth. The LD or LD prodrug including solid or fluid can be administered into the mouth at such a rate that a circulating plasma LD concentration greater than 800 ng/mL, 1,200 ng/mL, 1,600 ng/mL, or 2,000 ng/mL (e.g., from 800 to 1,500, from 1,000 to 2,000, from 1,600 to 2,500, or from 1,500 to 3,000 ng/mL, depending upon the condition of the patient) is continuously maintained for a period of at least 2 hours, 3 hours, 4 hours, 8 hours, 16 hours or 24 hours during the administration. The LD or LD prodrug including solid or fluid can be administered into the mouth at such a rate that a circulating plasma LD concentration greater than 400 ng/mL, 800 ng/mL, 1,200 ng/mL, 1,600 ng/mL, or 2,000 is achieved within 60 minutes of the initiation of the infusion. LD prodrug can be administered into the mouth at such a rate that the circulating plasma concentration of the LD prodrug during the administration does not exceed 100 ng/mL, 50 ng/mL, 30 ng/mL, or 10 ng/mL. The LD or LD prodrug including solid or fluid can be administered into the mouth at such a rate that a circulating plasma LD concentration less than 7,500 ng/mL, 5,000 ng/mL, 3,500 ng/mL, 3,000 ng/mL, 2,500, or 2,000 ng/mL is continuously maintained for a period of at least 8 hours during the administration. The patient can receive an average daily dose of less than 10 mL, 7.5 mL, 5 mL, 3 mL, or 2 mL of the LD or LD prodrug including solid or fluid. The LD or LD prodrug including solid or fluid can be administered into the mouth at such a rate that the circulating LD plasma concentration varies by less than +/- 20%, +/- 15%, or +/- 10% from its mean for a period of at least 1 hour, 2 hours, 3 hours, or 4 hours.

The method can further include the co-administration of an effective amount of a DDC inhibitor such as benserazide, carbidopa or carbidopa prodrug. Carbidopa can be co-administered as a solid, suspension or emulsion, or as a solution of one of its highly water soluble prodrug salts, exemplified by carbidopa ethyl ester hydrochloride, by carbidopa methyl ester hydrochloride or by carbidopa amide hydrochloride. The molar amount of the co-administered DDC inhibitor can be between one-tenth and one-half of the molar amount of LD, preferably about ¼ ± 1/8th of the molar amount of LD. Preparations of the carbidopa prodrugs, recognized to be L-DOPA decarboxylase inhibitors, are described, for example, in U.S. Patent Nos. 3,895,052 and 7,101,912, and Patent Publication Nos. DE2062285A and FR2052983A1. A LD or LD prodrug including fluid can include a greater than 0.5 M LD or LD prodrug (e.g., 0.5 ± 0.1,0.6 ± 0.1,0.7 ± 0.1,0.8 ± 0.2, 1.0 ± 0.3, 1.5 ± 0.5, 2.0 ± 0.5, 0.6 ± 0.3, 0.75 ± 0.25, 1.0 ± 0.5, 1.5 ± 0.5, 2.0 ± 0.5, 2.5 ± 0.5, 3.0 ± 0.5, 3.5 ± 0.5, greater than 1.5, greater than 2, greater than 2.5, or greater than 3.5 moles per liter). The LD or LD prodrug and the DDC inhibitor can be co-administered as separate solids or fluids, or are contained in a single solid or fluid and administered into the patient.

The method can alleviate a motor or non-motor complication in a patient afflicted with Parkinson's disease, such as tremor, akinesia, bradykinesia, dyskinesia, dystonia, cognitive impairment, and disordered sleep.

The following examples are put forth so as to provide those of ordinary skill in the art with a complete disclosure and description of how the methods and compositions claimed herein are performed, made, and evaluated, and are intended to be purely exemplary of the invention and are not intended to limit the scope of what the inventors regard as their invention.

### EXAMPLES

### Example 1. Concentrated (0.80 g/mL, 4.0 M) LD Edible (Canola) Oil Based Suspension for Continuous or Semicontinuous Infusion in the Mouth and its Continuous Pumping.

A suspension was made by grinding in a mortar for 25 min a mixture of 7.0 g canola oil and 13.1 g Ajinomoto (unmilled) LD. As the grinding progressed the suspension became increasingly soft, then fluid and it could be slowly poured. It was about as easy to pour or slightly easier to pour than honey at about 25°C. Because the density of canola oil is 0.92 g/mL and that of LD about 1.5 g/mL, the expected volume is 7.6 + 8.7 = 16.3 mL and the calculated density is 1.23 g/mL. 14.03 g of the soft suspension was transferred to a Cane CronoPAR pump reservoir with graduations. The cross sectional area of the reservoir of the CronoPAR pump is about 4.5 cm². The volume was 11.5 mL, consistent with a density of 1.22 g/mL, close to the calculated. In the mouth the suspension is tasteless, oily and its solid grains were felt by the tongue. The concentration of LD in the suspension (13.1 g LD in 16.3 mL) is 804 mg/mL or 4.08 M.

The suspension was then continuously pumped using a Cane CronoPAR pump through a 50 cm long tubing of 0.24 cm internal diameter at a flow rate of 1 mL/hour, such that in the reservoir of 4.5 cm² cross sectional area the flow rate per cm² was about 0.22 mL/hour. (If in the mouth the cross sectional area of the reservoir would be about 0.5 cm², this flow rate per cm² would provide a flow of about 0.11 mL/hour). After about 7.5 mL of the 10.5 mL initial suspension volume in the reservoir was pumped, i.e., after about 7.5 hours, the pump occluded. The occlusion was not caused by filtering, as there was no solid cake residue. It was apparently caused by oil-softening the rubber of the piston, which was hard rubber prior to the experiment. The oil-softened rubber piston protruded where pressed by the steel plunger of the pump, the protrusion blocking the exit-hole of the reservoir.

The experiment shows that the oil based concentrated (0.80 g/mL, 4.0 M) suspension can be pumped and that in the oil based suspension the pump-driven suspension separation is much less than in the aqueous suspension of Example 6. The experiment also shows the need for pump materials that are compatible (e.g., dimensionally stable, non-softened, non-leachable, non-extractable) with a non-aqueous (e.g., oil-based) formulation, such as a plunger or piston made of neoprene.

### Example 2. Concentrated Edible (Olive) Oil Based 0.86 g/mL 4.4 M LD Suspension for Infusion in the Mouth.

4 g of unmilled Ajinomoto LD was added to 2 mL of olive oil and the mixture was ground in a mortar until it was homogeneous. The resulting suspension, a lubricated powder, was dripping (i.e., gravitationally flowing), but very slowly. Assuming that the density of LD is about 1.5 g/mL, similar to the reported density of tyrosine, and the reported density of olive oil being about 0.92 g/mL, the volume is 4.04 mL and the LD concentration is 857 mg/mL or about 4.35 M.

### Example 3. Mineral Oil Based 0.92 g/mL, 4.7 M Lubricated Particle Suspension for Infusion in the Mouth.

4.66 g of a lubricating mineral oil and 12.32 g of LD from Ajinomoto were ground for 10 min in a mortar. A lubricated, easy to plastically deform, suspension that could be pumped was formed. Because the density of the mineral oil is about 0.9 g/mL and that of LD about 1.5 g/mL, the calculated volume is 13.4 mL and the suspension contains about 0.92 g LD/mL, i.e., the LD concentration is about 4.7 M.

### Example 4. 0.62 g/mL, 3.1 M Mineral Oil Based Suspension of LD that Can Re-suspended by Shaking Prior to Infusion in the Mouth.

To the suspension of Example 3, 5.89 g mineral oil was added for a total of 10.55 g. Grinding in a mortar for 10 more min resulted in a fluid suspension of a viscosity of about 0,1 Pa·s (100 cP). It could be syringed with a 14G (14 gauge) 38 mm long syringe packaged with the Cane CronoPar pump reservoir. Air bubbles rapidly rose to the top. The density of paraffin oil being about 0.9 g/mL and that of LD about 1.5 g/mL the calculated volume is 11.7 mL + 8.2 mL = 19.9 mL and the calculated density is 1.15 g/mL. The estimated concentration of LD is about 0.62 g/mL or about 3.1 M. After 12 hours, sedimentation (translucent liquid on top) was observed. The suspension was easy to re-homogenize by shaking.

### Example 5. Fluid 0.87 g/mL, 4.4 M Aqueous Suspension of LD with added Polysorbate 60 for Infusion in the Mouth.

11.9 g LD (Ajinomoto) was ground in a mortar with 8 g water of and 0.31 g of Polysorbate 60. The apparent viscosity of the mixture was greater than that of water but less than that of ethylene glycol. Following grinding hourly for about 10 min over 6 hours, 2.6 g of the initially 8 g of water evaporated, leaving a suspension comprising 5.4 g of water, its viscosity resembling that of ethylene glycol. The suspension was easy to syringe with the 14G 38 mm long needle packaged with the Cane CronoPAR pump reservoir. Assuming that the density of LD is 1.5 g/mL and knowing that the density of Polysorbate 60 is 1.044 g/mL the calculated volume of the suspension was 7.9 + 0.3 + 5.4= 13.6 mL and the concentration of LD is 873 mg/mL or 4.43 M. The calculated weight (after the evaporation of 2.6 mL water) is 11.9 + 0.31 + 5.4 = 17.6 g and the calculated density is 1.29 g/mL. The volume of 13.37 g of the suspension transferred to the graduated reservoir of the Cane CronoPAR pump was 10.5 mL corresponding to a density of 1.27 g/mL. A translucent watery top-layer was observed after the reservoir was allowed to stand vertically for 1 hour.

### Example 6. Viscous 0.76 g/mL, 3.9 M Aqueous Suspension of LD with added Polysorbate 60 for Infusion in the Mouth and its Continuous Pumping.

13.6 g LD (Ajinomoto) was ground in a mortar with 4.1 g of water and 0.7 g of Polysorbate 60. After 15 min grinding the suspension was soft, viscous and homogenous; it did not freely flow and trapped air bubbles were not visibly mobile in the soft suspension, but was easy to stir, i.e., it was flowing under pressure. The apparent fluidity of the mixture was less than that of honey at room temperature, similar to that of mustard preparations sold in jars, estimated at about 50 Pa·s (500 poise). Its taste was slightly bitter. There was no visually observable sedimentation or inhomogeneity after 12 hours standing vertically in the Cane CronoPAR pump reservoir.

Assuming that the density of LD is 1.5 g/mL and knowing that the density of Polysorbate 60 is 1.044 g/mL the calculated volume of the suspension was 9.07 + 0.7 + 4.1= 13.9 mL and the concentration of LD in the absence of trapped air would be 0.978 g/mL or 4.97 M. The actual weight of the suspension was 17.8 g, 0.6 g less than the expected 18.4 g, possibly because of water evaporation. The expected density of the suspension would be 17.8 / 13.9 = 1.28 g/mL if water did not evaporate and 17.8/13.3=1.34 g/mL if it did. 14.9 g of the suspension was transferred to a graduated reservoir of the Cane CronoPAR pump. Its expected volume was 14.9/1.34= 11.1 mL but the actually observed volume was 13.5 mL, i.e., the density was only 1.10 g/mL, showing that about 22 % of the volume was air. The calculated concentration adjusted for the air trapped in the suspension was 0.978 × 0.78= 0.76 g/mL or 3.9 M.

After 3 days' storage at about 25°C there was no visible sedimentation, nor did the trapped visible air bubbles rise.

When the suspension was pumped continuously for about 5 hours with the Cane CronoPAR pump through a 50 cm long tubing of 0.24 cm internal diameter at a flow rate of 1 mL/hour, i.e., after about 5 mL were pumped and about 8 mL were left the pump signaled occlusion. The cross sectional area of the reservoir of the Cane CronoPAR pump was about 4.5 cm², such that the flow rate per cm² was about 0.22 mL/hour. (If in the mouth the cross sectional area of the reservoir would be about 0.5 cm², this flow rate per cm² would provide a flow of about 0.11 mL/hour). The occlusion persisted when the tube was shortened to 20 cm, then 10 cm, then 2.5 cm then altogether removed. When the reservoir was opened it contained a solid cake that was easily broken to small solid pieces, clearly showing sedimentation and filtering under the pumping pressure and flow.

The experiment showed that the aqueous 0.76 g/mL, 3.9 M suspension of LD made with Polysorbate 60 can be pumped. However, the pumped suspension is not uniform: the suspension in the reservoir is sedimenting while pumped and the effluent is richer in water than the suspension, ultimately a solid cake, left behind. In comparison with the pumped oil-based similarly concentrated (0.8 g/mL, 4 M) suspension of Experiment 1, the water based suspension is less uniform and experiences greater pump-driven suspension separation.

### Example 7. Aqueous acidic 2 M solution of LDEE.HCl.

The preparation is carried out under nitrogen. The solution for infusion into the mouth can be prepared by dissolving 1 mole of gaseous HCl in 500 mL absolute ethanol with cooling such that the temperature does not exceed 30 °C. To the HCl solution in ethanol 0.5 moles of LD is added in small portions and with cooling and rapid stirring, the temperature maintained between 0 °C and 10 °C during addition. The excess ethanol and the HCl are stripped by distillation at 50±5°C under vacuum, at a pressure of 2,666-6,666 kPa (20-50 mm Hg). 200 mL of ethanol is added to dissolve the residue and stripped by distillation at 50±5°C under vacuum, at a pressure of 2,666-6,666 kPa (20- 50 mm Hg) the distillation continued until the weight of the residue is about constant. The volume of the residual LDEE.HCl is about 90 mL. To prepare the 2 M LDEE.HCl solution, 160 mL of an aqueous 66.6 mM solution of trisodium citrate is slowly added stirring and chilling in an ice bath, while the pH and the temperature are monitored. During the addition, the temperature kept between 0 °C and 10 °C and the pH kept below pH 4. The pH is then adjusted to pH 2.5-3.0 with drops of 6 M NaOH or 6 M HCl and the solution, under a nitrogen atmosphere, is stored refrigerated at 5±3°C. A reservoir intended for a patient requiring daily 1 g LD molar equivalent would contain 2.5 mL infusible 2 M LDEE.HCl, the total infused and non-infused fluid volume being less than 3 mL.

### Example 8. Acidic 1.5 M LDEE.HCl solution in ethanol.

The preparation is carried out under nitrogen. The solution for infusion into the mouth can be prepared by dissolving 1.5 moles of gaseous HCl in 500 mL absolute ethanol with cooling such that the temperature does not exceed 30°C. To the HCl solution in ethanol 0.75 moles of LD is added in small portions and with cooling and rapid stirring, the temperature maintained between 0 °C and 10 °C during addition. The excess ethanol and the HCl are stripped by distillation at 50±5 °C under vacuum, at a pressure of less than 6,666 kPa (50 mm Hg). 200 mL of ethanol is added to dissolve the residue and again stripped by distillation at 50±5 °C under vacuum, at a pressure of less than 6,666 kPa (50 mm Hg), and the step is repeated, the distillation now continued until the weight of the residue is about constant. The volume of the residual LDEE.HCl is about 135 mL. To prepare the 1.5 M LDEE.HCl solution, 365 mL of absolute ethanol is added with stirring while the temperature is kept between 10°C and 25°C. The solution is stored at ambient temperature under a nitrogen atmosphere. A reservoir intended for a patient requiring daily 1 g LD molar equivalent would contain 3.33 mL infusible 1.5 M LDEE.HCl, the total infused and non-infused fluid volume being less than 4 mL.

### Example 9. Acidic 1.5 M LDEE.HCl solution in 1:1 (v:v) propylene glycol:water with ascorbic acid.

The preparation is carried out under nitrogen. The solution for infusion into the mouth can be prepared by dissolving 1.5 moles of gaseous HCl in 500 mL absolute ethanol with cooling such that the temperature does not exceed 30 °C. To the HCl solution in ethanol 0.75 moles of LD is added in small portions and with cooling and rapid stirring, the temperature maintained between 0 °C and 10 °C during addition. The excess ethanol and the HCl are stripped by distillation at 50±5 °C under vacuum, at a pressure of 2,666-6,666 kPa (20-50 mm Hg). 200 mL of ethanol is added to dissolve the residue and again stripped by distillation at 50±5 °C under vacuum, at a pressure of 2,666-6,666 kPa (20-50 mm Hg), and the step is repeated, the distillation now continued until the weight of the residue is about constant. The volume of the residual LDEE.HCl is about 100 mL. To prepare the 1.5 M LDEE.HCl solution in propylene glycol:water 1:1 volume:volume ratio, 182 mL of water containing 20 g of ascorbic acid is added with stirring while the temperature is kept between 10 °C and 25 °C, then 183 mL of propylene glycol is added and the pH is adjusted with drops of 6 M NaOH or 6 M HCl to 3.0 ± 0.6. The solution is stored at ambient temperature under a nitrogen atmosphere. A reservoir intended for patient requiring daily 1 g LD molar equivalent would contain about 3.33 mL infused 1.5 M LDEE.HCl, the total infused and non-infused fluid volume being less than 4 mL.

### Example 10. Acidic aqueous 2 M LDME.HCl solution.

The synthesis is carried out under nitrogen. The solution for infusion into the mouth can be prepared by dissolving 1 mole of gaseous HCl in 500 mL methanol with cooling such that the temperature does not exceed 30 °C. To the HCl solution in methanol 0.5 moles of LD is added in small portions and with cooling and rapid stirring, the temperature maintained between 0 °C and 10 °C during addition. The excess methanol and the HCl are stripped by distillation at 45±5 °C under vacuum, at a pressure of 2,666-6,666 kPa (20-50 mm Hg). 200 mL of ethanol is added to dissolve the residue and stripped by distillation at 50±5 °C under vacuum, at a pressure of 2,666-6,666 kPa (20-50 mm Hg) and the step is repeated. To prepare the about 2 M LDME.HCl solution, 150 mL of an aqueous 66.6 mM solution of trisodium citrate is slowly added stirring and chilling in an ice bath, while the pH and the temperature are monitored. During the addition, the temperature kept between 0 °C and 10 °C and the pH kept below pH 4. The pH is then adjusted to pH 2.5-3.0 with drops of 6 M NaOH or 6 M HCl and the solution, under a nitrogen atmosphere, is stored refrigerated at 5±3 °C. A reservoir intended for a patient requiring daily 1 g LD molar equivalent would contain 2.5 mL infusible 2 M LDEE.HCl, the total infused and non-infused fluid volume being less than 3 mL.

### Example 11. Aqueous acidic solution of 1.5 M LDEE.HCl with 0.25 M Benserazide.HCl.

Under nitrogen, 250 mL volume of the 2 M solution of Example 7 is diluted with 83 mL of an aqueous solution containing 25 g of Benserazide.HCl and the pH is adjusted with drops of 6 M NaOH or 6 M HCl to 3.0 ± 0.6. The solution is stored refrigerated at 5 ± 3°C under nitrogen. A reservoir intended for a patient requiring daily 1 g LD molar equivalent and 0.25 g Benserazide.HCl would contain about 4 mL of the solution of which 3.33 mL would be delivered.

### Example 12. Aqueous acidic 2 M LDEE.HCl solution with suspended CD stabilized with xanthan gum.

The average diameter and the mean diameter of the CD particles are both of about 5 µm or less. To the about 250 mL volume of the 2 M solution of LDEE.HCl of Example 7, 12.5 g of CD is added under nitrogen, then xantham gum is added to 1 weight % under nitrogen, and the mixture is ball milled under nitrogen to form a homogeneous suspension. The suspension is stored refrigerated at 5 ± 3 °C under nitrogen. A reservoir intended for a patient requiring daily 1 g LD molar equivalent and 0.25 g CD would contain about 3 mL of the suspension of which 2.5 mL would be delivered. It would be shaken before use to re-suspend the particles if precipitated.

### Example 13. Aqueous acidic 2 M LDME.HCl solution with suspended CD stabilized with polyethylene glycol.

The average diameter and the mean diameter of the CD particles are both of about 5 µm or less. To the about 250 mL volume of the 2 M solution of LDME.HCl of Example 10, 12.5 g of CD is added under nitrogen, then xantham gum is added to 1 weight % under nitrogen, and the mixture is ball milled under nitrogen to form a homogeneous suspension. The suspension is stored refrigerated at 5 ± 3 °C under nitrogen. A reservoir intended for a patient requiring daily 1 g LD molar equivalent and 0.25 g CD would contain about 3 mL of the suspension of which 2.5 mL would be delivered.

### Example 14. Suspension of LD with CD for infusion into the mouth.

The average diameter and the mean diameter of the LD and CD particles are both between about 1 µm and 5 µm. To 100 mL of water 50 g of LD, 12.5 g of CD, 2.5 g of polyethylene glycol 3350 (average molecular weight 3350) and 25 g of carboxymethyl cellulose (average molecular weight 250,000), and 1 g of polyoxyethylene (20) oleyl ether are added under nitrogen and the mixture is ball milled under nitrogen to form a homogeneous suspension. The suspension is stored refrigerated at 5 ± 3 °C under nitrogen. A reservoir intended for a patient requiring daily 1 g LD and 0.25 g CD would contain about 4 mL of the suspension of which 3.33 mL would be delivered.

### Example 15. Suspension of LD with CD for infusion into the mouth.

The average diameters of the particles are between about 1 µm and 5 µm. To 50 mL of water 50 g of LD, 12.5 g of CD and xanthum gum to 2 weight % are added under nitrogen and the mixture is ground in a mortar to form a homogeneous suspension. The suspension is stored refrigerated at 5 ± 3 °C under nitrogen. A reservoir intended for a patient requiring daily 1 g LD and 0.25 g CD would contain about 2.5 mL of the suspension of which 2.2 mL would be delivered.

### Example 16. Aqueous Zaleplon solution.

Zaleplon (3 g) is dissolved in 2 liters of a saturated aqueous solution of methyl- *β* -cyclodextrin (Cavasol^{®} W7 M PH of Wacker Chemie, Burghausen, Germany). A typical dose of 10 mg is infused into the mouth from an about 7.5 mL solution containing reservoir by infusing 6.7 mL of the solution.

### Example 17. Preparation and pumping of 1:1 Sinemet 25/250 : propylene glycol suspension.

Ten Sinemet 25/250 CD/LD pills weighing a total of 4.5 g were ground using a large porcelain mortar and pestle. The powder was transferred into an agate mortar, 4.5 g propylene glycol was added and the suspension was ground. The suspension was more homogeneous than the equivalent 1:1 weight ratio aqueous suspension, and was more fluid. The suspension was transferred to the Cane CronoPAR pump reservoir with a spatula and pumped normally at a flow rate of 5 mL/hour through the 30 cm long 2.7 mm ID tubing.

### Example 18. Preparation of concentrated aqueous CD/LD suspension

Ground 0.256 g CD (OChem catalog number 821C497, lot no. 90630A1) and 0.988 g LD (Ajinomoto Lot R059K008 from JSTAR) using an agate mortar and pestle, then added 1.25 g water and again ground in the agate mortar for 10 minutes. A white suspension was produced, fluid enough for pipetting with glass pipette (~ 1 mm ID tip and disposable rubber suction bulb). Transferred 1.8 g to vial to observe sedimentation. After 4 hours a practically particle-free, only slightly light scattering, layer of water was seen, indicative of sedimentation.

### Example 19. Preparation of concentrated propylene glycol CD/LD suspension

0.267 g CD (Ochem catalog number 821C497, lot no. 90630A1), 1.000 g LD (Ajinomoto Lot R059K008 from JSTAR) were placed in an agate mortar and ground using a pestle, then 1.25 g propylene glycol > 99.5 % (Sigma Aldrich W294004-1kg-K Lot MKBP3539V) was added. These were ground in the agate mortar using a pestle for 10 min. A white suspension was produced, more viscous than the aqueous suspension of Example 17 but still fluid enough for slow pipetting with a glass pipette (~ 1 mm ID tip and disposable rubber suction bulb). The suspension appears more homogeneous that the aqueous suspension of Example 17. Transferred 1.5 g to vial to observe sedimentation. After 4 hours there was no readily visible indication of sedimentation-the suspension appeared to remain uniform.

### EXAMPLE 20. Preparation of a 625 mg/mL LD suspension.

2.50 g of about 3.5 µm average particle size LD was mixed with 3.08 g of a 65 weight % aqueous sucrose solution. The density of the resulting 44.8 weight % LD suspension was about 1.4 g/mL. The viscous suspension containing 625 mg LD per mL was soft, easy to stir, yet gravitationally extremely slowly flowing, nearly non-flowing.

### EXAMPLE 21. Preparation of an 574 mg/mL LD suspension.

2 g of about 3.5 µm average particle size LD was mixed with 2.15 g of water and mixed until homogeneous. There was no visible sedimentation or formation of a less light scattering top layer after 3 weeks. The soft gel-like suspension contained 574 mg LD per mL.

### Example 22. Aqueous bupivacaine.HCl solution.

A reservoir contains 6 mL of a solution of 5 mg/mL bupivacaine.HCl and 80 mg/mL glucose.

### Example 23. Elastomeric reservoir for a drug delivery device, for delivery into the mouth of 5 mL drug solution.

Two 1 mm thick elastomeric butyl rubber sheets of oval shape, 4 cm long 3.5 cm wide, are joined at their edges such as to allow injection with a syringe having a 1 mm long needle the drug solution. One of the sheets has a plugged orifice through which the drug is released, the diameter of the orifice tailored for the desired flow rate. 6 mL of the drug solution is injected with the 1 mm long needle expanding the elastomeric reservoir by about 5.5 mm to about 7.5 mm thickness. Prior to insertion in the mouth, the plug is removed from the orifice. The drug-solution filled pump is inserted proximal to the cheek in the mouth.

### Example 24. Semi-continuous intra-oral infusion of LDEE.

A 52-year old healthy subject infused into his mouth a solution containing about 620 mg LDEE, equal to 545 mg LD, in 5.5mL of aqueous 0.5M, pH 3.5 LDEE solution. The infusion was made into the cheek pouch of the lower left jaw, between the gum and the cheek. The pump infused the drug for 8 hours over a total period of 9 hours and 10 minutes.

The solution was infused using a Cane CronoPAR pump. A Neria (product #78-060-2738) infusion set of 60 cm length, 8mm needle was used, with its distal end cut off to remove the adhesive and needle. The distal end of tube was unsecured in his mouth, but remained in place with minimal movement. The drug infusion was continuous, but was stopped during lunch for a period of 70 minutes. According to the manual, the Cane CronoPAR pump infuses the drug in boluses of 22 *µ*L. The bolus frequency was about one bolus ever two minutes. Lodosyn tablets (25 mg carbidopa) were taken orally twice during the infusion period, for a total dose of 50 mg carbidopa.

During the infusion there was no irritation or discomfort in the mouth. Starting at about six hours into the infusion there was hypersensitivity at the gum line of one tooth at the lower left jaw where the subject has had gum recession. The exposed tooth surface at the gum line of this tooth has experienced hypersensitivity on and off over many years, typically when irritated by excessive brushing with a hard toothbrush. The hypersensitivity causes pain to brushing and cold liquids. The hypersensitivity was reduced but still present at 8:00 at 12 hours post-infusion.

At 24 hours post-infusion, there was an area of dead, grey, peeling skin visible at the bottom of the left cheek pocket, about 1,27 cm (0.5 inches) long and 0,508 cm (0.2 inches) wide. The peeling skin revealed tissue underneath that was more pink than the surrounding mucosa. When felt with the tongue, the tissue in the surrounding area had a rougher texture than the corresponding tissue in the right cheek pocket.

At 48 hours post-infusion, the cheek pocket was healing. The newly exposed tissue was white, about 0,508 cm (0.2 inches) long and 0,254 cm (0.1 inches) wide. At 60 hours post-infusion the white zone had shrunk to about 0,254 cm (0.1 inches). Tooth hypersensitivity was much diminished, but still present. At 3 days post-infusion the white zone was still visible, and the tooth hypersensitivity was gone. At 4 days post-infusion the white zone was no longer visible and the tissue appears normal.

The taste of the LDEE solution was initially slightly sweet and bitter, but not bothersome to the subject

The infusion stimulated in the subject salivation and, consequently, swallowing. The subject recorded the number of times that he swallowed over two five minute periods. He swallowed 10 and 11 times during these two five minute periods, equal to an average rate of 2.1 swallows per minute. By contrast, in two other 5 minute periods when the subject was not infusing the drug, he swallowed 6 times and 5 times, equal to an average rate of 1.1 swallows per minute.

### Example 25. Semi-continuous, intra-oral infusion of LDEE with ascorbate.

*Summary:* A 81-year old healthy subject infused in his mouth 636 mg LD equivalent plus 117 mg of sodium ascorbate as an aqueous 0.47 M LDEE solution. The infused volume was 6.87 mL and the flow rate was 0.83 mL/hour. The solution was infused in the cheek pouch of the lower right jaw, i.e., between the gum and the cheek. Because of three 40-50 min long interruptions for discussions and meals during which pumping was stopped the delivery took 11 hours, 8.28 actual infusion hours and 2.72 hours of interruptions. At the end of the infusion there were no symptoms, i.e., no toothache, no pain in gum or cheek and no peeling of proximal drug-exposed mucous membranes. The infused solution had a pleasant sweetish taste with lemony-sour twinge taste, no bitterness. The taste did not change through the 11 hours of the experiment.

The solution was infused using a Cane CronoPAR pump. A Neria (product #79-110-2936) infusion set of 110 cm length, 6mm needle was used, with its distal end cut off to remove the adhesive and needle.

118 mg of crystalline sodium ascorbate, molecular weight 198 (Sigma Catalog No. A7631) were placed in the drug reservoir; it dissolved practically instantaneously in the added 7 mL of 0.47 M LDEE. The pH, measured with 3.0-5.5 range pHdyrion paper, was 3.8 ± 0.2; and that measured with the Baker-pHIX pH 3.6-6.1 paper was 4.0 ± 0.3. Best estimate pH 3.8 ± 0.2.

At the end of the infusion there were no symptoms, i.e., no toothache, no pain in gum or cheek and no peeling of proximal drug-exposed mucous membranes.

### Example 26. Semi-continuous, intra-oral infusion of LDME/CD.

The same 52-year old healthy subject infused into his mouth about 475 mg LD equivalent of LDME and 115 mg of CD, in 4.3mL of aqueous, approximately 111 mg/mL, Sirio brand LDME/CD (Chiesi Farmaceutici S.p.A., Parma, Italy) suspension, of starting pH greater than 6. The infusion was made into the cheek pouch of the lower right jaw, between the gum and the cheek. The pump infused the drug for 5 hours and 45 minutes over a total period of 7 hours and 30 minutes. The terminating pH was about 5.5.

The LDME/CD suspension was prepared by crushing Sirio (25/100 CD/LD) dispersible tablets using a mortar and pestle, adding an equal weight of water, and continuing to grind the suspension in using the mortar and pestle until the fluid has stopped evolving COz.

The solution was infused using a Cane CronoPAR pump. A Codan US Corporation (catalog # BC 575) infusion set of 152 cm length, approximately 7 mL dead volume, and approximately 2.5 mm internal diameter was used, with its distal end cut off to reduce the length to 40 cm. The distal end of tube was unsecured in the mouth, but remained in place with minimal movement. Pump delivery was stopped during lunch for 1 hour 45 minutes for lunch. According to the manual, the Cane CronoPAR pump infuses the drug in boluses of 22 u L. The bolus frequency was 1 minute 47 seconds, based on timing with a stopwatch.

The infusion was terminated early, after 5 hours and 45 minutes of infusion over a 7 hour 30 minute period, because of a feeling of potential hypersensitivity in one tooth of the lower right jaw, and some potential irritation of the right cheek. At 3.5 hours post-infusion there is mild hypersensitivity to brushing and hot and cold water at the gumline of a tooth in the lower right jaw. At 14 hours post-infusion the most of the mild hypersensitivity had resolved, and the mouth was virtually normal. At 24 and 48 hours post-infusion all was normal.

The taste of the LDME suspension was mildly tart and sweet. The subject found it to be acceptable.

### Example 27. Semi-continuous, intra-oral infusion of LD/CD.

The same 52-year old healthy subject infused into his mouth about 0.69 g LD and 0.07 g carbidopa, in 3.0 mL of 1.2M LD aqueous suspension, of unknown pH. The infusion was made into the cheek pouch of the lower right jaw, between the gum and the cheek. The pump infused the drug for 8 hours and 10 minutes over a total period of 9 hours and 5 minutes.

During the infusion there was no irritation or discomfort in the mouth. At the end of the infusion there was no feeling of irritation or discomfort in the mouth, and no visible signs of irritation of the mucosa. At 24, 48 and 72 hours post-infusion all was normal.

The solution was infused using a Cane CronoPAR pump. Initially, the subject attempted to infuse the fluid through a Neria (product #78-110-2936) infusion set of 110 cm length, 6mm needle length, with its distal end cut off to remove the adhesive and needle and reduce the tubing length to 40 cm. However, the Cane CronoPAR pump was unable to prime the tubing. No fluid at all entered the tubing. The subject then substituted a Codan US Corporation (catalog # BC 575) infusion set of 152 cm length, approximately 7 mL dead volume, and approximately 2.5 mm internal diameter, with its distal end cut off to reduce the length to 30 cm. The Cane CronoPAR pump was able to prime the tubing and infuse the suspension.

According to the manual, the Cane CronoPAR pump infuses the drug in boluses of 22 *µ*L. The bolus frequency was 3 minute 37 seconds, based on timing with stopwatch. The pump delivery was stopped for 55 minutes for lunch.

During the infusion there was no irritation or discomfort in the mouth. At the end of the infusion there was no feeling of irritation or discomfort in the mouth, and no visible signs of irritation of the mucosa. At 24, 48 and 72 hours post-infusion all was normal.

The LD/CD suspension had no taste whatsoever, and was acceptable to the subject. The LD/CD infusion did not stimulate salivation and swallowing. During two five-minute periods the subject recorded the number of times that he swallowed during the infusion. He swallowed 6 times during each of these two five minute periods, equal to an average rate of 1.2 swallows per minute. This is consistent with the previously measured rate of 1.1 swallows per minute observed when not infusing a drug and is half the 2.1 swallows per minute rate the subject observed when infusing LDEE.

### EXAMPLE 28. Non-sedimenting, long shelf-life suspensions formed of 3.4 µm average diameter L-DOPA particles and 65 weight % aqueous sucrose.

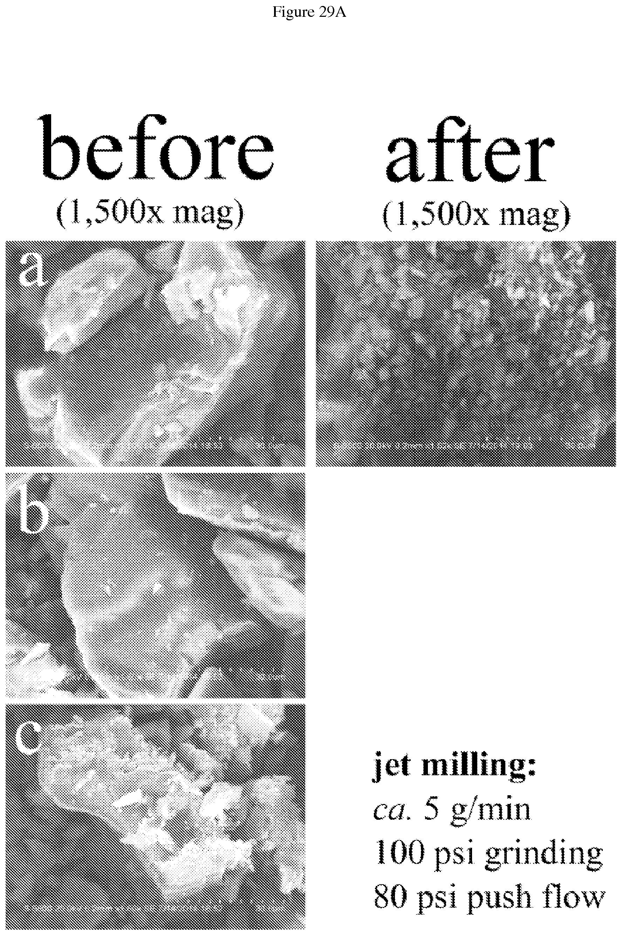

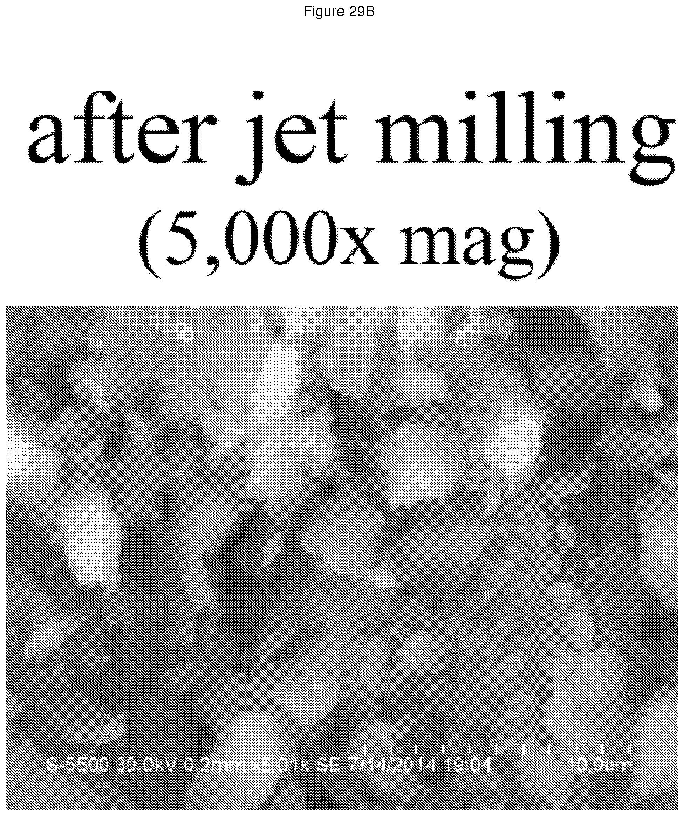

Jet Milled L-DOPA Particles. Using a laboratory jet miller rented from GlenMills Inc. (220 Delawanna Avenue Clifton, NJ 07014), LD (Ajinomoto North America Inc., 4020 Ajinomoto Drive, Raleigh, NC, 27610) was jet milled. The milling pressures were: 723,949 kPa (105 psi), supply line; 689,476 kPa (100 psi), grinding line; 551,581 kPa (80 psi), feed push line. The feed rate was about 1.5 (dial) or about 5 g per 20 minutes. Jet milling reduced the average size of the particles from 52 µm to 3.4 µm (excluding fines) as seen in Figures 29A and 29B.

A 65 weight % solution of sucrose was prepared by dissolving 65 g of the sugar in 35 g of water with mild heating. The clear solution was allowed to cool to room temperature, about 23 ± 2 °C. 3.42 g of the jet-milled LD was weighed in a small plastic cup. To the cup 3.44 g of the 65 weight % sucrose was added and the milled LD and sucrose solution were mixed with a spatula for 5 min until a homogeneous, viscous, toothpaste-like 1:1 wt/weight ratio suspension was obtained. 3 g of the suspension was transferred to a 2 mL capped plastic transparent mini-test-tube, filling it to the rim, showing that the density of the suspension was about 1.5 g/mL. (Water similarly filling the mini test-tube to the rim weighed about 2.0 g, showing that the volume of the test tube was about 2.0 mL and the density of the 3 g of suspension filling it about 1.5 g/mL). The suspension of 2 mL volume contained about 1.5 g of LD, i.e., 0.75 g LD per mL, or 3.8 millimoles LD per mL. The suspension also contained about 0.34 g sucrose per mL. There was no visible change, i.e., sedimentation, after 48 hours, or after 70 days.

In a second experiment, to the 1.5 g of the 1:1 weight ratio LD: 65 weight % sucrose suspension remaining in the plastic weighing cup an additional 1.5 g of the 65 weight % sucrose solution were added and mixed for 5 min with a spatula. About 2 mL of the resulting suspension, having honey-like viscosity, were transferred to a 2 mL mini-test tube. The suspension, though viscous, could still be poured. The suspension contained about 0.75 g of LD, i.e., 0.375 g LD per mL, or 1.9 millimoles LD per mL. The estimated sucrose concentration was about 0.8 g per mL. There was no visible change, i.e., sedimentation, after 48 hours, or after 70 days, but the soft suspension could no longer be poured. This suspension was much softer that the suspension containing 0.75 LD g/mL and 0.34 g sucrose/mL and consequently it is expected to be much easier to pump.

### Example 29. Composition of solid LD/CD for semi-continuous administration into the mouth using a gravure printed plastic ribbon.

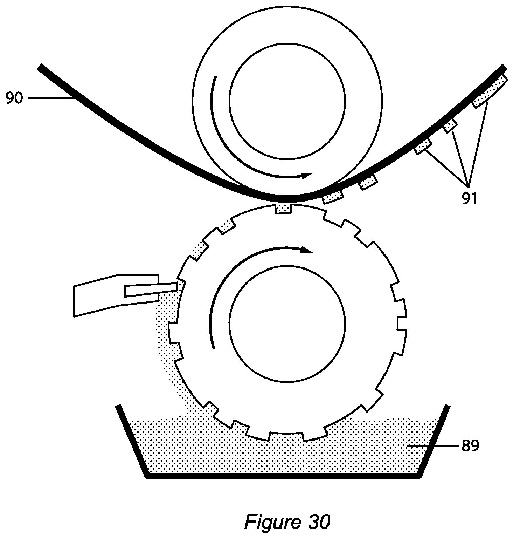

A suspension suitable for doctor blading can be made of 20 ±10 µM average particle size LD (100 g), 20 ±10 µM average particle size CD (25 g), and a 2 weight % aqueous starch solution (100 g). As illustrated in Figure 30, the paste **89** can be applied by gravure printing onto a sheet of calcium cross-linked alginic acid **90**, such that the drug containing printed island form a 2 dimensional ordered pattern of square features **91**, each feature of area 3.3 mm length × 3.3 mm width × 1.0 mm height. The center to center spacing between the features would be 0.5 cm. After drying each feature **91** would contain about 10 mg of LD and 2.5 mg CD. The sheet would be cut to 0.5 cm wide ribbons, each cm of the ribbon containing 20 mg LD and 5 mg CD, such that when the ribbon is delivered at a rate of 4 cm/hour the patient would receive a 10 mg LD dose every 7.5 min or 1.28 g LD per the 16 awake hours.

### Example 30. Tablets Glued to Plastic Ribbon

Sixty four (64) tablets, each containing 20 mg LD and 5 mg CD, of a composition similar to that in Sinemet^{®} 25/100 can be glued to a 33 cm long and 0.5 cm wide ribbon (with 0.5 cm center-to-center distances between the tablets) of Ca2+ cross-linked alginate for administering in the mouth every 15 min for the 16 awake hours a tablet to a PD patient requiring daily 1.28 g LD.

### Example 31. Drug loaded ribbon disintegrating in the mouth

A sheet containing LD and CD particles could be cast and sliced into ribbons that would be spooled and continuously fed into the mouth where the ribbon would disintegrate, releasing its LD and CD particles. The cast mixture would contain LD and CD particles of average and mean diameters between 1 µm and 20 µm mixed with calcium alginate. Optionally the cast sheet could also contain calcium alginate encapsulated citric acid particles and calcium alginate encapsulated sodium bicarbonate particles such that upon wetting by saliva in which the Ca²⁺ concentration is low the crosslinking Ca²⁺ ions of the alginate would be Na+ exchanged and the polymers will swell to form a hydrogel where reaction of the acid and the carbonate would produce COz, the evolving gas accelerating the disintegration.

### Example 32. Aqueous Newtonian Suspension

An about Newtonian suspension is a fluid suspension of drug particles in which the viscosity of the fluid is not substantially affected by shear. An aqueous Newtonian suspension of LD and CD can be generated by combining the drug powders with water, a low molecular weight hydrophilic polymer suspending agent, such as sodium carboxymethylcellulose (NaCMC), and a surfactant, such as polysorbate 80 (P80). Although a wide range of component concentrations could be used, one example could contain about 50% LD, about 12.5% CD, about 2% NaCMC, and about 0.2% P80. Other hydrophilic polymers that could be used in the formulation include hydroxypropyl cellulose (HPC), hydroxypropylmethyl cellulose (HPMC), hydroxyethyl cellulose (HEC), polyvinyl pyrrolidone (PVP), polyvinyl alcohol (PVA), and other neutral, negatively charged, or positively charged derivatives of cellulose, starch, other carbohydrates, chitin, etc. as well as neutral or charged polymers of short chained alcohols, carboxylic acids, amines, or other compounds. Other surfactants that could be used in the formulation include various polysorbates, sodium dodecyl sulfate and other charged fatty acid derivatives, fatty acids, block copolymers, etc. The suspension may include other excipients to enhance flowability, flavor, and appearance.

### Example 33. Aqueous Shear-Thinning (Pseudoplastic) Suspension

A shear-thinning suspension is a fluid suspension of drug particles in which the viscosity of the fluid decreases with increasing shear. Such suspensions are typically formulated with relatively high molecular weight hydrophilic polymers, such as gelatin, carbomers, and high molecular weight variants of the polymers listed in section 2.1. An example of an aqueous shear thinning suspension could contain about 30% LD, 7.5% CD, 0.5% Carbopol 934P, and 0.2% polysorbate 80.

### Example 34. Aqueous Shear-Thickening (Dilatent) Suspension

Some very high concentration suspensions thicken with increasing shear due to the squeezing out of lubricating liquid between the particles. Such a suspension could potentially be advantageous in that flow of the suspension would be interrupted if too much shear were applied to it, such as if the device were to be dislodged and the patient were to bite it. An example of such a formulation might be one containing about 60% LD, 15% CD, and 0.2% polysorbate 80 as a thick suspension in water.

### Example 35. Suspension in Low Molecular Weight PEG

Polyethylene glycol (PEG) is available in various average molecular weight ranges, and those with molecular weights below about 1000 are liquid at room temperature. An example of a liquid PEG suspension would be about 40% LD and 10% CD suspended in PEG-300. Other readily available PEG grades, such as PEG-400 and PEG-600 could also be used.

### Example 36. Suspension in Propylene Glycol

A suspension could be prepared by adding about 50 g of propylene glycol to about 40 g of LD powder and about 10 g of CD powder. The components are blended using a spatula or other mixing instrument to form a thick suspension.

### Example 37. Suspension in Glycerin

A suspension could be prepared by adding 60 g of glycerin to 32 g of LD powder and 8 g of CD powder. The components are blended using a spatula or other mixing instrument to form a thick suspension.

### Example 38. Suspension in Edible Oil

Various liquid triglycerides, such as soybean oil, sesame oil, olive oil, corn oil, medium chain triglyceride (MCT) oil, with or without low hydrophile/lipophile balance (HLB) surfactants, can be used to form suspensions of LD and CD powders. For example a suspension could be prepared by combining 49.5 g of MCT oil and 0.5 g of sorbitan monooleate and mixing to uniformly disperse. The liquid could then be added to 40 g of LD powder and 10 g of CD powder and blended using a stirring implement or a high-shear mixer to form a uniform suspension.

### Example 39. Suspension in Non-digested Oil

A non-digested oil, such as a low viscosity grade of mineral oil, could also be used as a suspending fluid in a similar manner as described in section 3.4. Non-digested does not infer that such oils are toxic; only that they are not digested and absorbed as food. For example, mineral oil is used at much higher doses as a laxative.

### Example 40. Aqueous Nanosuspensions

An aqueous nanosuspension could contain 32% LD, 8% CD, and about 2% of a surfactant, such as polysorbate 80. The nanosuspension would be prepared by combining the ingredients to form a suspension and then passing the suspension through a microfluidizer, media mill, or other high energy particle size reduction device. Various other surfactants could be used in the formulation, potentially including other polysorbates, sorbitan esters, polyethylene glycol fatty acid esters, other ethers or esters of alkanes and alkenes with hydrophilic polymers, lecithin, etc.

### Example 41. Non-aqueous Nanosuspension

A non-aqueous nanosupension could be prepared by combining up to 32% LD and up to 8% CD with a low-viscosity triglyceride oil with or without a low HLB surfactant. The resulting suspension would then be processed by passing it through a microfluidizer, media mill, or other device to reduce the size of drug particles into the submicron range.

### Example 42. Temperature Sensitive Suspension in Cocoa Butter

Cocoa butter is an edible oil extracted from cocoa beans. The oil has a typical melting range of about 34°C - 36.5°C, so that it is a solid at room temperature but becomes liquid at body temperature. A suspension can be prepared by melting about 50 g of cocoa butter at about 40°C and then stirring in 40 g of LD and 10 g of CD. The suspension can then be put into a device or a container, and, upon cooling will solidify.

### Example 43. Temperature Sensitive Suspension in Butter

A suspension can be prepared by melting at about 40°C butter (a water-in-oil emulsion remaining solid when refrigerated, melting between about 32°C and about 35°C), then stirring in 40 g of LD and 10 g of CD. The suspension can then be placed in the drug reservoir, and, upon placement in a refrigerator will solidify.

### Example 44. Temperature Sensitive Suspension in Low Melting Range Edible Oil

Two factors influence the melting range of triglyceride oils, the chain length and degree of saturation of the component fatty acid chains. Saturated medium chain length oils, such as coconut and palm oils, and long chain length oils containing a mixture of saturated and unsaturated fatty acid chains can be solid at or slightly below room temperature but liquid at body temperature. Saturated oils, such as tristearin, can also be combined with unsaturated oils, such as olive oil or soybean oil, in different ratios to obtain a target melting range. Similar to cocoa butter, such low melting range oils or mixtures of oil can be used to formulate a suspension of LD and CD that is solid during storage but becomes fluid once inserted into the patient's mouth.

As an example, a suspension could be prepared by heating about 20 grams of tristearin until it melts and then mixing in about 30 grams of purified olive oil. The mixed oils could then be mixed with 40 g of LD and 10 g of CD to form a thick suspension that is solid at low temperature but becomes fluid at body temperature.

### Example 45. Temperature Sensitive Suspension in Low Melting Range Non-digested Oil

Mineral oil and paraffin waxes can be combined in the correct ratio to form materials that are solid or semi-solid at room temperature but liquid at body temperature. Such mixtures may serve as a basis of a temperature sensitive suspension. Petrolatum is a well-known mixture of high and low melting hydrocarbons that is semi-solid at room temperature but melts near body temperature. Non-digested does not infer that such oils are toxic; only that they are not digested and absorbed as food.

A suspension can be prepared by warming 50 grams of white petrolatum to 40°C and then mixing in 40 grams of LD and 10 grams of CD.

### Example 46. Temperature Sensitive Suspension in PEG 1000/PEG 600 Blend

The melting range of PEG increases with increasing molecular weight. PEG-600 has a melting range of 20° - 25°C, whereas PEG-1000 has a melting range of 35° - 40°C. Combining the two can yield a material with a melting range between room temperature and body temperature.

A suspension can be prepared by warming 20 g of PEG-600 and 30 g of PEG-1000 to about 40°C and allowing the higher molecular weight PEG to melt. 40 g of LD and 10 g of CD can then be blended in to form a uniform suspension.

### Example 47. Extruded and Spheronized Microparticulates

An alternative to a standard suspension formulation is a microparticulate formulation in which the formulated drug substance is packaged into tiny beads that are released essentially one at a time. The beads could then be formulated in a non-solvent liquid that would in essence act as a lubricant to facilitate their movement within and from the device.

Extrusion and spheronization is a process that is commonly utilized in preparing capsule formulations of drug products. A suspension of the drug and excipients is prepared and extruded through small holes, and the extrudate is then released onto a plate that has a high rate of rotation within a stationary bowl, creating shear forces that break up the extrudate and form the pieces into tiny spheres.

A spheronized particle formulation could be prepared by combining about 64 grams of LD with 16 grams of CD and 20 grams of microcrystalline cellulose. The ingredients would then be blended and wetted by addition of water to form a thick suspension. The suspension would then be fed through an extruder and the extrudate fed into a spheronizer to form uniform spheres.

### Example 48. Microparticulates Generated by Spray Drying

Spray drying is another technique that can be used to generate particles of relatively uniform shape and diameter. A suspension of the API and excipients is forced through a spray nozzle at high pressure into a chamber in which a heated air flow quickly dries the droplets into particles.

Spray-dried particles could be generated by forming a suspension of micronized LD and CD and a binder, such as a hydrophilic cellulose derivative or PVP, in water or a short-chain alcohol. The relatively thin suspension would then be pumped through the spray nozzle of a spray drier to form drug-containing particles. While it may be easier to form smaller particles with this technique, the particle density may be less than with particles generated using other techniques.

### Example 49. Microparticulates Generated by Wurster Coating

Wurster coating is a bottom spray fluid bed coating methodology used to form even coats on particles. In pharmaceutical Wurster coating applications, it is typical to start with a seed particle, such as a sugar sphere, and apply the coating to that. The need to utilize a seed particle limits the drug load that can be obtained using this methodology.

To form Wurster-coated drug particles, a suspension similar to that described for spray drying would be generated. Inert particles of as small a size as possible would then be coated to form uniform drug spheres.

### Example 50. Microparticulates Generated by Granulation and Milling

Granulation is a process in which powders are compacted, with or without the addition of a liquid, to form hard aggregates. The aggregates can then be milled by various processes to form smaller aggregates, potentially of more uniform size.

A granulated formulation might contain LD and CD in the correct ratio along with a binding agent, such as microcrystalline cellulose, another cellulose derivative, PVP, or another hydrophilic polymer.

### Example 51. Lubricating Excipients for Microparticulate Formulations

Regardless of the methodology used to generate microparticulates, release of the drug particles from the device could be aided by formulating them as a suspension in a non-solvent fluid, such as a vegetable oil or a mineral oil. Polar solvents such as propylene glycol, low molecular weight PEGs, and glycerol might also be used for this purpose if dissolution of the particles into these fluids does not occur appreciably.

### Example 52. Propellant-driven LD/CD Suspension and Drug Delivery Device

LD/CD can be formulated as a viscous aqueous suspension. The suspension can contain about 0.6 g LD per mL, 0.15 g CD per mL and 0.34 g sucrose per mL. The suspension can contain LD and or CD with a bimodal particle size distribution, the larger with peaks at about 5 µm and 1 µm, the weight of the larger drug particles exceeding 1.5 fold the weight of the smaller ones. The viscosity can be greater than 20 Pa·s (200 Poise). The suspension may optionally contain a lubricant.

1.5 g of the suspension is placed in the ascending spiral of the device of Figures 20C and 20D. It is separated from the propellant by an elastomeric plunger whose resistance to flow is about 10 times greater than that of the suspension. 0.25 mL of refrigerated liquid propellant 1,1,1,2 tetrafluoroethane is placed into the central cylinder and the device os sealed.

The device is configured to continuously deliver LD at a rate of 62.5 mg/hr and CD at a rate of 15.6 mg/hr for 8 hours, equal to 0.5 g of LD and 0.125 g of CD over the 8 hour delivery period. The average rate of drug delivery can vary by less than ± 20% per hour over a period of 8 hours, at constant 37 °C and constant 89,632 kPa (13.0 psia). The drug delivery device can administer the LD and CD at a rate in the range of 80% - 120% of the average rate in less than about 60 minutes after the first insertion of the device into the patient's mouth. The drug delivery device includes several features such that (a) the average rate of delivery of the LD and CD is increased or decreased by less than about 20% at 101,353 kPa (14.7 psia) and at 77,911 kPa (11.3 psia), as compared to the average rate of delivery at 89,632 kPa (13.0 psia), and (b) no substantial drug bolus is delivered to the patient when the patient sucks on the device. These features would include: (a) calibrating the device such that it delivers drug at its target rate at 89,632 kPa (13.0 psia), (b) maintaining an internal pressure of greater than about 810.6 kPa (8 atm), (c) incorporation of a short tube (not shown in Figures 20C and 20D) protruding from the orifice of the device and forming a fluidic channel that is designed such that when the drug is being infused via a pressure head, the fluidic channel inflated and when low pressure is created by the mouth, the fluidic channel collapsed, causing it to kink and temporarily halt the infusion of the drug, and (d) a float valve in the fluidic channel.

The device can include a fastener to removably attach the device to the teeth. The above-mentioned short tube protruding from the orifice of the device would also serve to turn the drug delivery on and off when the device is inserted and removed from the mouth. When the drug delivery device is unfastened from the teeth, the fluidic channel kinks due to a change in the radius of curvature of the fluidic channel, halting the flow of drug. When the device is fastened to the teeth, the kink is removed and the drug can flow.

The device could be configured to deliver a bolus of less than 5% of the contents of a fresh drug reservoir, when immersed for five minutes in a stirred physiological saline solution at about 55 °C, as compared to an identical drug delivery device immersed for five minutes in a physiological saline solution of pH 7 at 37 °C.

The drug reservoir includes structural elements to withstand a bite by the patient with a force of at least 200 Newtons. The drug reservoir is an oral liquid impermeable reservoir. Saliva and other oral liquids are prevented from entering into the drug reservoir through the exit orifice due to several features of the device, including: (a) the continuous flow of fluid out of the device, (b) a pressure-sensitive valve (not shown in the figure), and (c) selection of dimensions for the exit orifice and channel that can prevent or reduce substantial ingress of oral liquids.

The oral fluid contacting surfaces of the drug delivery device can be selected to be compatible with oral fluids, such that the average rate of delivery of the LD and CD increased or decreased by less than about 20% after the deviceis immersed for five minutes in a stirred physiological saline solution at about 37 °C under any one of the following conditions, as compared to an identical drug delivery device immersed for five minutes in a physiological saline solution of pH 7 at 37 °C: (a) pH of about 2.5; (b) pH of about 9.0; (c) 5% by weight olive oil; and (d) 5% by weight ethanol.

The drug delivery device incorporates several features substantially eliminating the pump-driven separation of the suspension. These include: (a) use of a formulation with a bimodal particle size distribution with a ratio of average particle sizes of about 5:1; (b) a packing density of the LD/CD particles of about 95% of the theoretical maximum; (c) use of a flared orifice; (d) use of an orifice inner diameter of greater than 10 times the maximum effective particle size.

### Example 53. Propellant-driven Solid LD/CD Formulation and Drug Delivery Device

LD/CD is formulated as solid pills. A total of 48 pills are prepared containing a total of about 0.75 g LD, 0.19 g CD, and 0.19 g of dispersant. Each pill is substantially spherical and smooth, weighing 23.4 mg, with a volume of about 0.016 mL, and a diameter of about 0.31 cm. The pills are made by compression in a die and are orally disintegrating. The pills are placed in the ascending spiral of the device of Figures 20A and 20B, forming a single row of pills whose edges can contact each other with substantially no gaps. The volume surrounding the pills in the channel is filled with an edible vegetable oil. The pills and oil are separated from the propellant by an elastomeric plunger whose resistance to flow is about 10 times greater than that of the pills and oil. 0.25 mL of refrigerated liquid propellant 1,1,1,2 tetrafluoroethane is placed into the central cylinder and the device is sealed.

The device is configured to intermittently deliver LD at an average rate of 62.5 mg/hr and CD at an average rate of 15.6 mg/hr for 8 hours, equal to 0.75 g of LD and 0.19 g of CD over the 8 hour delivery period. A pill is delivered into the mouth every 10 minutes. The average rate of drug delivery is varied by less than ± 20% per hour over a period of 16 hours, at constant 37 °C and constant 89,632 kPa (13.0 psia). The drug delivery device administers the LD and CD at a rate in the range of 80% - 120% of the average rate in less than about 60 minutes after the first insertion of the device into the patient's mouth. The drug delivery device comprises several features such that (a) the average rate of delivery of the LD and CD increased or decreased by less than about 20% at 101,353 kPa (14.7 psia) and at 77,911 kPa (11.3 psia), as compared to the average rate of delivery at 89,632 kPa (13.0 psia), and (b) no substantial drug bolus is delivered to the patient when the patient sucks on the device. These features included: (a) calibrating the device such that it delivers drug at its target rate at 89,632 kPa (13.0 psia), (b) maintaining an internal pressure of greater than about 810.6 kPa (8 atm), and (c) incorporation of a short tube (not shown in Figures 20A and 20B) protruding from the orifice of the device and forming a fluidic channel that is designed such that when the drug is being infused via a pressure head, the fluidic channel inflates and when low pressure is created by the mouth, the fluidic channel collapses, causing it to kink and temporarily halting the infusion of the drug.

The device comprises a fastener to removably attach the device to the teeth. The above-mentioned short tube protruding from the orifice of the device also serves to turn the drug delivery on and off when the device is inserted and removed from the mouth. When the drug delivery device is unfastened from the teeth the fluidic channel kinked due to a change in the radius of curvature of the fluidic channel, halting the flow of drug. When the device is fastened to the teeth the kink is removed and the drug flows.

The device is configured to deliver a bolus of less than 5% of the contents of a fresh drug reservoir, when immersed for five minutes in a stirred physiological saline solution at about 55 °C, as compared to an identical drug delivery device immersed for five minutes in a physiological saline solution of pH 7 at 37 °C.

The drug reservoir comprises structural elements that are able to withstand a bite from the patient with a force of at least 200 Newtons. The drug reservoir is an oral liquid impermeable reservoir. Saliva and other oral liquids are prevented from entering into the drug reservoir through the exit orifice due to several features of the device, including: (a) the flow of pills and oil out of the device, (b) a duck-bill valve (not shown in the figure), and (c) the presence of the edible oil, which prevented the ingress of the oral fluids.

The oral fluid contacting surfaces of the drug delivery device were selected to be compatible with oral fluids, such that the average rate of delivery of the LD and CD increased or decreased by less than about 20% after the device is immersed for five minutes in a stirred physiological saline solution at about 37 °C comprising any one of the following conditions, as compared to an identical drug delivery device immersed for five minutes in a physiological saline solution of pH 7 at 37 °C: (a) pH of about 2.5; (b) pH of about 9.0; (c) 5% by weight olive oil; and (d) 5% by weight ethanol.

### Example 54. Delivery of silicone oil using a constant force spring.

A silicone oil viscosity standard of 200 P was used to model the delivery of a drug by a constant force pump prototype, illustrated in Figures 12A and 12B. The prototype comprised a polyethylene drug reservoir and a 16G nozzle bonded to it. The drug reservoir of the device was filled with approximately 1.2 mL of the silicone oil. The prototype used a 2,224 N (0.5 IbF) constant force spring as the driving force for the plunger. The spring was retracted and maintained in that state until the reservoir filled with 1.2 mL of silicone oil was placed inside the device. The entire device was then placed on a balance to obtain a baseline weight measurement. This measurement was compared to measurements of mass over time to determine mass loss (and correspondingly flow) vs time. The spring force was released and time was measured.

The device was placed on a bench and the effluent silicone oil was captured on a weigh boat. The device was placed onto the balance to obtain the mass loss measurement at 10-15 minute intervals. The test was run for 1.5 hours and the results showed a consistent flow rate of 0.08 mL/hr.

### Example 55. Preparation of a concentrated (0.79 g/mL, 4.0 M) temperature sensitive (cocoa butter) suspension of levodopa and its pumping.

Cocoa butter (Caribbean Cacao^{®}, unrefined, raw, organic) was purchased from Amazon. The cocoa butter was a hard oily solid that could be cut with a sharp knife at the ambient temperature of 23°C. A mixture of 6.55 g of the cocoa butter and 12.06 g of LD (Ajinomoto) were hand ground in a mortar for about 30 min and 16.2 g of the resulting powder was transferred to a reservoir of the Cane CronoPAR pump. The reservoir was plugged and the plunger was kept inserted to prevent water and moisture from entering. The plugged reservoir was warmed up in hot water, raising the temperature of the powder to about 55°C, causing it to densify to form a fluid suspension. Next, the plunger was removed and the warm suspension was stirred with a small spatula completing thereby the separation of the air initially trapped in the powder. The plunger was re-inserted and the now separated air was pushed out of the reservoir along with some suspension, leaving 15.0 g of the suspension in the reservoir. The volume of the 15.0 g in the reservoir was 12.1 mL, i.e., the density of the suspension was 1.24 g/mL. The calculated density, assuming that the density of cocoa butter is 0.9 g/mL and that of LD is 1.5 g/mL, is 1.21 g/mL, showing that the suspension was fluid enough to allow the escape of air-bubbles that would have reduced the density.

The reservoir was next attached to a Cane CronoPAR pump and its luer plug was replaced by an about 2.5 cm long, 2.4 mm internal diameter, plastic tubing terminated by a luer. The assembly and a tightly capped 12 oz jar containing hot water were placed in a Potato Express^{®} bag, which is a thick thermally insulating bag made of layers of cloth, used to microwave-bake potatoes. Pumping, with the pumping rate set to 1 mL/hour, was started when the temperature in the bag was about 50°C. After 1 hour the temperature in the bag dropped to about 43 °C and the flow persisted. After 2 hours the temperature in the bag was about 40°C and the flow continued. After about 2.5 hours the temperature dropped to about 36°C and the flow still persisted. However, when the temperature dropped after about 3 hours to about 33 °C, the flow, if any, was very slow. The volume left after the experiment was about 9 mL, i.e. about 3.1 mL were pumped, consistent with pumping for 3 hours at a rate of 1 mL/hour. After the paste cooled to room temperature it had the consistency of solid soap, i.e. it was no longer fluid and sedimentation of LD was unlikely, if not impossible.

The experiment shows that a concentrated LD suspension (0.79 g/mL, 4.0 M) made with a temperature sensitive carrier liquid (cocoa butter) (a) can be pumped, i.e., it flows, at about 36°C or above; (b) does not flow, or flows very slowly, at or below about 33°C. Thus flow from a small-volume continuously orally LD delivering device where the drug is suspended in cocoa butter should stop when it is removed from the mouth and the ambient temperature is less than about 33°C. The experiment also shows that at an ambient temperature of less than about 36°C sedimentation leading to a non-uniform LD concentration in a suspension could be avoided by making the LD suspension with cocoa butter or another temperature sensitive liquid carrier that is a solid below 33°C.

This application claims benefit of U.S. Provisional Patent Application No. 62/042,553, filed August 27, 2014, U.S. Provisional Patent Application No. 61/987,899, filed May 2, 2014, U.S. Provisional Patent Application No. 61/926,022, filed January 10, 2014, and U.S. Provisional Patent Application No. 61/899,979, filed November 15, 2013.

## Claims

1. A drug delivery device configured to be removably inserted in a patient's mouth and for continuous or semi-continuous intraoral administration of a pharmaceutical composition comprising a drug, said device comprising:
(i) a fastener to removably secure said drug delivery device to a surface of said patient's mouth;
(ii) a mechanical pump selected from a spring, an elastomer, compressed gas, and a propellant-driven pump;
(iii) an oral liquid impermeable drug reservoir comprising a pharmaceutical composition, the volume of said drug reservoir being from 0.1 mL to 5 mL; and
(iv) a flow restrictor for controlling the flow of said pharmaceutical composition from said drug reservoir.

2. The drug delivery device of claim 1, wherein said pharmaceutical composition has a viscosity of greater than 0,1 Pa·s (100 cP) at 37°C.

3. The drug delivery device of claim 2, wherein said pharmaceutical composition comprises levodopa and/or carbidopa, or a pharmaceutically acceptable salt thereof.

4. The drug delivery device of any one of claims 1-3, wherein said mechanical pump comprises a propellant-driven pump.

5. The drug delivery device of claim 4, wherein said propellant driven pump comprises 1,1,1,2 tetrafluoroethane or 1,1,1,2,3,3,3 heptafluoropropane.

6. The drug delivery device of any one of claims 1-4, or wherein said device comprises a suction-induced flow limiter.

7. The drug delivery device of any one of claims 1-4, wherein said device comprises a pressure-invariant pump.

8. The drug delivery device of claim 7, wherein said device comprises a propellant-driven pump configured such that the average rate of drug delivery increases or decreases by less than 10% at 101,353 kPa (14.7 psia) and at 77,911 kPa (11.3 psia), as compared to said average rate of delivery at 89,632 kPa (13.0 psia).

9. The drug delivery device of any one of claims 1-4, wherein said device is configured to deliver an average rate of volume of from 0.015 mL/hour to 1.25 mL/hour over a period of from 4 hours to 168 hours at 37 °C and a constant atmospheric pressure of 89,632 kPa (13 psia), wherein said average rate varies by less than ± 20% per hour over a period of 4 or more hours.

10. The drug delivery device of claim 1, wherein said device comprises one or more drug reservoirs and one or more pumps, wherein said drug reservoirs or said pumps are configured to be worn in the buccal vestibule, and comprising a fluidic channel from the buccal side to the lingual side of said patient's teeth for dispensing said pharmaceutical composition on the lingual side of the teeth.

11. The drug delivery device of any one of claims 1-10, wherein:
(a) said drug reservoir contains a pharmaceutical composition comprising a suspension of particles;
(b) said drug delivery device comprises a fluidic channel or orifice for dispensing of said pharmaceutical composition; and
(c) the particle diameters at all maxima of the particle size distribution are smaller than 1/5th of the narrowest internal diameter of said fluidic channel or orifice.

12. The drug delivery device of claim 11, wherein said particles comprise solid drug particles.

13. The drug delivery device of claim 11, wherein said pharmaceutical composition comprises a paste.

14. The drug delivery device of claim 8, wherein the vapor pressure of said propellant is greater than 400 kPa (4 bars) at 37°C.

15. The drug delivery device of claim 1, wherein said drug reservoir comprises a suspension comprising a drug suitable for continuous or frequent intermittent intra-oral delivery, said suspension comprising at 37 °C solid particles of said drug, a concentration of said drug greater than 2 M, and a viscosity of greater than 10 Pa•s (100 Poise) wherein said suspension remains free of sedimented solid drug for 6 months or more.

16. A fluid pharmaceutical composition comprising a suspension comprising a drug suitable for continuous or frequent intermittent intra-oral delivery, said suspension comprising at 37 °C solid particles of said drug, a concentration of said drug greater than 2 M, and a viscosity of greater than 10 Pa·s (100 Poise) wherein said suspension remains free of sedimented solid drug for 6 months or more, wherein said drug is levodopa, a levodopa prodrug, baclofen, or a pharmaceutically acceptable salt thereof, and wherein said suspension comprises a non-aqueous carrier fluid.

17. The pharmaceutical composition of claim 16, wherein said non-aqueous carrier fluid comprises an oil.

18. The pharmaceutical composition of claim 16, wherein said solid particles of said drug comprise an average diameter of less than 100 micrometers.

19. The pharmaceutical composition of claim 16, wherein the volume fraction of said solid drug is greater than 0.2.

20. The pharmaceutical composition of claim 16, wherein said suspension comprises an emulsion.

21. The pharmaceutical composition of claim 16, wherein said pharmaceutical composition comprises more than 500 mg levodopa per mL, or more than 500 mg of levodopa and carbidopa per mL.

22. A stable, infusible pharmaceutical composition comprising a suspension of carbidopa particles in a fluid at a concentration of 50 mg/mL to 500 mg/mL, wherein the amount of hydrazine is less than 1 mg per 500 mg CD after storage at 5 °C for a period of 1 year, or at 25 °C for a period of 3 months, and wherein said fluid comprises a non-aqueous carrier fluid, an oil, or an edible oil.

23. The pharmaceutical composition of any one of claims 16 to 22 for use in a method of administering said pharmaceutical composition to a patient, said method comprising removably attaching the device of any one of claims 1-15 to an intraoral surface of said patient; or said method comprising continuous or semi-continuous administration of said pharmaceutical into the mouth of said patient.

24. The pharmaceutical composition for use in a method according to claim 23, wherein said pharmaceutical composition is delivered to said patient for a period of not less than 4 hours and not more than 7 days.

25. The pharmaceutical composition for use in a method according to claim 23, wherein said pharmaceutical composition is delivered to said patient continuously or at least once every 30 minutes.

26. The pharmaceutical composition for use in a method according to claim 23, wherein the fluctuation index of said drug is less than or equal to 2.0 during the delivery period.

27. The pharmaceutical composition for use in a method according to claim 23, wherein said pharmaceutical composition comprises baclofen or a pharmaceutically acceptable salt thereof and said patient has spasticity; or wherein said pharmaceutical composition comprises levodopa, a levodopa prodrug, or a pharmaceutically acceptable salt thereof and said patient has Parkinson's disease, the use optionally including:
(a) inserting the drug delivery device into said patient's mouth, said device having a drug reservoir comprising levodopa, a levodopa prodrug, or a pharmaceutically acceptable salt thereof;
(b) administering into said patient's mouth said levodopa, a levodopa prodrug, or a pharmaceutically acceptable salt thereof for a period of at least 8 hours at an hourly rate in the range of 10 - 125 mg/hour; and
(c) removing said drug delivery device from the mouth.

28. A drug delivery device for use in treating Parkinson's disease in a subject, wherein the drug delivery device is designed to be inserted into said subject's mouth, said device having
(i) a fastener to removably secure said drug delivery device to a surface of said patient's mouth;
(ii) a mechanical pump selected from a spring, an elastomer, compressed gas, and a propellant-driven pump;
(iii) an oral liquid impermeable drug reservoir having a volume of from 0.1 ml to 5 ml comprising a suspension or solid containing drug prepared to be administered into said patient's mouth continuously or semi-continuously; and
(iv) a flow restrictor for controlling the flow of said pharmaceutical composition from said drug reservoir;
and said drug delivery device is designed to be removed from the mouth of the subject after administration of the drug.

29. The drug delivery device for the use in treating Parkinson's disease according to claim 28, wherein said drug is levodopa, a levodopa prodrug, or a pharmaceutically acceptable salt thereof.

30. The drug delivery device for the use in treating Parkinson's disease according to claim 28, wherein said suspension is a paste prepared to be administered into said subject's mouth continuously or at a frequency of at least once every 30 minutes.

31. The drug delivery device for the use in treating Parkinson's disease according to claim 28, wherein said composition comprises a non-aqueous carrier fluid, an oil, or an edible oil.

32. The drug delivery device for the use in treating Parkinson's disease according to claim 28, wherein the drug delivery device comprises a suction-induced flow limiter.

33. The drug delivery device for the use in treating Parkinson's disease according to claim 28, wherein the subject has delayed gastric emptying or retarded gastrointestinal transit.

34. The drug delivery device for the use in treating Parkinson's disease according to any one of claims 28-33, wherein the drug is levodopa or a levodopa prodrug and wherein one or more of said drug delivery devices are inserted into said subject's mouth and the suspension or solid is prepared to be administered to the subject for a period of at least 8 hours at an hourly rate in the range of 10 - 125 mg/hour.

35. The drug delivery device for the use in treating Parkinson's disease according to any one of claims 28-33, wherein the drug reservoir comprises a composition comprising a suspension of solid particles of said levodopa or levodopa prodrug, wherein the composition has a concentration of said levodopa or levodopa prodrug greater than 2 M and a viscosity of greater than 10 Pa•s (100 Poise).

## Patentansprüche

1. Vorrichtung zur Verabreichung von Arzneimitteln, die so konfiguriert ist, dass sie abnehmbar in den Mund eines Patienten eingeführt werden kann und zur kontinuierlichen oder halbkontinuierlichen intraoralen Verabreichung einer pharmazeutischen Zusammensetzung, die ein Arzneimittel umfasst, wobei die Vorrichtung umfasst:
(i) ein Befestigungselement zur abnehmbaren Befestigung der Vorrichtung zur Verabreichung von Arzneimitteln an einer Oberfläche des Mundes des Patienten;
(ii) eine mechanische Pumpe, ausgewählt aus einer Feder, einem Elastomer, Druckgas und einer Treibmittel-betriebenen Pumpe;
(iii) ein orales flüssigkeitsundurchlässiges Arzneimittelreservoir, das eine pharmazeutische Zusammensetzung umfasst, wobei das Volumen des Arzneimittelreservoirs von 0,1 ml bis 5 ml beträgt; und
(iv) einen Durchflussbegrenzer zur Steuerung des Flusses der pharmazeutischen Zusammensetzung aus dem Arzneimittelreservoir.

2. Vorrichtung zur Verabreichung von Arzneimitteln nach Anspruch 1, wobei die pharmazeutische Zusammensetzung bei 37 °C eine Viskosität von mehr als 0,1 Pa·s (100 cP) aufweist.

3. Vorrichtung zur Verabreichung von Arzneimitteln nach Anspruch 2, wobei die pharmazeutische Zusammensetzung Levodopa und/oder Carbidopa oder ein pharmazeutisch verträgliches Salz davon umfasst.

4. Vorrichtung zur Verabreichung von Arzneimitteln nach einem der Ansprüche 1 bis 3, wobei die mechanische Pumpe eine Treibmittel-betriebene Pumpe umfasst.

5. Vorrichtung zur Verabreichung von Arzneimitteln nach Anspruch 4, wobei die Treibmittel-betriebene Pumpe 1,1,1,2-Tetrafluorethan oder 1,1,1,2,3,3,3-Heptafluorpropan umfasst.

6. Vorrichtung zur Verabreichung von Arzneimitteln nach einem der Ansprüche 1 bis 4, oder wobei die Vorrichtung einen sauginduzierten Durchflussbegrenzer umfasst.

7. Vorrichtung zur Verabreichung von Arzneimitteln nach einem der Ansprüche 1 bis 4, wobei die Vorrichtung eine druckinvariante Pumpe umfasst.

8. Vorrichtung zur Verabreichung von Arzneimitteln nach Anspruch 7, wobei die Vorrichtung eine Treibmittel-betriebene Pumpe umfasst, die so konfiguriert ist, dass die durchschnittliche Rate der Arzneimittelverabreichung bei 101.353 kPa (14,7 psia) und bei 77.911 kPa (11,3 psia) um weniger als 10 % zunimmt oder abnimmt, verglichen mit der durchschnittlichen Verabreichungsrate bei 89.632 kPa (13,0 psia).

9. Vorrichtung zur Verabreichung von Arzneimitteln nach einem der Ansprüche 1 bis 4, wobei die Vorrichtung so konfiguriert ist, dass sie bei 37 °C und einem konstanten atmosphärischen Druck von 89.632 kPa (13 psia) eine durchschnittliche Volumenrate von 0,015 ml/Stunde bis 1,25 ml/Stunde über einen Zeitraum von 4 Stunden bis 168 Stunden abgibt, wobei die durchschnittliche Rate um weniger als ± 20 % pro Stunde über einen Zeitraum von 4 oder mehr Stunden schwankt.

10. Vorrichtung zur Verabreichung von Arzneimitteln nach Anspruch 1, wobei die Vorrichtung ein oder mehrere Arzneimittelreservoirs und eine oder mehrere Pumpen umfasst, wobei die Arzneimittelreservoirs oder die Pumpen so konfiguriert sind, dass sie im bukkalen Vestibulum getragen werden, und einen Fluidkanal von der bukkalen Seite zur lingualen Seite der Zähne des Patienten umfassen, um die pharmazeutische Zusammensetzung auf der lingualen Seite der Zähne abzugeben.

11. Vorrichtung zur Verabreichung von Arzneimitteln nach einem der Ansprüche 1 bis 10, wobei:
(a) das Arzneimittelreservoir eine pharmazeutische Zusammensetzung enthält, die eine Suspension von Partikeln umfasst;
(b) die Vorrichtung zur Verabreichung von Arzneimitteln einen Fluidkanal oder eine Öffnung zur Abgabe der pharmazeutischen Zusammensetzung umfasst; und
(c) die Partikeldurchmesser bei allen Maxima der Partikelgrößenverteilung kleiner als 1/5 des engsten Innendurchmessers des Fluidkanals oder der Öffnung sind.

12. Vorrichtung zur Verabreichung von Arzneimitteln nach Anspruch 11, wobei die Partikel feste Arzneimittelpartikel umfassen.

13. Vorrichtung zur Verabreichung von Arzneimitteln nach Anspruch 11, wobei die pharmazeutische Zusammensetzung eine Paste umfasst.

14. Vorrichtung zur Verabreichung von Arzneimitteln Anspruch 8, wobei der Dampfdruck des Treibmittels bei 37 °C mehr als 400 kPa (4 bar) beträgt.

15. Vorrichtung zur Verabreichung von Arzneimitteln nach Anspruch 1, wobei das Arzneimittelreservoir eine Suspension umfasst, die ein Arzneimittel umfasst, das zur kontinuierlichen oder häufig intermittierenden intraoralen Verabreichung geeignet ist, wobei die Suspension bei 37 °C feste Partikel des Arzneimittels umfasst, mit einer Konzentration des Arzneimittels von mehr als 2 M und einer Viskosität von mehr als 10 Pa·s (100 Poise), wobei die Suspension für 6 Monate oder länger frei von sedimentiertem festem Arzneimittel bleibt.

16. Flüssige pharmazeutische Zusammensetzung, umfassend eine Suspension, die ein Arzneimittel umfasst, das zur kontinuierlichen oder häufig intermittierenden intraoralen Verabreichung geeignet ist, wobei die Suspension bei 37 °C feste Partikel des Arzneimittels umfasst, mit einer Konzentration des Arzneimittels von mehr als 2 M und eine Viskosität von mehr als 10 Pa·s (100 Poise), wobei die Suspension 6 Monate oder länger frei von sedimentiertem festem Arzneimittel bleibt, wobei das Arzneimittel Levodopa, ein Levodopa-Prodrug, Baclofen oder ein pharmazeutisch verträgliches Salz davon ist und wobei die Suspension eine nichtwässrige Trägerflüssigkeit umfasst.

17. Pharmazeutische Zusammensetzung nach Anspruch 16, wobei die nicht-wässrige Trägerflüssigkeit ein Öl umfasst.

18. Pharmazeutische Zusammensetzung nach Anspruch 16, wobei die festen Partikel des Arzneimittels einen durchschnittlichen Durchmesser von weniger als 100 Mikrometer aufweisen.

19. Pharmazeutische Zusammensetzung nach Anspruch 16, wobei der Volumenanteil des festen Arzneimittels größer als 0,2 ist.

20. Pharmazeutische Zusammensetzung nach Anspruch 16, wobei die Suspension eine Emulsion umfasst.

21. Pharmazeutische Zusammensetzung nach Anspruch 16, wobei die pharmazeutische Zusammensetzung mehr als 500 mg Levodopa pro ml oder mehr als 500 mg Levodopa und Carbidopa pro ml umfasst.

22. Stabile, zur Infusion geeignete pharmazeutische Zusammensetzung, die eine Suspension von Carbidopa-Partikeln in einer Flüssigkeit mit einer Konzentration von 50 mg/ml bis 500 mg/ml umfasst, wobei die Menge an Hydrazin weniger als 1 mg pro 500 mg CD nach Lagerung bei 5 °C für einen Zeitraum von 1 Jahr oder bei 25 °C für einen Zeitraum von 3 Monaten beträgt und wobei die Flüssigkeit eine nicht-wässrige Trägerflüssigkeit, ein Öl oder ein Speiseöl umfasst.

23. Pharmazeutische Zusammensetzung nach einem der Ansprüche 16 bis 22 zur Verwendung in einem Verfahren zur Verabreichung der pharmazeutischen Zusammensetzung an einen Patienten, wobei das Verfahren das abnehmbare Anbringen der Vorrichtung nach einem der Ansprüche 1 bis 15 an einer intraoralen Oberfläche des Patienten umfasst; oder das Verfahren die kontinuierliche oder halbkontinuierliche Verabreichung des Arzneimittels in den Mund des Patienten umfasst.

24. Pharmazeutische Zusammensetzung zur Verwendung in einem Verfahren nach Anspruch 23, wobei die pharmazeutische Zusammensetzung dem Patienten über einen Zeitraum von nicht weniger als 4 Stunden und nicht mehr als 7 Tagen verabreicht wird.

25. Pharmazeutische Zusammensetzung zur Verwendung in einem Verfahren nach Anspruch 23, wobei die pharmazeutische Zusammensetzung dem Patienten kontinuierlich oder mindestens einmal alle 30 Minuten verabreicht wird.

26. Pharmazeutische Zusammensetzung zur Verwendung in einem Verfahren nach Anspruch 23, wobei der Fluktuationsindex des Arzneimittels während des Verabreichungszeitraums kleiner oder gleich 2,0 ist.

27. Pharmazeutische Zusammensetzung zur Verwendung in einem Verfahren nach Anspruch 23, wobei die pharmazeutische Zusammensetzung Baclofen oder ein pharmazeutisch verträgliches Salz davon umfasst und der Patient an Spastizität leidet; oder wobei die pharmazeutische Zusammensetzung Levodopa, ein Levodopa-Prodrug oder ein pharmazeutisch verträgliches Salz davon umfasst und der Patient an der Parkinson-Krankheit leidet, wobei die Verwendung optional einschließt:
(a) Einführen der Vorrichtung zur Verabreichung von Arzneimitteln in den Mund des Patienten, wobei die Vorrichtung ein Arzneimittelreservoir aufweist, das Levodopa, ein Levodopa-Prodrug oder ein pharmazeutisch verträgliches Salz davon umfasst;
(b) Verabreichung des Levodopa, eines Levodopa-Prodrugs oder eines pharmazeutisch verträglichen Salzes davon in den Mund des Patienten über einen Zeitraum von mindestens 8 Stunden mit einer stündlichen Rate im Bereich von 10 bis 125 mg/Stunde; und
(c) Entfernen der Vorrichtung zur Verabreichung von Arzneimitteln aus dem Mund.

28. Vorrichtung zur Verabreichung von Arzneimitteln zur Verwendung bei der Behandlung der Parkinson-Krankheit bei einem Patienten, wobei die Vorrichtung zur Verabreichung von Arzneimitteln so gestaltet ist, dass sie in den Mund des Patienten eingeführt werden kann, wobei die Vorrichtung Folgendes aufweist
(i) ein Befestigungselement zur abnehmbaren Befestigung der Vorrichtung zur Verabreichung von Arzneimitteln an einer Oberfläche des Mundes des Patienten;
(ii) eine mechanische Pumpe, ausgewählt aus einer Feder, einem Elastomer, Druckgas und einer Treibmittel-betriebenen Pumpe;
(iii) ein orales flüssigkeitsundurchlässiges Arzneimittelreservoir mit einem Volumen von 0,1 ml bis 5 ml, das eine Suspension oder einen Feststoff umfasst, enthaltend ein Arzneimittel, das zur kontinuierlichen oder semikontinuierlichen Verabreichung in den Mund des Patienten vorbereitet ist; und
(iv) einen Durchflussbegrenzer zur Steuerung des Flusses der pharmazeutischen Zusammensetzung aus dem Arzneimittelreservoir;
und die Vorrichtung zur Verabreichung des Arzneimittels so gestaltet ist, dass sie nach der Verabreichung des Arzneimittels aus dem Mund des Patienten entfernt werden kann.

29. Vorrichtung zur Verabreichung von Arzneimitteln zur Verwendung bei der Behandlung der Parkinson-Krankheit nach Anspruch 28, wobei das Arzneimittel Levodopa, ein Levodopa-Prodrug oder ein pharmazeutisch verträgliches Salz davon ist.

30. Vorrichtung zur Verabreichung von Arzneimitteln zur Verwendung bei der Behandlung der Parkinson-Krankheit nach Anspruch 28, wobei die Suspension eine Paste ist, die so zubereitet ist, dass sie kontinuierlich oder mindestens einmal alle 30 Minuten in den Mund des Patienten verabreicht werden kann.

31. Vorrichtung zur Verabreichung von Arzneimitteln zur Verwendung bei der Behandlung der Parkinson-Krankheit nach Anspruch 28, wobei die Zusammensetzung eine nicht-wässrige Trägerflüssigkeit, ein Öl oder ein Speiseöl umfasst.

32. Vorrichtung zur Verabreichung von Arzneimitteln zur Verwendung bei der Behandlung der Parkinson-Krankheit nach Anspruch 28, wobei die Vorrichtung zur Verabreichung von Arzneimitteln einen sauginduzierten Durchflussbegrenzer umfasst.

33. Vorrichtung zur Verabreichung von Arzneimitteln zur Verwendung bei der Behandlung der Parkinson-Krankheit gemäß Anspruch 28, wobei der Patient eine verzögerte Magenentleerung oder einen verzögerten Magen-Darm-Transit aufweist.

34. Vorrichtung zur Verabreichung von Arzneimitteln zur Verwendung bei der Behandlung der Parkinson-Krankheit nach einem der Ansprüche 28 bis 33, wobei das Arzneimittel Levodopa oder ein Levodopa-Prodrug ist und wobei eine oder mehrere der Vorrichtungen zur Verabreichung von Arzneimitteln in den Mund des Patienten eingeführt werden und die Suspension oder der Feststoff so zubereitet wird, dass sie dem Patienten über einen Zeitraum von mindestens 8 Stunden mit einer Stundenrate im Bereich von 10 bis 125 mg/Stunde verabreicht werden.

35. Vorrichtung zur Verabreichung von Arzneimitteln zur Verwendung bei der Behandlung der Parkinson-Krankheit nach einem der Ansprüche 28 bis 33, wobei das Arzneimittelreservoir eine Zusammensetzung umfasst, die eine Suspension fester Partikel des Levodopa- oder Levodopa-Prodrugs umfasst, wobei die Zusammensetzung eine Konzentration des Levodopa- oder Levodopa-Prodrugs von mehr als 2 M und eine Viskosität von mehr als 10 Pa·s (100 Poise) aufweist.

## Revendications

1. Dispositif d'administration de médicament configuré pour être inséré de manière amovible dans la bouche d'un patient et pour une administration intra-orale continue ou semi-continue d'une composition pharmaceutique comprenant un médicament, ledit dispositif comprenant :
(i) une fixation pour fixer de manière amovible ledit dispositif d'administration de médicament à une surface de ladite bouche du patient ;
(ii) une pompe mécanique sélectionnée parmi un ressort, un élastomère, un gaz comprimé et une pompe à propergol ;
(iii) un réservoir de médicament imperméable aux liquides ingérables comprenant une composition pharmaceutique, le volume dudit réservoir de médicament étant de 0,1 mL à 5 mL ; et
(iv) un limitateur d'écoulement pour commander l'écoulement de ladite composition pharmaceutique à partir dudit réservoir de médicament.

2. Dispositif d'administration de médicament selon la revendication 1, dans lequel ladite composition pharmaceutique présente une viscosité supérieure à 0,1 Pa.s (100 cP) à 37°C.

3. Dispositif d'administration de médicament selon la revendication 2, dans lequel ladite composition pharmaceutique comprend de la lévodopa et/ou de la carbidopa, ou un sel pharmaceutiquement acceptable de celles-ci.

4. Dispositif d'administration de médicament selon l'une quelconque des revendications 1 à 3, dans lequel ladite pompe mécanique comprend une pompe entraînée par un agent propulseur.

5. Dispositif d'administration de médicament selon la revendication 4, dans lequel ladite pompe entraînée par un agent propulseur comprend du 1,1,1,2 tétrafluoroéthane ou du 1,1,1,2,3,3,3 heptafluoropropane.

6. Dispositif d'administration de médicament selon l'une quelconque des revendications 1 à 4, dans lequel ledit dispositif comprend un limiteur d'écoulement induit par aspiration.

7. Dispositif d'administration de médicament selon l'une quelconque des revendications 1 à 4, dans lequel ledit dispositif comprend une pompe à pression invariable.

8. Dispositif d'administration de médicament selon la revendication 7, dans lequel ledit dispositif comprend une pompe entraînée par un agent propulseur configurée de telle sorte que le taux moyen d'administration de médicament augmente ou diminue de moins de 10 % à 101 353 kPa (14,7 psia) et à 77 911 kPa (11,3 psia), par rapport audit taux moyen d'administration à 89 632 kPa (13,0 psia).

9. Dispositif d'administration de médicament selon l'une quelconque des revendications 1 à 4, dans lequel ledit dispositif est configuré pour délivrer un débit volumique moyen de 0,015 mL/heure à 1,25 mL/heure sur une période de 4 heures à 168 heures à 37°C et une pression atmosphérique constante de 89 632 kPa (13 psia), dans lequel ledit débit moyen varie de moins de ± 20 % par heure sur une période de 4 heures ou plus.

10. Dispositif d'administration de médicament selon la revendication 1, dans lequel ledit dispositif comprend un ou plusieurs réservoirs de médicament et une ou plusieurs pompes, dans lequel lesdits réservoirs de médicament ou lesdites pompes sont configurés pour être portés dans le vestibule buccal, et comprenant un canal fluidique du côté buccal au côté lingual desdites dents du patient pour distribuer ladite composition pharmaceutique sur le côté lingual des dents.

11. Dispositif d'administration de médicament selon l'une quelconque des revendications 1 à 10, dans lequel :
(a) ledit réservoir de médicament contient une composition pharmaceutique comprenant une suspension de particules ;
(b) ledit dispositif d'administration de médicament comprend un canal ou un orifice fluidique pour une distribution de ladite composition pharmaceutique ; et
(c) les diamètres de particule à tous les maxima de la distribution de taille de particule sont inférieurs à 1/5ème du diamètre interne le plus étroit dudit canal ou orifice fluidique.

12. Dispositif d'administration de médicament selon la revendication 11, dans lequel lesdites particules comprennent des particules de médicament solides.

13. Dispositif d'administration de médicament selon la revendication 11, dans lequel ladite composition pharmaceutique comprend une pâte.

14. Dispositif d'administration de médicament selon la revendication 8, dans lequel la pression de vapeur dudit propulseur est supérieure à 400 kPa (4 bars) à 37°C.

15. Dispositif d'administration de médicament selon la revendication 1, dans lequel ledit réservoir de médicament comprend une suspension comprenant un médicament approprié pour une administration intra-orale intermittente continue ou fréquente, ladite suspension comprenant à 37°C des particules solides dudit médicament, une concentration dudit médicament supérieure à 2 M, et une viscosité supérieure à 10 Pa (100 Poises), dans lequel ladite suspension reste exempte de médicament solide sédimenté pendant 6 mois ou plus.

16. Composition pharmaceutique fluide comprenant une suspension comprenant un médicament approprié pour une administration intra-orale intermittente continue ou fréquente, ladite suspension comprenant à 37°C des particules solides dudit médicament, une concentration dudit médicament supérieure à 2 M, et une viscosité supérieure à 10 Pa.s (100 Poises), dans laquelle ladite suspension reste exempte de médicament solide sédimenté pendant 6 mois ou plus, dans laquelle ledit médicament est la lévodopa, un promédicament de lévodopa, du baclofène, ou un sel pharmaceutiquement acceptable de ceux-ci, et dans laquelle ladite suspension comprend un fluide porteur non aqueux.

17. Composition pharmaceutique selon la revendication 16, dans laquelle ledit fluide porteur non aqueux comprend une huile.

18. Composition pharmaceutique selon la revendication 16, dans laquelle lesdites particules solides dudit médicament comprennent un diamètre moyen inférieur à 100 micromètres.

19. Composition pharmaceutique selon la revendication 16, dans laquelle la fraction volumique dudit médicament solide est supérieure à 0,2.

20. Composition pharmaceutique selon la revendication 16, dans laquelle ladite suspension comprend une émulsion.

21. Composition pharmaceutique selon la revendication 16, dans laquelle ladite composition pharmaceutique comprend plus de 500 mg de lévodopa par mL, ou plus de 500 mg de lévodopa et de carbidopa par mL.

22. Composition pharmaceutique stable et infusible comprenant une suspension de particules de carbidopa dans un fluide à une concentration de 50 mg/mL à 500 mg/mL, dans laquelle la quantité d'hydrazine est inférieure à 1 mg par 500 mg CD après stockage à 5°C pendant une période de 1 an, ou à 25°C pendant une période de 3 mois, et dans laquelle ledit fluide comprend un fluide porteur non aqueux, une huile ou une huile comestible.

23. Composition pharmaceutique selon l'une quelconque des revendications 16 à 22, à utiliser dans un procédé d'administration de ladite composition pharmaceutique à un patient, ledit procédé comprenant la fixation amovible du dispositif selon l'une quelconque des revendications 1 à 15 à une surface intra-orale dudit patient ; ou ledit procédé comprenant une administration continue ou semi-continue dudit produit pharmaceutique dans la bouche dudit patient.

24. Composition pharmaceutique à utiliser dans un procédé selon la revendication 23, dans laquelle ladite composition pharmaceutique est administrée audit patient pendant une période d'au moins 4 heures et d'au plus 7 jours.

25. Composition pharmaceutique à utiliser dans un procédé selon la revendication 23, dans laquelle ladite composition pharmaceutique est administrée audit patient en continu ou au moins une fois toutes les 30 minutes.

26. Composition pharmaceutique à utiliser dans un procédé selon la revendication 23, dans laquelle l'indice de fluctuation dudit médicament est inférieur ou égal à 2,0 pendant la période d'administration.

27. Composition pharmaceutique à utiliser dans un procédé selon la revendication 23,
dans laquelle ladite composition pharmaceutique comprend du baclofène ou un sel pharmaceutiquement acceptable de celui-ci et ledit patient présente une spasticité ; ou dans laquelle ladite composition pharmaceutique comprend de la lévodopa, un promédicament de lévodopa, ou un sel pharmaceutiquement acceptable de celle-ci/celui-ci et ledit patient présente la maladie de Parkinson, l'utilisation comprenant facultativement les étapes consistant à :
(a) insérer le dispositif d'administration de médicament dans la bouche dudit patient, ledit dispositif présentant un réservoir de médicament comprenant de la lévodopa, un promédicament de lévodopa, ou un sel pharmaceutiquement acceptable de celui-ci ;
(b) administrer dans la bouche dudit patient ladite lévodopa, un promédicament de lévodopa, ou un sel pharmaceutiquement acceptable de celui-ci pendant une période d'au moins 8 heures à un débit horaire compris entre 10 et 125 mg/heure ; et
(c) retirer ledit dispositif d'administration de médicament de la bouche.

28. Dispositif d'administration de médicament à utiliser dans le traitement de la maladie de Parkinson chez un sujet, dans lequel le dispositif d'administration de médicament est conçu pour être inséré dans la bouche dudit sujet, ledit dispositif présentant
(i) une fixation pour fixer de manière amovible ledit dispositif d'administration de médicament à une surface de ladite bouche du patient ;
(ii) une pompe mécanique sélectionnée parmi un ressort, un élastomère, un gaz comprimé et une pompe à propergol ;
(iii) un réservoir de médicament imperméable aux liquides ingérables présentant un volume de 0,1 ml à 5 ml comprenant une suspension ou un solide contenant un médicament préparé pour être administré dans la bouche dudit patient de manière continue ou semi-continue ; et
(iv) un limiteur d'écoulement pour réguler l'écoulement de ladite composition pharmaceutique à partir dudit réservoir de médicament ;
et ledit dispositif d'administration de médicament est conçu pour être retiré de la bouche du sujet après administration du médicament.

29. Dispositif d'administration de médicament à utiliser dans le traitement de la maladie de Parkinson selon la revendication 28, dans lequel ledit médicament est la lévodopa, un promédicament de lévodopa, ou un sel pharmaceutiquement acceptable de celle-ci/celui-ci.

30. Dispositif d'administration de médicament à utiliser dans le traitement de la maladie de Parkinson selon la revendication 28, dans lequel ladite suspension est une pâte préparée pour être administrée dans la bouche dudit sujet en continu ou à une fréquence d'au moins une fois toutes les 30 minutes.

31. Dispositif d'administration de médicament à utiliser dans le traitement de la maladie de Parkinson selon la revendication 28, dans lequel ladite composition comprend un fluide porteur non aqueux, une huile ou une huile comestible.

32. Dispositif d'administration de médicament à utiliser dans le traitement de la maladie de Parkinson selon la revendication 28, dans lequel le dispositif d'administration de médicament comprend un limiteur d'écoulement induit par aspiration.

33. Dispositif d'administration de médicament à utiliser dans le traitement de la maladie de Parkinson selon la revendication 28, dans lequel le sujet présente une vidange gastrique retardée ou un transit gastro-intestinal retardé.

34. Dispositif d'administration de médicament à utiliser dans le traitement de la maladie de Parkinson selon l'une quelconque des revendications 28 à 33, dans lequel le médicament est la lévodopa ou un promédicament de lévodopa et dans lequel un ou plusieurs desdits dispositifs d'administration de médicament sont insérés dans la bouche dudit sujet et la suspension ou le solide est préparé(e) pour être administré(e) au sujet pendant une période d'au moins 8 heures à un débit horaire dans la plage de 10 à 125 mg/heure.

35. Dispositif d'administration de médicament à utiliser dans le traitement de la maladie de Parkinson selon l'une quelconque des revendications 28 à 33, dans lequel le réservoir de médicament comprend une composition comprenant une suspension de particules solides dudit promédicament de lévodopa ou de lévodopa, dans lequel la composition présente une concentration dudit promédicament de lévodopa ou de lévodopa supérieure à 2 M et une viscosité supérieure à 10 Pa.s (100 Poises).
